(19) Europäisches Patentamt
European Patent Office
Office européen des brevets

(11) EP 2 650 293 A1

(12) **EUROPEAN PATENT APPLICATION**
published in accordance with Art. 153(4) EPC

(43) Date of publication:
**16.10.2013 Bulletin 2013/42**

(21) Application number: **11846471.8**

(22) Date of filing: **08.12.2011**

(51) Int Cl.:
***C07D 487/04*** (2006.01) ***A61K 31/53*** (2006.01)
***A61P 35/00*** (2006.01) ***A61P 35/02*** (2006.01)

(86) International application number:
**PCT/CN2011/002052**

(87) International publication number:
**WO 2012/075683 (14.06.2012 Gazette 2012/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.12.2010 CN 201010579221**

(71) Applicant: **Shanghai Institute Materia Medica, Chinese Academy Of Sciences**
**Pudong, Shanghai 201203 (CN)**

(72) Inventors:
- **DUAN, Wenhu**
  **Pudong**
  **Shanghai 201203 (CN)**
- **GENG, Meiyu**
  **Pudong**
  **Shanghai 201203 (CN)**
- **CHEN, Fang**
  **Pudong**
  **Shanghai 201203 (CN)**
- **AI, Jing**
  **Pudong**
  **Shanghai 201203 (CN)**
- **CHEN, Yi**
  **Pudong**
  **Shanghai 201203 (CN)**
- **ZHAN, Zhengsheng**
  **Pudong**
  **Shanghai 201203 (CN)**
- **LV, Yongcong**
  **Pudong**
  **Shanghai 201203 (CN)**
- **WANG, Ying**
  **Pudong**
  **Shanghai 201203 (CN)**
- **DING, Jian**
  **Pudong**
  **Shanghai 201203 (CN)**

(74) Representative: **HOFFMANN EITLE**
**Patent- und Rechtsanwälte**
**Arabellastrasse 4**
**81925 München (DE)**

# (54) [1,2,4]TRIAZOLO[4,3-B][1,2,4]TRIAZINE COMPOUND, PREPARATION METHOD AND USE THEREOF

(57) The present invention relates to a structurally novel [1,2,4]triazolo[4,3-b][1,2,4]triazine compounds represented by formula (I) or formula (II), pharmaceutically acceptable salts thereof, prodrugs thereof, hydrates or solvates thereof, and also relates to a preparation method of the compounds, a pharmaceutical composition comprising a therapeutically effective amount of the compounds, as well as the use thereof as protein tyrosine kinase inhibitors, particularly as c-Met inhibitors, in the preparation of medicaments for the prevention and/or treatment of diseases associated with c-Met abnormality.

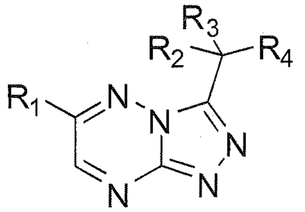

(I)

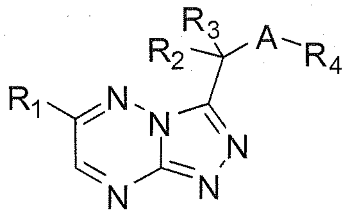

(II)

EP 2 650 293 A1

## Description

### Field of the Invention

The present invention relates to the field of pharmaceutical chemistry and pharmaceutical therapeutics, specifically relates to [1, 2, 4] triazolo [4, 3- b] [1, 2, 4] triazine compounds, pharmaceutically acceptable salts, prodrugs, hydrates or solvates thereof, and also relates to a preparation method of the compounds, pharmaceutical compositions comprising the compounds, and a use thereof as protein tyrosine kinase inhibitors, especially as c- Met inhibitors, in the preparation of medicaments for the prevention and/or treatment of diseases associated with c- Met abnormality.

### Background of the Invention

For a long time, it is a worldwide difficult problem to treat malignant tumors which threaten the human life seriously. The survival rate of cancer patients is extremely low although the diagnosis and treatment thereof has made progress. In recent years, several new anti-tumor targets were found, in which the protein tyrosine kinases have become new promising anti-tumor targets.

Protein tyrosine kinases (PTKs) are a group of enzymes having the functions of regulating a variety of important biological processes, including cell growth, differentiation, organogenesis, angiogenesis, tissue repair, regeneration, and the like. Protein tyrosine kinases exert their biological effects through catalyzing the phosphorylation of protein tyrosine residues, and then regulate the biological activity of the substrate protein. The disorder of a class of protein tyrosine kinase may lead to the tumor formation and development, and further plays an important role in the survival and evolution of tumors (Blume JP, Hunter T. Oncogenic kinase signaling [J]. Nature, 2001, 411 (6835): 355-365). Therefore, the protein tyrosine kinase closely related to the tumor represents a class of the most important protein targets related to cancer treatment and drug development.

c-Met, which is a receptor-type protein tyrosine kinase, is a MET proto-oncogene encoding hepatocyte growth factor receptor (HGFR). The mature form of c-Met is composed of an extracellular alpha-chain (50KDa) and a transmembrane beta-chain (145KDa, the intracellular segment containing the kinase section is anchored on the cell membrane) so as to form a heterodimer structure to perform its function. c-Met has a high expression in most of solid tumors and part of malignancies, such as lung cancer, breast cancer, colon cancer, prostate cancer, pancreatic cancer, gastric cancer, liver cancer, ovarian cancer, renal carcinoma, neurospongioma and melanoma, and has been closely associated with poor clinical outcomes. c-Met activates the tyrosine kinases in the intracellular segment via the interaction with its ligand HGF/SF, or other routes to induce tumor cell proliferation, cell invasion, cell motility, inhibit apoptosis, and promote tumor angiogenesis, and thus plays an important role during the tumor generation and development.

Unlike other kinases, c-Met may interact with other tumor-associated moleculars on the cell surface, such as the integrin family, death related receptors, other receptor-type tyrosine kinases etc., so as to crosslinkly activate and enlarge the tumor-associated effects, and thus greatly promote tumor generation and development, wherein c-Met has played a pivotal role. Therefore, the inhibition of c-met may inhibit a plural of tumor targets.

It has been reported that the acquired drug- resistance of the clinical applied EGFR receptor tyrosine kinase inhibitor (EGFR- TKIs) is just caused for the ERBB3 signaling pathway activated by MET amplification. Therefore, the combination of c- Met inhibitor and EGFR inhibitor can delay or overcome the generation of the acquired drug- resistance of EGFR- TKIs, which has important clinical significance.

As mentioned above, it is an important strategy for the cancer treatment by inhibiting the c- Met signaling pathway. Recently, it has been found that many compounds can effectively block the HGF/c- Met signal transduction pathways, for example, a series of small molecule compounds developed by the Sugen company may inhibit the Met kinase activity at the nanomolar level (WO2005004607, WO2005004808, WO2005005378, and WO2005010005), however, there is no report on the data of *in- vivo* activity of these compounds; the phase I clinical trial of SGX 126 developed by SGX Company has been interrupted due to renal toxicity (WO2008051808) ; the triazolopyridazine compound JNJ-38877605 of Johnson & Johnson (WO2007075567) and PF- 04217903 of Pfizer (US2007265272) have entered the phase I clinical trial. In addition, a plurality of multi- targeted tyrosine kinase inhibitors that can effectively block the HGF/c- Met signal transduction pathway have entered the clinical studies, however there is still no small molecule c-Met tyrosine kinase inhibitor has been approved for clinical use, thus it is necessary to find more safe and effective inhibitors. The present inventors have designed and synthesized [1, 2, 4] triazolo [4, 3- b] [1, 2, 4] triazine compounds with a novel structure, and the inventors found small molecule drugs with high activity at enzyme levels, cell levels and *in- vivo* tests by optimizing the substituents.

**Summary of the Invention**

According to the characteristics of the high expression and the abnormal activation of c-Met in the tumor, the present inventors designed and synthesized a series of compounds with novel structure based on the crystal structure of c-Met and the structure-activity relationships of the other tyrosine kinase inhibitors. By screening these compounds through the molecular and cell screening model, the inventors found that these compounds can significantly inhibit the enzymatic activity of c-Met, and also have a significant inhibitory action for c-Met phosphorylation/activation on the cellular level. In addition, the compounds can significantly inhibit the growth of the human tumor xenografts in nude mice.
It is an object of the present invention to provide [1, 2, 4] triazolo [4, 3- b] [1, 2, 4] triazine compounds having a structure as shown in the following formula (I) or (II), pharmaceutically acceptable salts, prodrugs, hydrates or solvates thereof.

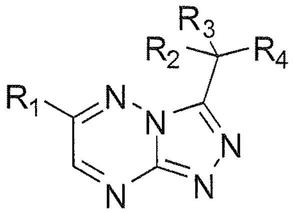
(I)

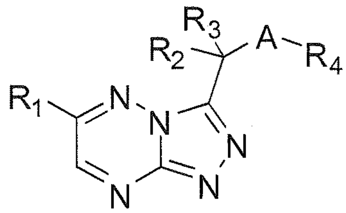
(II)

It is another object of the present invention to provide a method for preparing the above [1, 2, 4] triazolo [4, 3- b] [1, 2, 4] triazine compounds.
It is still another object of the present invention to provide a pharmaceutical composition comprising a therapeutically effective dose of the above- mentioned [1, 2, 4] triazolo [4, 3- b] [1, 2, 4] triazine compounds.
It is still another object of the present invention to provide a use of the above- mentioned [1, 2, 4] triazolo [4, 3- b] [1, 2, 4] triazine compounds as protein tyrosine kinase inhibitors, especially as c- Met inhibitors, in the preparation of medicaments for the prevention and/or treatment of diseases associated with c- Met abnormality.
Specifically, the present invention relates to [1, 2, 4] triazolo [4, 3- b] [1, 2, 4] triazine compounds having a formula (I) or (II), pharmaceutically acceptable salts, prodrugs, hydrates or solvates thereof:

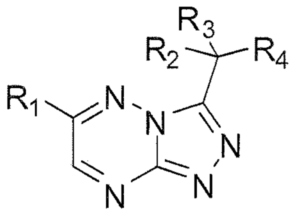
(I)

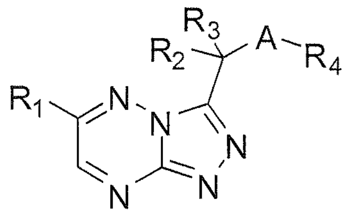
(II)

wherein:

$R_1$ and $R_4$ may each independently be a substituted or unsubstituted aryl or heteroaryl,
the aryl may be a $C_6$-$C_{20}$ monocyclic or fused ring group having at least one aromatic ring, such as phenyl, substituted phenyl, naphthyl or xenyl;
the heteroaryl group may be a 5- to 10-membered heteroaryl group containing 1 to 5 hetero atoms selected from the group consisting of N, S, P and O, such as pyrazolyl, furyl, pyrrolyl, pyridyl, thienyl, imidazolyl, quinolyl, isoquinolyl or indolyl;
the substituents for the substitution are 1-4 group(s) selected from the group consisting of halogen, $C_1$-$C_6$ linear or branched alkyl, nitro group, amino, hydroxyl, hydroxymethyl, trifluoromethyl, trifluoromethoxy, carboxyl, $C_1$-$C_4$ alkoxy, benzyloxy, $C_1$-$C_4$ acyl, benzyl, piperidyl, tert-butoxycarbonyl-substituted piperidyl and methoxyformyl.
$R_2$ and $R_3$ may each independently be hydrogen atom or halogen.
A is an oxygen atom, a sulfur atom, an amine group or $C_1$-$C_6$ alkyl substituted amine group or a carbon atom.

Preferably, in the [1, 2, 4] triazolo [4, 3- b] [1, 2, 4] triazine compounds having a structure of formula (I), pharmaceutically acceptable salts, prodrugs, hydrates or solvates thereof in the present invention, $R_2$ and $R_3$ are each

independently hydrogen atom.

Preferably, in the [1, 2, 4] triazolo [4, 3- b] [1, 2, 4] triazine compounds having a structure of formula (I), pharmaceutically acceptable salts, isomers, prodrugs, hydrates or solvates thereof in the present invention, $R_2$ and $R_3$ are each independently fluorine atom.

Preferably, in the [1, 2, 4] triazolo [4, 3- b] [1, 2, 4] triazine compounds having a structure of formula (I), pharmaceutically acceptable salts, prodrugs, hydrates or solvates thereof in the present invention, $R_2$ and $R_3$ are each independently hydrogen atom or fluorine atom; $R_4$ is substituted or unsubstituted phenyl, quinolyl or indolyl, the substituents are 1- 4 group (s) selected from the group consisting of halogen, $C_1$- $C_6$ linear or branched alkyl, nitro group, amino, hydroxyl, hydroxymethyl, trifluoromethyl, trifluoromethoxy, carboxyl, $C_1$- $C_4$ alkoxy, benzyloxy, $C_1$- $C_4$ acyl, benzyl, piperidyl, tert- butoxycarbonyl- substituted piperidyl and methoxyformyl.

Preferably, in the [1, 2, 4] triazolo [4, 3- b] [1, 2, 4] triazine compounds having a structure of formula (II), pharmaceutically acceptable salts, prodrugs, hydrates or solvates thereof in the present invention, A is independently an oxygen atom, a sulfur atom, an amine group or a carbon atom, $R_2$ and $R_3$ are each independently a hydrogen atom.

Preferably, in the [1, 2, 4] triazolo [4, 3- b] [1, 2, 4] triazine compounds having a structure of formula (II), pharmaceutically acceptable salts, prodrugs, hydrates or solvates thereof in the present invention, A is independently an oxygen atom, a sulfur atom, an amine group or a carbon atom, $R_2$ and $R_3$ are each independently a hydrogen atom; $R_4$ is substituted or unsubstituted phenyl, quinolyl or indolyl, the substituents are 1- 4 group (s) selected from the group consisting of halogen, $C_1$- $C_6$ linear or branched alkyl, nitro group, amino, hydroxyl, hydroxymethyl, trifluoromethyl, triftuoromethoxy, carboxyl, $C_1$- $C_4$ alkoxy, benzyloxy, $C_1$- $C_4$ acyl, benzyl, piperidyl, tert- butoxycarbonyl- substituted piperidyl and methoxyformyl.

Preferably, the [1, 2, 4] triazolo [4, 3- b] [1, 2, 4] triazine compounds, pharmaceutically acceptable salts, prodrugs, hydrates or solvates thereof in the present invention are the compounds in the following Table 1:

Table 1

| No. | Name | Structure |
|---|---|---|
| I-1 | 3-(6-quinolylmethyl)-6-phenyl-[1,2,4]triazolo[4,3-b][1,2,4]triazine | 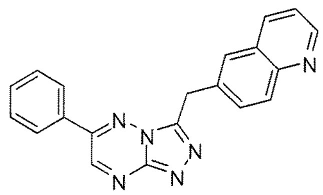 |
| I-2 | 3-(6-quinolylmethyl)-6-(4-benzyloxyphenyl)-[1,2,4]triazolo[4,3-b][1,2,4]triazine | 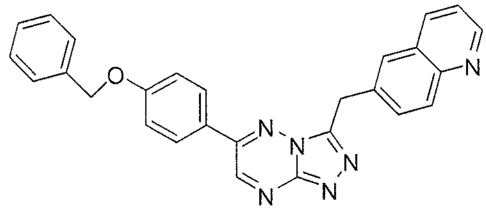 |
| I-3 | 3-(6-quinolylmethyl)-6-(2-thienyl)-[1,2,4]triazolo[4,3-b][1,2,4]triazine | 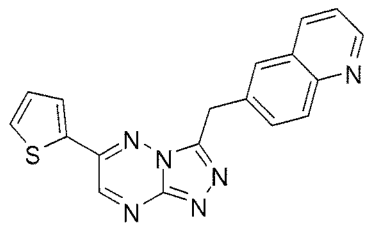 |
| I-4 | 3-(6-quinolylmethyl)-6-(3,4-dichlorophenyl)-[1,2,4]triazolo[4,3-b][1,2,4]triazine | 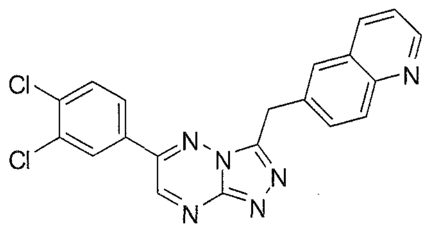 |

(continued)

| No. | Name | Structure |
|---|---|---|
| I-5 | 3-(6-quinolylmethyl)-6-[(1-methyl)-4-pyrazoly1]-[1,2,4] triazolo[4,3-b][1,2,4]triazine | 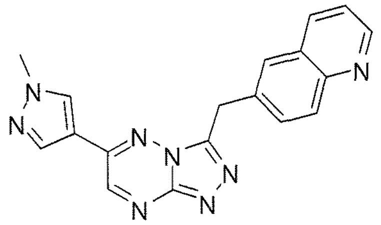 |
| I-6 | 3-(6-quinolylmethyl)-6-[(1-ethyl)-4-pyrazolyl]-[1,2,4] triazolo[4,3-b][1,2,4]triazine | 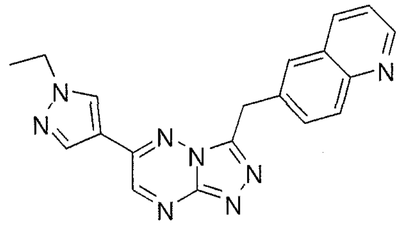 |
| I-7 | 3-(6-quinolylmethyl)-6-[(1-benzyl)-4-pyrazoly1]-[1,2,4] triazolo[4,3-b][1,2,4]triazine | 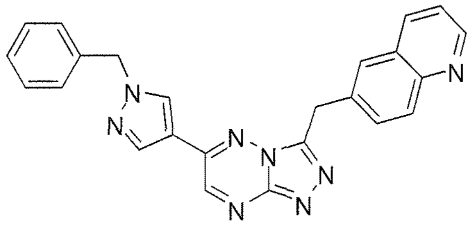 |
| I-8 | 3-(6-quinolylmethyl)-6-(3-quinolyl)-[1,2,4]triazolo[4,3-b] [1,2,4]triazine | 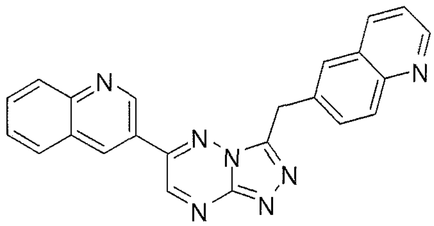 |
| I-9 | 3-(3-indolylmethyl)-6-[(1-propyl)-4-pyrazolyl]-[1,2,4] triazolo[4,3-b][1,2,4]triazine | 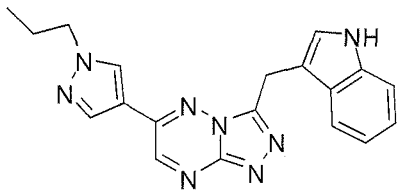 |
| I-10 | 3-(3-indolylmethyl)-6-(4-bromophenyl)-[1,2,4] triazolo [4,3-b][1,2,4]triazine | 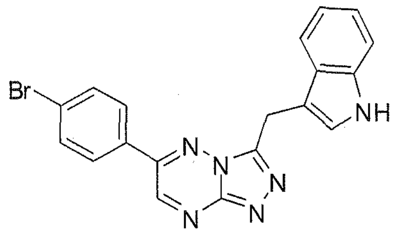 |
| I-11 | 3-(3-indolylmethyl)-6-(3-hydroxyphenyl)-[1,2, 4]triazolo [4,3-b][1,2,4]triazine | 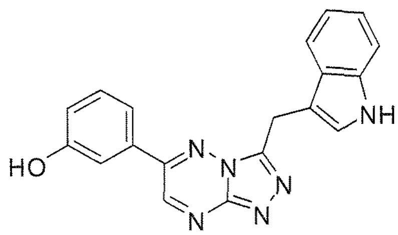 |

(continued)

| No. | Name | Structure |
|---|---|---|
| I-12 | 3-(6-quinolylmethyl)-6-[(1-propyl)-4-pyrazolyl ]-[1,2,4] triazolo[4,3-b][1,2,4]triazine | 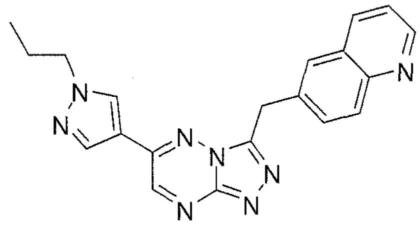 |
| I-13 | 3-(6-quinolylmethyl)-6-[-(1-tert-butoxycarbo nyl-4-piperidyl)-4-pyrazolyl]-[1,2,4]triazolo[4, 3-b][1,2,4] triazine | 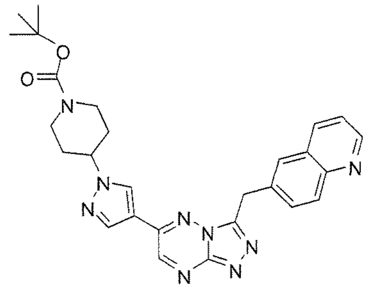 |
| I-14 | 3-(6-quinolylmethyl)-6-[1-(4-piperidyl)-4-pyra zolyl]-[1,2,4]triazolo[4,3-b][1,2,4]triazine | 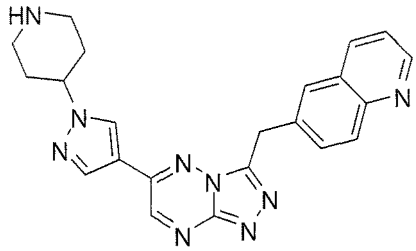 |
| I-15 | 3-(6-quinolylmethyl)-6-(3-nitrophenyl)-[1,2,4]t riazolo [4,3-b][1,2,4]triazine | 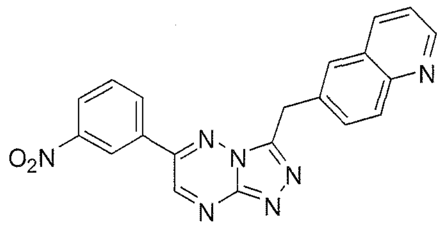 |
| I-16 | 3-(6-quinolylmethyl)-6-(4-chlorophenyl)-[1,2,4]triazolo [4,3-b][1,2,4]triazine | 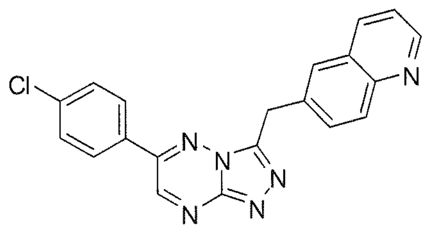 |
| I-17 | 3-(6-quinolylmethyl)-6-(4-bromophenyl)-[1,2,4]triazolo [4,3-b][1,2,4]triazine | 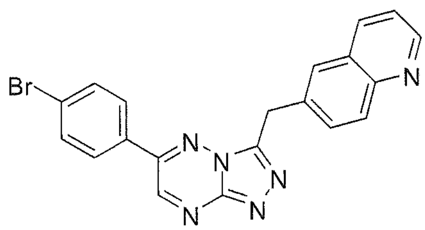 |

(continued)

| No. | Name | Structure |
|---|---|---|
| I-18 | 3-(3-indolylmethyl)-6-(4-benzyloxyphenyl)-[1,2,4] triazolo[4,3-b][1,2,4]triazine | 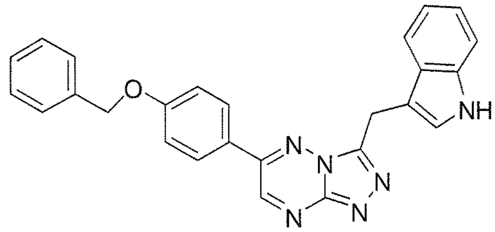 |
| I-19 | 3-[(6-quinolyl)difluoromethyl]-6-[(1-methyl)-4-pyrazolyl]-[,2,4]triazolo[4,3-b][1,2,4]triazine | 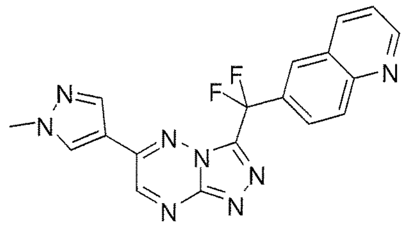 |
| I-20 | 3-[(6-quinolyl)difluoromethyl]-6-[1-(1-tert-butoxycarbonyl-4-piperidyl)-4-pyrazolyl]-[1,2,4]tr iazolo [4,3-b][1,2,4]triazine | 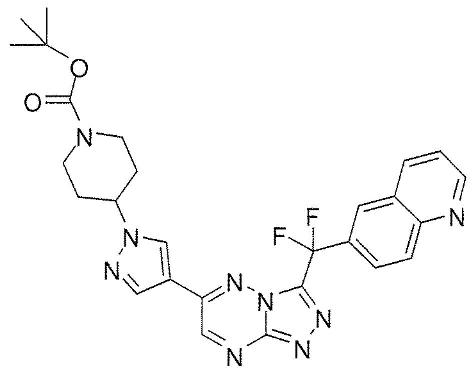 |
| I-21 | 3-[(6-quinolyl)difluoromethyl]-6-[1-(4-piperidyl)-4-pyrazolyl]-[1,2,4]triazolo[4,3-b][1,2,4]tri azine | 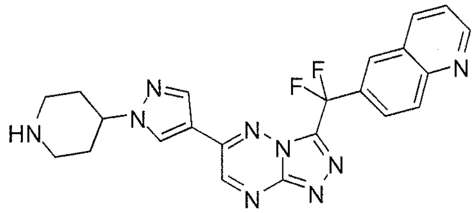 |
| I-22 | 3-[(6-quinolyl)difluoromethyl]-6-phenyl-[1,2,4]triazolo [4,3-b][1,2,4]triazine | 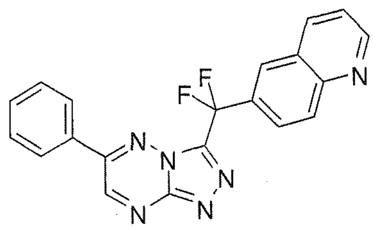 |
| I-23 | 3-[(6-quinolyl)difluoromethyl]-6-(2-thienyl)-[1,2,4] triazolo[4,3-b][1,2,4]triazine | 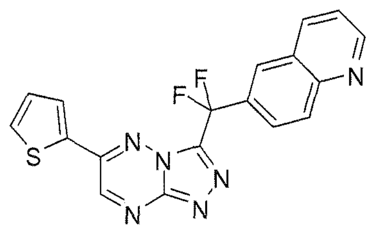 |

(continued)

| No. | Name | Structure |
|---|---|---|
| I-24 | 3-[(6-quinolyl)difluoromethyl]-6-(4-benzyloxyphenyl)-[1,2,4]triazolo[4,3-b][1,2,4]triazine | 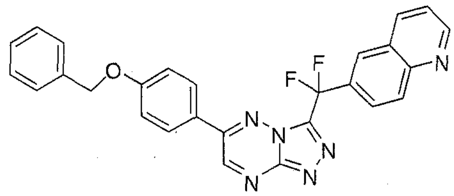 |
| II-1 | 3-[(7-methoxy-4-quinolyl)oxymethyl]-6-(4-fluorophenyl)-[1,2,4]triazolo[4,3-b][1,2,4]triazine | 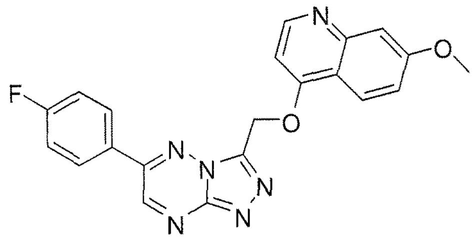 |
| II-2 | 3-[(7-methoxy-4-quinolyl)oxymethyl]-6-(4-chlorophenyl)-[1,2,4]triazolo[4,3-b][1,2,4]triazine | 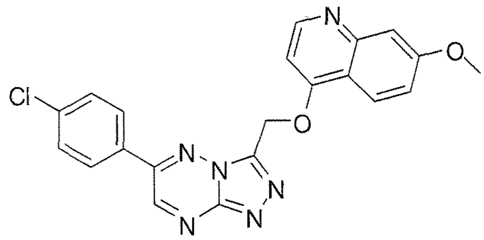 |
| II-3 | 3-[(7-methoxy-4-quinolyl)oxymethyl]-6-(4-bromophenyl)-[1,2,4]triazolo[4,3-b][1,2,4]triazine | 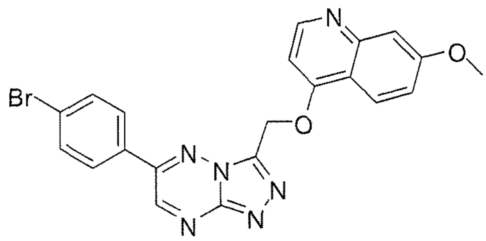 |
| II-4 | 3-[(7-methoxy-4-quinolyl)oxymethyl]-6-(2-thienyl)-[1,2,4]triazolo[4,3-b][1,2,4]triazine | 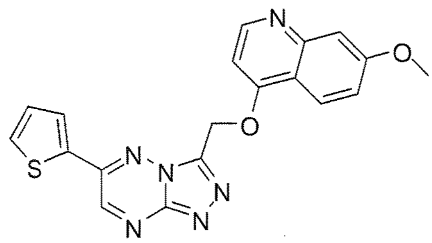 |
| II-5 | 3-[(7-methoxy-4-quinolyl)oxymethyl]-6-(4-methoxyformylphenyl)-[1,2,4]triazolo[4,3-b][1,2, 4] triazine | 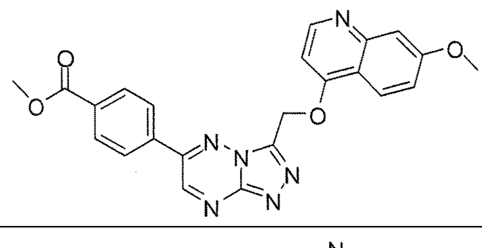 |
| II-6 | 3-[(7-methoxy-4-quinolyl)oxymethyl]-6-(4-nitrophenyl)-[1,2,4]triazolo[4,3-b][1,2,4]triazine | 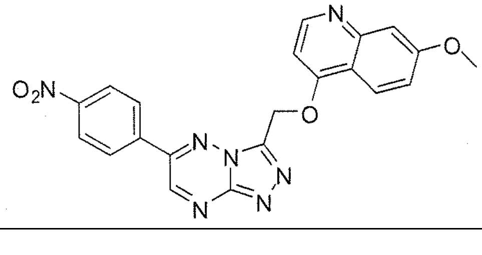 |

(continued)

| No. | Name | Structure |
|---|---|---|
| II-7 | 3-[(7-methoxy-4-quinolyl)oxymethyl]-6-pheny1-[1,2,4] triazolo[4,3-b][1,2,4]triazine | 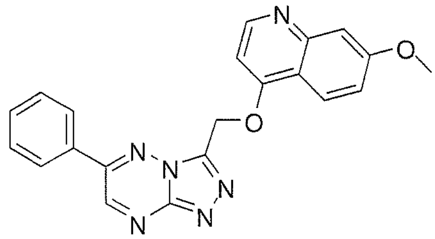 |
| II-8 | 3-[(7-methoxy-4-quinolyl)oxymethyl]-6-(4-benzyloxyphenyl)-[1,2,4]triazolo[4,3-b][1,2,4]tri azine | 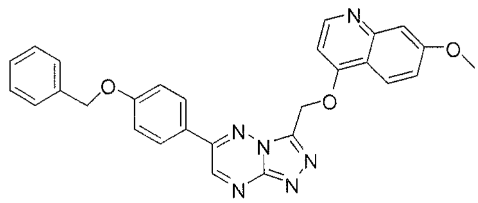 |
| II-9 | 3-[(7-methoxy-4-quinolyl)oxymethyl]-6-(4-methoxyphenyl)-[1,2,4]triazolo[4,3-b][1,2,4]triazi ne | 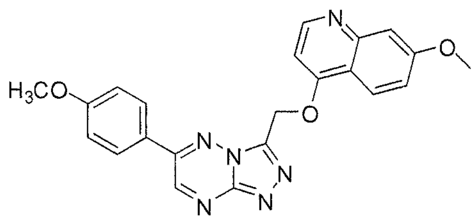 |
| II-10 | 3-[(7-methoxy-4-quinolyl)oxymethyl]-6-(4-hydroxyphenyl)-[1,2,4]triazolo[4,3-b][1,2,4]thiazine | 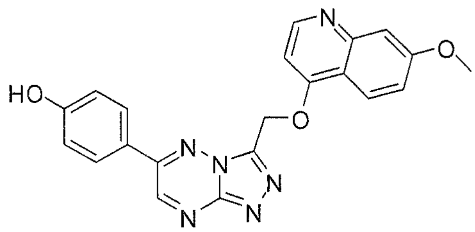 |
| II-11 | 3-[(7-methoxy-4-quinolyl)oxymethyl]-6-(3-methoxyphenyl)-[1,2,4]triazolo[4,3-b][1,2,4]triazine | 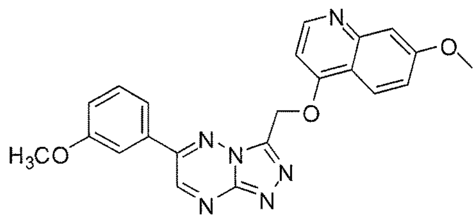 |
| II-12 | 3-[(7-methoxy-4-quinolyl)oxymethyl]-6-(3-hydroxyphenyl)-[1,2,4]triazolo[4,3-b][1,2,4]triazine | 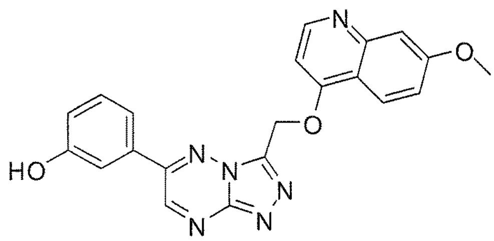 |
| II-13 | 3-[(7-methoxy-4-quinolyl)oxymethyl]-6-(3-nitrophenyl)-[1,2,4]triazolo[4,3-b][1,2,4]triazine | 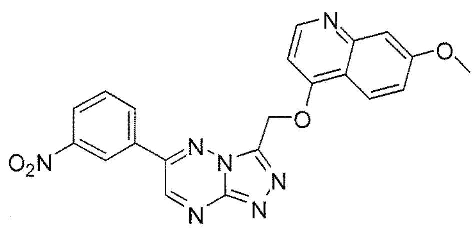 |

(continued)

| No. | Name | Structure |
| --- | --- | --- |
| II-14 | 3-[(6,7-dimethoxy-4-quinolyl)oxymethyl]-6-(4-fluorophenyl)-[1,2,4]triazolo[4,3-b][1,2,4]triazine | 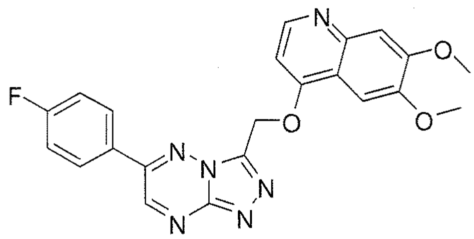 |
| II-15 | 3-[(6,7-dimethoxy-4-quinolyl)oxymethyl]-6-(4-chlorophenyl)-[1,2,4]triazolo[4,3-b][1,2,4]triazine | 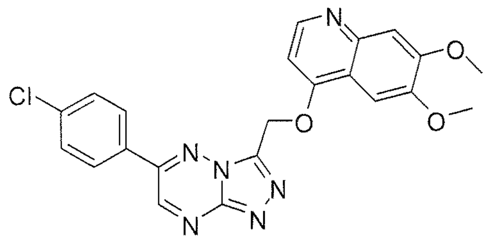 |
| II-16 | 3-[(6,7-dimethoxy-4-quinolyl)oxymethyl]-6-(4-bromophenyl)-[1,2,4]triazolo[4,3-b][1,2,4]triazine | 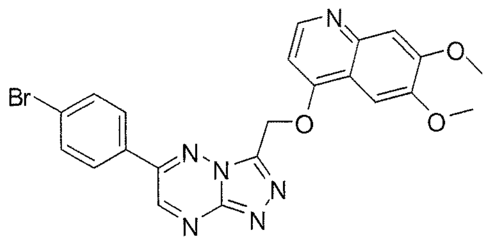 |
| II-17 | 3-[(6,7-dimethoxy-4-quinolyl)oxymethyl]-6-phenyl-[1,2,4]triazolo[4,3-b][1,2,4]triazine | 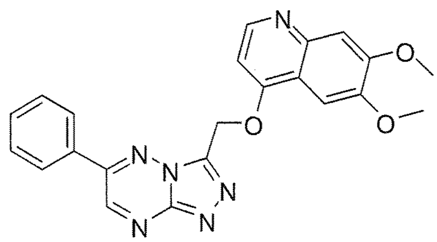 |
| II-18 | 3-[(6,7-dimethoxy-4-quinolyl)oxymethyl]-6-(4-methoxyformylphenyl)-[1,2,4]triazolo[4,3-b][ 1,2,4] triazine | 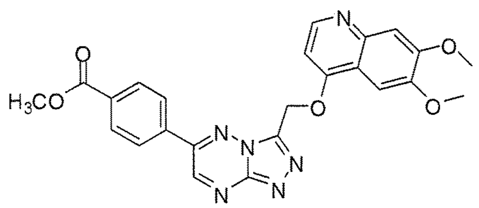 |
| II-19 | 3-[(6,7-dimethoxy-4-quinolyl)oxymethyl]-6-(4-benzyloxyphenyl)-[1,2,4]triazolo[4,3-b][1,2,4 ]triazine | 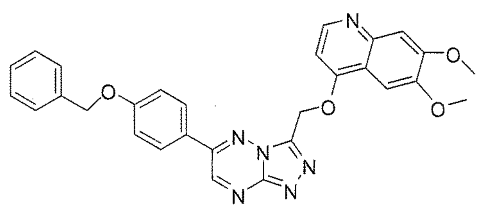 |
| II-20 | 3-[(7-methoxy-4-quinolyl)oxymethyl]-6-(3,4-dichlorophenyl)-[1,2,4]triazolo[4,3-b][1,2,4]triazine | 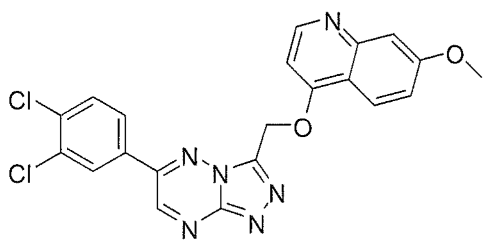 |

(continued)

| No. | Name | Structure |
|---|---|---|
| II-21 | 3-(4-quinolyloxymethyl)-6-(4-fluorophenyl)-[1,2,4] triazolo[4,3-b][1,2,4]triazine | 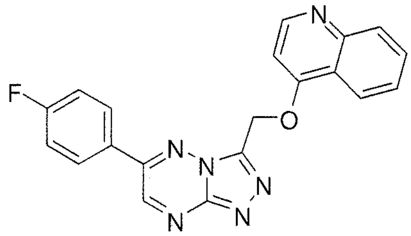 |
| II-22 | 3-(4-quinolyloxymethyl)-6-phenyl-[1,2,4]triazolo[4,3-b] [1,2,4]triazine | 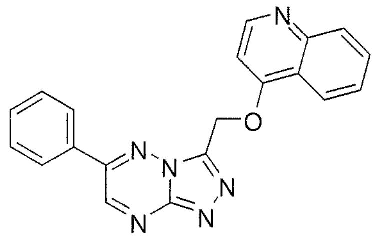 |
| II-23 | 3-(4-quinolyloxymethyl)-6-(4-methoxyphenyl)-[1,2,4] triazolo[4,3-b][1,2,4]triazine | 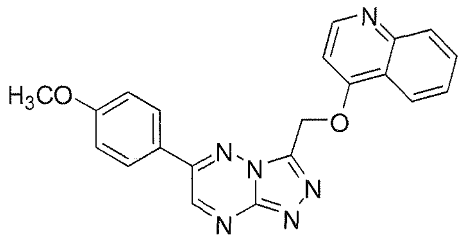 |
| II-24 | 3-(4-quinolyloxymethyl)-6-(4-hydroxyphenyl)-[1,2,4] triazolo[4,3-b][1,2,4]triazine | 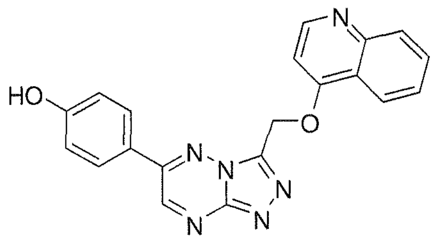 |
| II-25 | 3-(4-quinolyloxymethyl)-6-(4-methoxyformylphenyl)-[1,2,4]triazolo[4,3-b][1,2,4] triazine | 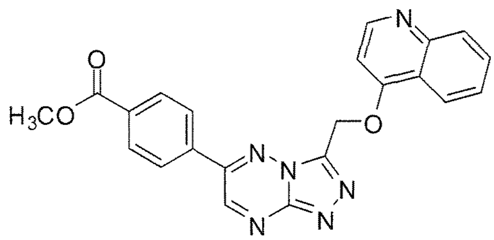 |
| II-26 | 3-(4-quinolyloxymethyl)-6-(3-methoxyphenyl)-[1,2,4] triazolo[4,3-b][1,2,4]triazine | 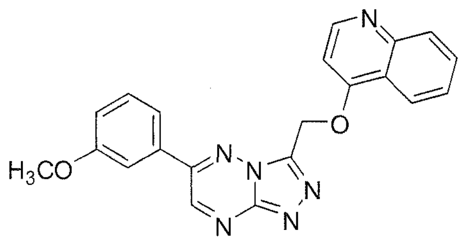 |

(continued)

| No. | Name | Structure |
|---|---|---|
| II-27 | 3-(4-quinolyloxymethyl)-6-(3-hydroxyphenyl)-[1,2,4] triazolo[4,3-b][1,2,4]triazine | 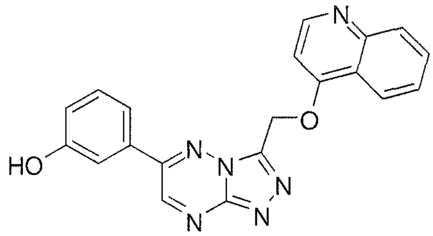 |
| II-28 | 3-(4-quinolyloxymethyl)-6-(3-nitrophenyl)-[1,2,4] triazolo[4,3-b][1,2,4]triazine | 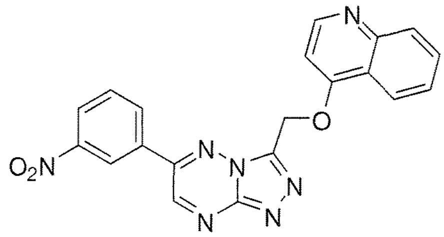 |
| II-29 | 3-[(7-methoxy-4-quinolyl)oxymethyl]-6-[(1-methyl)-4-pyrazolyl]-[1,2,4]triazolo[4,3-b][1,2,4 ]triazine | 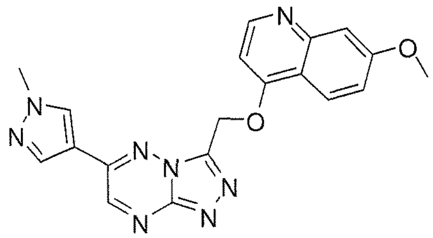 |

The pharmaceutically acceptable salts of the compounds of the present invention are prepared by a direct salification between the free base of the compound with inorganic or organic acids. The inorganic or organic acids may be selected from the group consisting of hydrochloric acid, hydrobromic acid, hydrofluoric acid, sulfuric acid, phosphoric acid, nitric acid, formic acid, acetic acid, picric acid, citric acid, maleic acid, methane sulfonic acid, trifluoromethane sulfonic acid, ethane sulfonic acid, p-toluenesulfonic acid and the like.

The present invention also relates to a method of preparing the [1, 2, 4] triazolo [4, 3- b] [1, 2, 4] triazine compounds having a structure of formula (I) or (II), comprising the following steps:

starting from an acetyl compound via oxidizing, aldehyde protecting, synthesizing 3- methylthio- 1, 2, 4- triazine ring, oxidizing, replacing methylsulfinyl group or methanesulfonyl group with hydrazine, condensing to give an acethydrazide, and cyclizing to give the target compound.

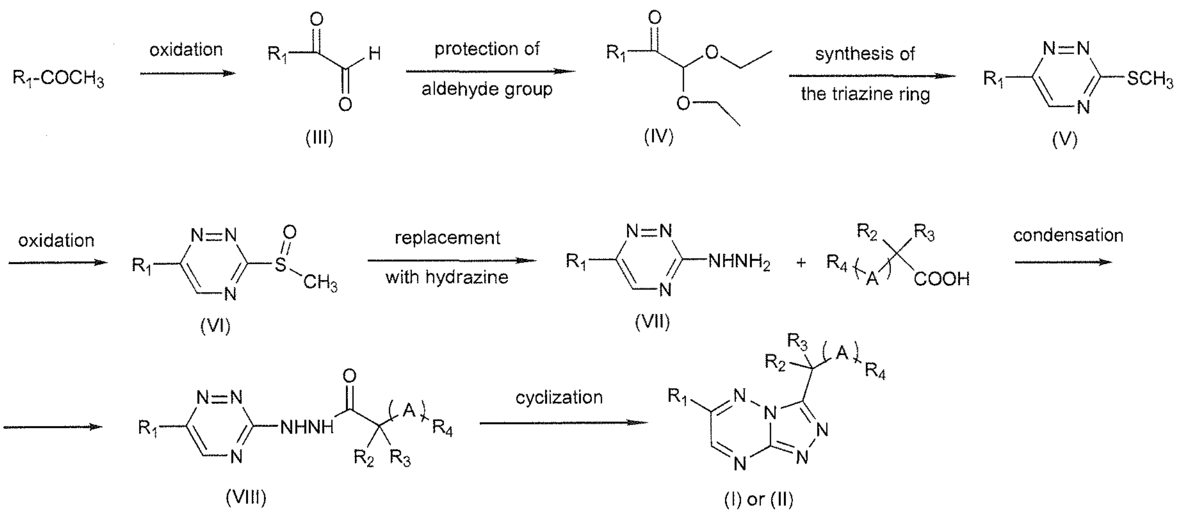

Specifically, the preparation method comprises:

oxidizing an acetyl compound by selenium dioxide or hydrobromic acid and dimethyl sulfoxide, to give a compound (III);
reacting the compound (III) and triethyl ortho-formate in the presence of p-toluenesulfonic acid as a catalyst, to synthesize an acetal (IV);
reacting the compound (IV) and thiosemicarbazide via acid catalyzed condensation and methylation to give a compound (V);
oxidizing the methylthio of the compound (V) by m-chloroperoxybenzoic acid, hydrogen peroxide, potassium permanganate, manganese dioxide, potassium periodate or potassium dichromate to give a compound (VI);
substituting the compound (VI) with hydrazine as a nucleophilic reagent to give compound (VII);
condensing the compound (VII) and a carboxylic acid compound by dicyclohexyl- carbodiimide or 1- (3- dimethylaminopropyl)- 3- ethylcarbodiimide hydrochloride, or reacting the compound (VII) with an acyl chloride prepared from the carboxylic acid to synthesize a hydrazide compound (VIII) ;
cyclizing the hydrazide compound (VIII) to give the target compound (I) or (II);
wherein, the cyclizing reagent is phosphorous oxychloride, formic acid, acetic acid, methane sulfonic acid, trifluoromethane sulfonic acid, p-toluenesulfonic acid or ethane sulfonic acid.

The preparation method of [1, 2, 4] triazolo [4, 3- b] [1, 2, 4] triazines of the present invention has the advantages that the reaction conditions are mild, raw materials are abundance and easy to get, and the operation and post- process are simple.

The present invention also relates to a pharmaceutical composition for the prevention and/or treatment of diseases associated with c- Met abnormality comprising the [1, 2, 4] triazolo [4, 3- b] [1, 2, 4] triazine compounds of formula (I) or (II), or pharmaceutically acceptable salts thereof, and at least one pharmaceutically acceptable carrier, which may be used for *in- vivo* prevention of the diseases associated with c- Met abnormality. The pharmaceutical compositions can be formulated into various forms depending on the different routes of administration.

The present invention also relates to a use of the [1, 2, 4] triazolo [4, 3- b] [1, 2, 4] triazine compounds of formula (I) or (II) as protein tyrosine kinase inhibitors, especially as a c- Met inhibitors, in the preparation of medicaments for the prevention and/or treatment of a disease associated with c- Met abnormality.

Among others, the disease associated with c-Met abnormality is tumor.

The tumor includes lung cancer, breast cancer, colon cancer, prostate cancer, pancreatic cancer, gastric cancer, liver cancer, ovarian cancer, renal cancer, neurospongioma, melanoma, pancreatic cancer, head and neck cancer, bladder cancer, cervical cancer, bile duct cancer, nasopharyngeal cancer, thyroid cancer, osteosarcoma, synovial sarcoma, rhabdomyosarcoma, fibrosarcoma, leiomyosarcoma, multiple myeloma, lymphoma, leukemia and the like.

## Brief Descriptions of the Drawings

Figure 1 is a graph illustrating the influence of the compounds of the present invention to the phosphorylation of the TPR-Met in NIH3T3/TPR-Met cells;
Figure 2 is a graph illustrating the inhibition of the compounds of the present invention against the growth of the human neurospongioma U-87MG xenograft in nude mice;
Figure 3 is a graph illustrating the influence of the compounds of the present invention to the weight of nude mice bearing the human neurospongioma U-87MG.

## Description of the Preferred Embodiments

The present invention will be further described in the following Examples. These Examples are merely used to illustrate the present invention but not to limit the present invention in any way.

Example 1 : 3-(6-quinolylmethyl)-6-phenyl-[1,2,4]triazolo[4,3-b][1,2,4]triazine (I-1)

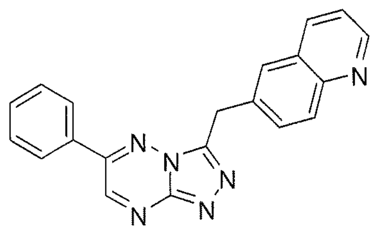

Step 1: 2-oxo-2-phenylacetaldehyde(III-1)

14 g of selenium dioxide, 62 ml of dioxane and 2.5 ml of water were added to a 100 ml round bottomed flask, and stirred at 50°C until the selenium dioxide was dissolved, followed by addition of 15 g of acetophenone. The reaction liquid was kept for 4 hours under reflux, then cooled, filtered and treated under reduced pressure to remove the solvent. The residue was purified by column chromatography (ethyl acetate: petroleum ether = 1: 5), to obtain the product III- 1 as white solid (9.8 g, yield 58.5%) . Mp 120- 122°C. $^1$H- NMR (300Hz, $CDCl_3$) $\delta$: 9.67 (s, 1H), 8.04- 8.15 (m, 2H), 7.53- 7.66 (m, 3H) .

Step 2: 2,2-diethoxyacetophenone (IV-1)

5.58 g of 2- oxo- 2- phenylacetaldehyde (III- 1), 12.34 g of triethyl orthoformate, and 400 mg of p- toluenesulfonic acid were dissolved in 40 ml of methylene chloride with stirring under reflux for 1 hour. Then the mixture was cooled to room temperature and treated under reduced pressure to remove the solvent. The residue was purified by column chromatography (ethyl acetate: petroleum ether = 1: 50) to give a colorless oil IV- 1 (5.83 g, yield 67.21%) . $^1$H- NMR (300Hz, $CDCl_3$) $\delta$: 8.16 (d, *J* = 6.9 Hz, 2H), 7.41- 7.55 (m, 3H), 5.27 (s, 1H), 3.61- 3.78 (m, 4H), 1.26 (t, *J* = 6.9 Hz, 6H) .

Step 3: 3-methylthio-6-phenyl-1,2,4-triazine (V-1)

5.5 g of 2, 2- diethoxy- acetophenone (IV- 1), 2.4 g of thiosemicarbazide, 25 mg of p- toluenesulfonic acid and 80 ml of ethanol were added to a 250 ml round bottomed flask and heated to 60 °C. After 4 hours, the reaction liquid was cooled to room temperature and 3.75 g of methyl iodide was added therein. Then the mixture was stirred at room temperature overnight and treated under reduced pressure on the next morning to remove the solvent. After that, to the residue was added 50 ml of glacial acetic acid, the resulted mixture was kept under reflux for 3 hours and treated under reduced pressure to remove the solvent. The residue was purified by column chromatography (ethyl acetate: petroleum ether = 1: 40) to give a yellowish solid V- 1 (1.91 g, yield 35.5%) . $^1$H- NMR (300Hz, $CDCl_3$) $\delta$: 8.78 (s, 1H), 8.06 (m, 2H), 7.56 (m, 3H), 2.73 (s, 3H) .

Step 4: 3-methylsulfinyl-6-phenyl-1,2,4-triazine (VI-1).

1.88 g of 3- methylthio- 6- phenyl- 1, 2, 4- triazine (V- 1) was dissolved in 30 ml of methylene chloride, the mixture was cooled in an ice water bath, then 1.6 g m- chloroperoxybenzoic acid solution in 30 ml methylene chloride was added dropwise. After the dropwise was completed, the mixture was raised to room temperature, and 50 ml of water and 10 ml of saturated sodium thiosulfate solution were added therein. The resulted liquid was separated, extracted with methylene chloride, washed with saturated sodium bicarbonate solution, dried over anhydrous sodium sulfate, filtered, concentrated and dried to give a white solid VI- 1 (1.91 g, yield 94.2%) . $^1$H- NMR (300Hz, $CDCl_3$) $\delta$: 7.18 (s, 1H), 8.16 (s, 2H), 7.63 (m, 3H), 3.14 (s, 3H) .

Step 5: 3-hydrazino-6-phenyl-1,2,4-triazine (VII-1)

1.81 g of 3- methylsulfinyl- 6- phenyl- 1, 2, 4- triazine (VI- 1) and 30 ml of tetrahydrofuran were added to a 100 ml round bottomed flask and 2 ml of a 85% solution of hydrazine hydrate was added dropwise therein. The mixture was stirred at room temperature for 1 hour and treated under reduced pressure to remove the solvent, then the residue was washed with water and dried to give a yellow solid VII- 1 (1.31 g, yield 84.7%) . $^1$H- NMR (300Hz, DMSO- $d_6$) $\delta$: 8.85 (s, 2H), 8.01 (d, *J* = 8.1 Hz, 2H), 7.42- 7.49 (m, 3H), 4.43 (s, 2H) .

Step 6: 6-phenyl-3-(6-quinolylacetylhydrazino)-1,2,4-triazine (VIII-1)

100 mg of 6- quinoline acetic acid, 100 mg of 3- hydrazino- 6- phenyl- 1, 2, 4- triazine (VII- 1), 122 mg of N-ethyl- N'- (3- dimethylaminopropyl) carbodiimide hydrochloride, 70 mg of 1- hydroxybenzotriazole, 195 mg of N, N-diisopropyl ethyl amine and 8 ml of N, N- dimethyl formamide were added to a 25 ml round bottomed flask, then stirred at room temperature overnight. The solvent was removed under reduced pressure on the next morning and the residue was recrystallized from ethyl acetate to give a white solid VIII- 1 (113 mg, yield 61.7%) . $^1$H- NMR (300Hz, DMSO- $d_6$) δ: 10.39 (s, 1H), 9.71 (br, 1H), 8.97 (s, 1H), 8.87 (dd, *J* = 1.5, 4.2 Hz, 1H), 8.34 (d, *J* = 7.5 Hz, 1H), 7.92- 8.05 (m, 4H), 7.77 (dd, *J* = 2.1, 8.4 Hz), 7.46- 7.55 (m, 4H), 3.79 (s, 2H) .

Step 7: 3-(6-quinolylmethyl)-6-phenyl-[1,2,4]triazolo[4,3-b][1,2,4]triazine (I-1)

100 mg of 6- phenyl- 3- (6- quinolylacetylhydrazino)- 1, 2, 4- triazine (VIII- 1) and 15 ml of glacial acetic acid were added to a 25 ml round bottomed flask, the mixture was stirred and heated under reflux for 5 hours, and treated under reduced pressure to remove the solvent. The resulted residue was purified by column chromatography (dichloromethane: methanol = 25: 1) to give a white solid I- 1 (87 mg, yield 81.2%) . $^1$H- NMR (300Hz, DMSO- $d_6$) δ: 9.35 (s, 1H), 8.85 (dd, *J* = 1.5, 4.2 Hz, 1H), 8.32 (d, *J* = 5.3 Hz), 8.17 (m, 2H), 7.99 (m, 2H), 7.82 (dd, *J* = 1.5, 5.4 Hz, 1H), 7.62 (m, 3H), 7.51 (dd, *J* = 4.2, 8.7 Hz, 1H) .

The target compounds were prepared with similar methods.

Example 2:

3-(6-quinolylmethyl)-6-(4-benzyloxyphenyl)-[1,2,4]triazolo[4,3-b][1,2,4]triazine (I-2)

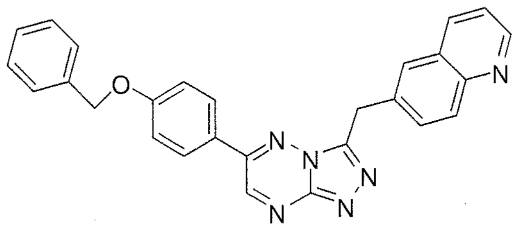

Step 1: 2-oxo-2-(4-benzyloxyphenyl)acetaldehyde(III-2)

21.82 g of 4- benzyloxy- acetophenone was dissolved in 200 ml of DMSO, and 33 ml of 48% hydrobromic acid was added dropwise at room temperature under stirring. After the dropwise was completed, the mixture was stirred and heated to 70 °C for 8 hours, and then the reaction liquid was cooled and poured into 200 ml of ice- water. After filtration, the filter cake was washed with water and dried to give a white solid III- 2 (21.47 g, yield 92.7%) . $^1$H- NMR (300Hz, DMSO- $d_6$) δ: 8.05 (d, *J* = 9.0 Hz, 2H), 7.34- 7.48 (m, 5H), 7.13 (d, *J* = 9.0 Hz, 2H), 5.22 (s, 2H) .

Step 2: 2,2-diethoxy-1-(4-benzyloxy-phenyl)-ethanone (IV-2)

All used starting materials, reagents and preparation were the same as those in step 2 of Example 1, except that the 2- oxo- 2- phenylacetaldehyde (III- 1) was replaced by 2- oxo- 2- (4- benzyloxyphenyl}- acetaldehyde (III- 2), to give a yellowish oil IV- 2. $^1$H- NMR (300Hz, $CDCl_3$) δ: 8.16 (d, *J* = 8.7 Hz, 2H), 7.32- 7.44 (m, 5H), 7.00 (d, *J* = 8.7 Hz, 2H), 5.23 (s, 1H), 5.13 (s, 2H), 3.59- 3.80 (m, 4H), 1.24 (t, *J* = 7.2 Hz, 6H) .

Step 3: 3-methylthio-6-(4-benzyloxyphenyl)-1,2,4-triazine (V-2)

All used starting materials, reagents and preparation were the same as those in step 3 of Example 1, except that the 2, 2- diethoxyacetophenone (IV- 1) was replaced by 2, 2- diethoxy- 1- (4- benzyloxyphenyl)- ethanone (IV- 2), to give a yellowish solid V- 2. $^1$H- NMR (300Hz, $CDCl_3$) δ: 8.73 (s, 1H), 8.01 (d, *J* = 9.0 Hz, 2H), 7.35- 7.48 (m, 5H), 7.13 (d, *J* = 9.0 Hz, 2H), 5.16 (s, 2H), 2.72 (s, 3H) .

Step 4: 3-methylsulfinyl-6-(4-benzyloxy-phenyl)-1,2,4-triazine (VI-2)

All used starting materials, reagents and preparation were the same as those in step 4 of Example 1, except that the 3- (methylthio)- 6- phenyl- 1, 2, 4- triazine (V- 1) was replaced by 3- (methylthio)- 6- (4- benzyloxyphenyl)- 1, 2,4- triazine (V- 2), to give a yellowish solid VI- 2.

Step 5: 3-hydrazino-6-(4-benzyloxyphenyl)-1,2,4-triazine (VII-2)

All used starting materials, reagents and preparation were the same as those in step 5 of Example 1, except that the 3- methylsulfinyl- 6- phenyl- 1, 2, 4- triazine (VI- 1) was replaced by 3- (methylsulfinyl)- 6- (4- benzyloxy- phenyl)- 1, 2, 4- triazine (VI- 2), to give a yellowish solid VII- 2. $^1$H- NMR (300Hz, DMSO- $d_6$) δ: 8.82 (s, 1H), 8.74 (br, 1H), 7.96 (d, *J* = 9.0 Hz, 2H), 7.34- 7.49 (m, 5H), 7.15 (d, *J* = 9.0 Hz, 2H), 5.18 (s, 2H), 4.41 (br, 2H) .

Step 6: 6-(4-benzyloxy-phenyl)-3-(6-quinolylacetylhydrazino)-1,2,4-triazine (VIII-2)

All used starting materials, reagents and preparation were the same as those in step 6 of Example 1, except that the 3- hydrazino- 6- phenyl- 1, 2, 4- triazine (VII- 1) was replaced by 3- hydrazino- 6- (4- benzyloxyphenyl)- 1, 2, 4- triazine (VII- 2), to give a yellow solid VIII- 2. $^1$H- NMR (300Hz, DMSO- $d_6$) δ: 10.39 (s, 1H), 9.62 (br, 1H), 8.94 (s, 1H), 8.88 (d, *J* = 2.4 Hz, 1H), 8.35 (d, *J* =8.4Hz, 1H), 7.99 (d, *J* =8.7 Hz, 3H), 7.93 (s, 1H), 7.76 (d, *J* = 8.1 Hz, 1H), 7.34- 7.55 (m, 6H), 7.17 (d, *J* = 8.7 Hz, 2H), 5.19 (s, 2H), 3.79 (s, 2H) .

Step 7: 3-(6-quinolylmethyl)-6-(4-benzyloxyphenyl)-[1,2,4]triazolo[4,3-b][1,2,4]triazine (1-2)

All used starting materials, reagents and preparation were the same as those in step 7 of Example 1, except that the 6- phenyl- 3- (6- quinolylacetylhydrazino)- 1, 2, 4- triazine (VIII- 1) was replaced by 6- (4- benzyloxy- phenyl)- 3- (6- quinolylacetylhydrazino)- 1, 2, 4- triazine (VIII- 2), to give a white solid 1- 2. $^1$H- NMR (300Hz, $CDCl_3$) δ: 8.94 (s, 1H), 8.88 (d, *J* = 4.5 Hz, 1H), 8.09 (t, *J*= 6.9, 8.4 Hz, 2H), 7.82- 7.93 (m, 4H), 7.37- 7.49 (m, 6H), 7.15 (d, *J*= 8.7 Hz, 2H), 5.18 (s, 2H), 4.80 (s, 2H) .

**Example 3:** 3-(6-quinolylmethyl)-6-(2-thienyl)[1,2,4]triazolo[4,3-b][1,2,4]triazine **(1-3)**

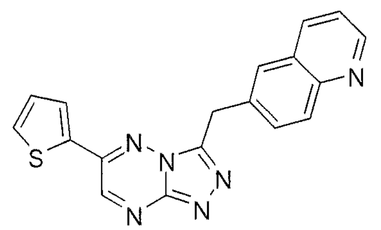

Step 1: 2-oxo-2-(2-thienyl)acetaldehyde **(III-3)**

All used starting materials, reagents and preparation method were the same as those used in Step 1 of Example 1, except that acetophenone was replaced with 2- acetyl thiophene. As a result, 2- oxo- 2- (2- thienyl) acetaldehyde **(III-3)** was obtained as yellow solid.

Step 2: 2,2-diethoxy-1-(2-thienyl)ethanone **(IV-3)**

All used starting materials, reagents and preparation method were the same as those used in Step 2 of Example 1, except that 2- oxo- 2- phenylacetaldehyde **(III- 1)** was replaced with 2- oxo- 2- (2- thienyl) acetaldehyde **(III- 3) .** As a result, compound IV- 3 was obtained as yellowish oil.

$^1$H- NMR (300Hz, $CDCl_3$) δ: 8.06 (d, *J* = 5.4 Hz, 1H), 7.66 (dd, *J* = 0.9, 4.8 Hz, 1H), 7.14 (m, 1H), 5.12 (s, 1H), 3.63- 3.78 (m, 4H), 1.23- 1.28 (m, 6H) .

Step 3: 3-methylthio-6-(2-thienyl)-1,2,4-triazine **(V-3)**

All used starting materials, reagents and preparation method were the same as those used in Step 3 of Example 1, except that 2, 2- diethoxyacetophenone **(IV- 1)** was replaced with 2, 2- diethoxy- 1- (2- thienyl) ethanone (**IV- 3**) . As a result, compound V- 3 was obtained as yellowish solid.

$^1$H- NMR (300Hz, $CDCl_3$) δ: 8.70 (s, 1H), 7.68 (dd, *J* = 0.9, 3.9 Hz, 1H), 7.54 (dd, *J* = 0.9, 5.4 Hz, 1H), 7.19

(dd, $J$ = 3.9, 5.4 Hz, 1H), 2.71 (s, 3H) .

Step 4: 3-methylsulfinyl-6-(2-thienyl)-1,2,4 triazine **(VI-3)**

All used starting materials, reagents and preparation method were the same as those used in Step 4 of Example 1, except that 3- methylthio- 6- phenyl- 1, 2, 4- triazine (**V- 1**) was replaced with 3- methylthio- 6- (2- thienyl)- 1, 2, 4- triazine (**V- 3**) . As a result, compound VI- 3 was obtained as yellowish solid.

$^1$H- NMR (300Hz, $CDCl_3$) δ: 9.08 (s, 1H), 7.94 (dd, $J$ = 0.9, 3.9 Hz, 1H), 7.76 (d, $J$= 5.4 Hz, 1H), 7.31 (dd, $J$ = 4.2, 4.8 Hz, 1H), 3.51 (s, 3H) .

Step 5: 3-hydrazino-6-(2-thienyl)-1,2,4-triazine (**VII-3**)

All used starting materials, reagents and preparation method were the same as those used in Step 5 of Example 1, except that 3- methylsulfinyl- 6- phenyl- 1, 2, 4- triazine (**VI- 1**) was replaced with 3- methylsulfinyl- 6- (2- thienyl)- 1, 2, 4- triazine **(VI- 3) .** As a result, compound **VII- 3** was obtained as yellowish solid.

$^1$H- NMR (300Hz, DMSO- $d_6$) δ: 8.88 (s, 2H), 7.72 (d, $J$ = 3.3 Hz, 1H), 7.61 (d, $J$ = 5.4 Hz, 1H), 7.18 (dd, $J$ = 4.2, 4.8 Hz, 1H), 4.41 (br, 2H) .

Step 6: 6-(2-thienyl)-3-(6-quinolylacetylhydrazino)-1,2,4-triazine **(VIII-3)**

All used starting materials, reagents and preparation method were the same as those used in Step 6 of Example 1, except that 3- hydrazino- 6- phenyl- 1, 2, 4- triazine **(VII- 1)** was replaced with 3- hydrazino- 6- (2- thienyl)- 1, 2, 4- triazine **(VII- 3) .** As a result, compound VIII- 3 was obtained as yellowish solid.

$^1$H- NMR (300Hz, DMSO- $d_6$) δ: 9.35 (s, 1H), 8.84 (d, $J$ = 2.4 Hz, 1H), 8.23- 8.30 (m, 2H), 7.92- 7.97 (m, 3H), 7.80 (d, $J$ = 8.1 Hz, 1H), 7.51 (m, 1H), 7.31 (m, 1H), 4.69 (s, 2H) .

Step 7: 3-(6-quinolylmethyl)-6-(2-thienyl)[1,2,4]triazolo[4,3-b][1,2,4]triazine **(1-3)**

All used starting materials, reagents and preparation method were the same as those used in Step 7 of Example 1, except that 6- phenyl- 3- (6- quinolylacetylhydrazino)- 1, 2, 4- triazine **(VIII- 1)** was replaced with 6- (2- thienyl)- 3- (6- quinolylacetylhydrazino)- 1, 2, 4- triazine **(VIII- 3) .** As a result, compound 1- 3 was obtained as white solid.

$^1$H- NMR (300Hz, DMSO- $d_6$) δ: 10.39 (s, 1H), 9.70 (br, 1H), 9.00 (s, 1H), 8.87 (d, $J$ = 4.2 Hz, 1H), 8.34 (d, $J$ = 8.1 Hz, 1H), 7.91- 7.99 (m, 2H), 7.65- 7.79 (m, 3H), 7.54 (dd, $J$ = 4.5, 8.4 Hz, 1H), 7.21 (dd, $J$ = 0.9, 5.4 Hz, 1H) .

**Example 4:**

3-(6-quinolylmethyl)-6-(3,4-dichlorophenyl)[1,2,4]triazolo[4,3-b][1,2,4]triazine (**I-4**)

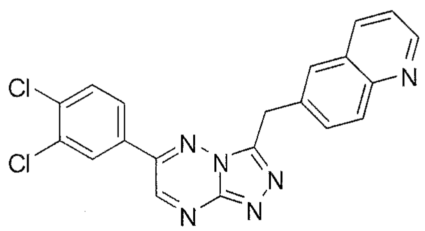

Step 1: 2-(3,4-dichlorophenyl)-2-oxoacetaldehyde (**III-4**)

All used starting materials, reagents and preparation method were the same as those used in Step 1 of Example 1, except that acetophenone was replaced with 3,4-dichloroacetophenone. As a result, compound III-4 was obtained as yellow solid.

Step 2: 2,2-diethoxy-1-(3,4-dichlorophenyl)ethanone (**IV-4**)

All used starting materials, reagents and preparation method were the same as those used in Step 2 of Example

1, except that 2- oxo- 2- phenylacetaldehyde (**III- 1**) was replaced with 2- (3, 4- dichlorophenyl)- 2- oxoacetaldehyde (**III- 4**) . As a result, compound IV- 4 was obtained as yellowish oil.

$^1$H- NMR (300Hz, $CDCl_3$) δ: 8.26 (d, *J* = 1.8 Hz, 1H), 8.04 (dd, *J* = 1.8, 8.4 Hz, 1H), 7.54 (d, *J* = 8.4 Hz, 1H), 5.12 (s, 1H), 3.81- 3.60 (m, 4H), 1.25 (t, *J* = 7.2 Hz, 6H) .

Step 3: 3-methylthio-6-(3,4-dichlorophenyl)-1,2,4-triazine (**V-4**)

All used starting materials, reagents and preparation method were the same as those used in Step 3 of Example 1, except that 2, 2- diethoxyacetophenone **(IV- 1)** was replaced with 2, 2- diethoxy- 1- (3, 4- dichlorophenyl) ethanone (**IV- 4**) . As a result, compound V- 4 was obtained as yellowish solid.

$^1$H- NMR (300Hz, DMSO- $d_6$) δ: 8.75 (s, 1H), 8.19 (d, *J* = 2.4 Hz, 1H), 7.99 (dd, *J* = 2.4, 8.4Hz, 1H), 7.64 (d, *J* = 8.4Hz, 1H), 2.73 (s, 3H) .

Step 4: 3-methylsulfinyl-6-(3,4-dichlorophenyl)-1,2,4-triazine **(VI-4)**

All used starting materials, reagents and preparation method were the same as those used in Step 4 of Example 1, except that 3- methylthio- 6- phenyl- 1, 2, 4- triazine (**V- 1**) was replaced with 3- methylthio- 6- (3, 4- dichlorophenyl)- 1, 2, 4- triazine (**V- 4**) . As a result, compound VI- 4 was obtained as yellowish solid.

Step 5: 3-hydrazino-6-(3,4-dichlorophenyl)-1,2,4-triazine **(VII-4)**

All used starting materials, reagents and preparation method were the same as those used in Step 5 of Example 1, except that 3- methylsulfinyl- 6- phenyl- 1, 2, 4- triazine (**VI- 1**) was replaced with 3- methylsulfinyl- 6- (3, 4- dichlorophenyl)- 1, 2, 4- triazine (**VI- 4**) . As a result, compound VII- 4 was obtained as yellowish solid.

$^1$H- NMR (300Hz, DMSO- $d_6$) δ: 9.07 (br, 1H), 8.94 (s, 1H), 8.28 (d, *J* = 2.1 Hz, 1H), 8.05 (dd, *J* = 2.1, 8.7Hz, 1H), 7.79 (d, *J* = 8.7 Hz, 1H), 4.50 (br, 2H) .

Step 6: 6-(3,4-dichlorophenyl)-3-(6-quinolylacetylhydrazino)-1,2,4-triazine **(VIII-4)**

All used starting materials, reagents and preparation method were the same as those used in Step 6 of Example 1, except that 3- hydrazino- 6- phenyl- 1, 2, 4- triazine (**VII- 1**) was replaced with 3- hydrazino- 6- (3, 4- dichlorophenyl)- 1, 2, 4- triazine **(VII- 4) .** As a result, compound VIII- 4 was obtained as yellowish solid.

Step 7: 3-(6-quinolylmethyl)-6-(3,4-dichlorophenyl) [1,2,4]triazolo[4,3-b] [1,2,4]triazine (**I-4**)

All used starting materials, reagents and preparation method were the same as those used in Step 7 of Example 1, except that 6- phenyl- 3- (6- quinolylacetylhydrazino)- 1, 2, 4- triazine (**VIII- 1**) was replaced with 6- (3, 4- dichlorophenyl)- 3- (6- quinolylacetylhydrazino)- 1, 2, 4- triazine (VIII- 4) . As a result, compound 1- 4 was obtained as white solid.

$^1$H- NMR (300Hz, DMSO- $d_6$) δ: 9.39 (s, 1H), 8.87- 8.85 (dd, *J* = 1.8, 4.2 Hz, 1H), 8.41 (d, *J* = 2.1 Hz, 1H), 8.33 (d, *J* = 7.2 Hz, 1H), 8.18- 8.14 (dd, *J* = 2.1, 8.4 Hz, 1H), 8.00- 7.97 (m, 2H), 7.93 (d, *J* = 8.4 Hz, 1H), 7.83- 7.79 (dd, *J* = 1.8, 8.7 Hz, 1H), 7.53- 7.49 (dd, *J* = 8.1, 8.4 Hz, 1H), 4.81 (s, 2H) .

Example 5:

3-(6-quinolylmethyl)-6-[(1-methyl)-4-pyrazolyl][1,2,4]triazolo[4,3-b][1,2,4]triazine (**I-5**)

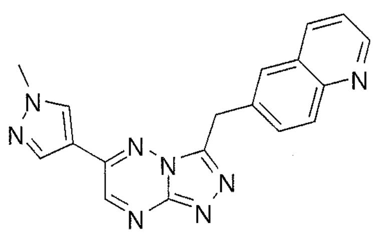

Step 1: 1-methyl-4-iodopyrazole

1H- 4- iodopyrazole (20 g), potassium carbonate (28.45 g) and acetone (120 mL) were added into a round bottomed flask (250 mL) and agitated for 10 min. Then iodomethane (17.56 g) was added and the mixture was agitated at room temperature over night. After that, the mixture was filtrated and concentrated, the residue was recrystallized from n- hexane to prodce 1- methyl- 1H- 4- iodopyrazole (15 g) as white acicular crystal.
$^1$H- NMR (300Hz, $CDCl_3$) δ: 7.48 (s, 1H), 7.40 (s, 1H), 3.92 (s, 3H) .

Step 2: 1-[(1-methyl)-4-pyrazolyl]ethanone

1- methyl- 4- iodopyrazole (10 g), vinyl- n- butyl ether (24 g), palladium acetate (396 mg), 1, 3- bi (diphenyl phosphine) propane (988 mg), sodium carbonate (12.7 g) and n- butylalcohol (100 mL) were added in a round bottomed flask (250 mL) . The mixture was refluxed under an argon atmosphere for 4 h, cooled to room temperature, filtrated and treated under reduced pressure to remove solvent thereof. The residue was purified by column chromatography (dichloromethane: methanol=50: 1) to get 1- [(1- methyl)- 4- pyrazolyl] ethanone (2.4 g) as yellowish solid.
$^1$H- NMR (300Hz, $CDCl_3$) δ: 7.88 (s, 1H), 7.85 (s, 1H), 3.93 (s, 3H), 2, 42 (s, 3H) .

Step 3: 2-[(1-methyl)-4-pyrazolyl]-2-oxoacetaldehyde (**III-5**)

All used starting materials, reagents and preparation method were the same as those used in step 1 of Example 1, except that acetophenone was replaced with 1- [(1- methyl)- 4- pyrazolyl] ethanone. As a result, compound III- 5 was obtained as colorless oil.
$^1$H- NMR (300Hz, $CDCl_3$) δ: 9.27 (br, 1H), 8.03 (s, 2H), 3.98 (s, 3H), 3.49 (s, 3H) .

Step 4: 2,2-diethoxy-1-[(1-methyl)-4-pyrazolyl]ethanone (**IV-5**)

All used starting material, reagents and preparation method were the same as those used in step 2 of Example 1, except that 2- oxo- 2- phenylacetaldehyde **(III- 1)** was replaced with 2- [(1- methyl)- 4- pyrazolyl]- 2- oxoacetaldehyde (**III- 5**) . As a result, compound IV- 5 was obtained as colorless oil.
$^1$H- NMR (300Hz, $CDCl_3$) δ: 8.07 (s, 1H), 8.05 (s, 1H), 4.87 (s, 1H), 3.91 (s, 3H), 3.57- 3.75 (m, 4H), 1.21 (t, *J* = 6.9 Hz, 6H) .

Step 5: 3-methylthio-6-[(1-methyl)-1H-4-pyrazolyl]-1,2,4-triazine (**V-5**)

All used starting materials, reagents and preparation method were the same as those used in step 3 of Example 1, except that 2, 2- diethoxyacetophenone **(IV- 1)** was replaced with 2, 2- diethoxy- 1- [(1- methyl)- 4- pyrazolyl] ethanone (**IV- 5**) . As a result, compound V- 5 was obtained as yellowish solid.
$^1$H- NMR (300Hz, DMSO- $d_6$) δ: 8.96 (s, 1H), 8.47 (s, 1H), 8.14 (s, 1H), 3.91 (s, 3H), 2.61 (s, 3H) .

Step 6: 3-methylsulfinyl-6-[(1-methyl)-4-pyrazolyl]-1,2,4-triazine **(VI-5)**

All used starting materials, reagents and preparation method were the same as those used in step 4 of Example 1, except that 3- methylthio- 6- phenyl- 1, 2, 4- triazine (**V- 1**) was replaced with 3- methylthio- 6- [(1- methyl)- 4- pyrazolyl]- 1, 2, 4- triazine (**V- 5**) . As a result, compound VI- 5 was obtained as yellowish solid.
$^1$H- NMR (300Hz, $CDCl_3$) δ: 8.90 (s, 1H), 8.23 (s, 1H), 8.15 (s, 1H), 4.05 (s, 3H), 3.08 (s, 3H) .

Step 7: 3-hydrazino-6-[(1-methyl)-1H-4-pyrazolyl]-1,2,4-triazine (**VII-5**)

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- [(1- methyl)- 4- pyrazolyl]- 1, 2, 4- triazine **(VI- 5) .** As a result, compound VII- 5 was obtained as yellowish solid.
$^1$H- NMR (300Hz, DMSO- $d_6$) δ: 8.62 (s, 1H), 8.58 (br, 1H), 8.26 (s, 1H), 7.97 (s, 1H), 4.32 (br, 2H), 3.88 (s, 3H) .

Step 8: 6-[(1-methyl)-4-pyrazolyl]-3-(6-quinolylacetylhydrazino)-1,2,4-triazine **(VIII-5)**

All used starting materials, reagents and preparation method were the same as those used in step 6 of Example 1, except that 3- hydrazino- 6- phenyl- 1, 2, 4- triazine (**VII- 1**) was replaced with 3- hydrazino- 6- [(1- methyl)- 4- pyrazolyl]- 1, 2, 4- triazine **(VII- 5) .** As a result, compound VIII- 5 was obtained as yellowish solid.

$^1$H- NMR (300Hz, DMSO- $d_6$) δ: 10.31 (s, 1H), 9.45 (s, 1H), 8.87 (dd, *J* = 4.2, 1, 5 Hz, 1H), 8.73 (s, 1H), 8.33 (s, 2H), 8.01 (s, 1H), 7.98 (d, *J* = 8.7 Hz, 1H), 7.91 (d, *J* = 1.5 Hz, 1H), 7.75 (dd. *J* = 9.0, 2.1 Hz, 1H), 7.53 (dd, *J* = 8.4, 4.2 Hz, 1H), 3.89 (s, 3H), 3.75 (s, 2H) .

Step 9:

3-(6-quinolylmethyl)-6-[(1-methyl)-4-pyrazolyl][1,2,4]triazolo[4,3-b][1,2,4]triazine (**I-5**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- [(1- methyl)- 4- pyrazolyl]- 3- (6- quinolylacetylhydrazino)- 1, 2, 4- triazine **(VIII- 5) .** As a result, compound 1- 5 was obtained as white solid.
$^1$H- NMR (300Hz, DMSO- $d_6$) δ: 9.09 (s, 1H), 8.83 (d, *J* = 1.2 Hz, 1H), 8.62 (s, 1H), 8.33 (dd, *J* = 5.4, 1.5 Hz, 1H), 8.24 (d, *J* = 1.5 Hz, 1H), 7.98 (m, 2H), 7.81 (dd, *J* = 9.0, 2.1 Hz, 1H), 7.52 (dd, *J* = 8.4, 4.2 Hz, 1H), 4.70 (s, 2H), 3.94 (s, 3H) .

Example 6:

3-(6-quinolylmethyl)-6-[(1-ethyl)-4-pyrazolyl][1,2,4]triazolo[4,3-b][1,2,4]triazine (**I-6**)

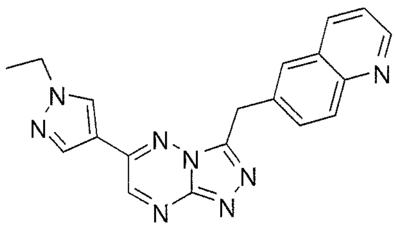

Step 1 : 1-ethyl-4-iodopyrazole

All used starting materials, reagents and preparation method were the same as those used in step 1 of Example 5, except that iodomethane was replaced with iodoethane. As a result, 1- ethyl- 4- iodopyrazole was obtained as colorless oil.
$^1$H- NMR (300Hz, $CDCl_3$) δ: 7.49 (s, 1H), 7.43 (s, 1H), 4.21 (m, *J* = 6.9 Hz, 2H), 1.49 (t, *J* = 6.9 Hz, 3H) .

Step 2: 1-[(1-ethyl)-4-pyrazolyl]ethanone

All used starting materials, reagents and preparation method were the same as those used in step 2 of Example 5, except that 1- methyl- 4- iodopyrazole was replaced with 1- ethyl- 4- iodopyrazole. As a result, 1- [(1- ethyl)- 4- pyrazolyl] ethanone was obtained as yellowish oil.
$^1$H- NMR (300Hz, $CDCl_3$) δ: 7.88 (s, 2H), 4.22 (m, *J* = 6.9 Hz, 2H), 2.41 (s, 3H), 1.52 (t, *J* = 6.9 Hz, 3H) .

Step 3: 2-[(1-ethyl)-4-pyrazolyl]-2-oxoacetaldehyde (**III-6**)

All used starting materials, reagents and preparation method were the same as those used in step 1 of Example 1, except that acetophenone was replaced with 1- [(1- ethyl)- 4- pyrazolyl] ethanone. As a result, compound III- 6 was obtained as colorless oil.

Step 4: 2,2-diethoxy-1-[(1-ethyl)-4-pyrazolyl]ethanone (**IV-6**)

All used starting materials, reagents and preparation method were the same as those used in step 2 of Example 1, except that 2- oxo- 2- phenylacetaldehyde (III- 1) was replaced with 2- [(1- ethyl)- 4- pyrazolyl]- 2- oxoacetaldehyde (**III- 6**) . As a result, compound IV- 6 was obtained as colorless oil.
$^1$H- NMR (300Hz, $CDCl_3$) δ: 8.08 (s, 1H), 8.05 (s, 1H), 4.88 (s, 1H), 4.16 (m, *J* = 6.9 Hz, 2H), 3.56- 3.77 (m, 4H), 1.49 (t, *J* = 6.9 Hz, 3H), 1.18- 1.24 (m, 6H) .

Step 5: 3-methylthio-6-[(1-ethyl)-4-pyrazolyl]-1,2,4-triazine (**V-6**)

All used starting materials, reagents and preparation method were the same as those used in step 3 of Example 1, except that 2, 2- diethoxyacetophenone **(IV- 1)** was replaced with 2, 2- diethoxy- 1- [(1- ethyl)- 4- pyrazolyl] ethanone (**IV- 6**) . As a result, compound V- 6 was obtained as yellowish solid.

$^1$H- NMR (300Hz, $CDCl_3$) δ: 8.54 (s, 1H), 8.10 (s, 1H), 8.03 (s, 1H), 4.16 (m, *J* = 6.9 Hz, 2H), 2.69 (s, 1H), 1.49 (t, *J* = 6.9 Hz, 3H) .

Step 6: 3-methylsulfinyl-6-[(1-ethyl)-4-pyrazolyl]-1,2,4-triazine **(VI-6)**

All used starting materials, reagents and preparation method were the same as those used in step 4 of Example 1, except that 3- methylthio- 6- phenyl- 1, 2, 4- triazine (**V- 1**) was replaced with 3- methylthio- 6- [(1- ethyl)- 4- pyrazolyl]- 1, 2, 4- triazine (**V- 6**) . As a result, compound VI- 6 was obtained as yellowish solid.

$^1$H- NMR (300Hz, $CDCl_3$) δ: 8.91 (s, 1H), 8.26 (s, 1H), 8.16 (s, 1H), 4.16 (m, *J* = 6.9 Hz, 2H), 3.08 (s, 3H), 1.49 (t, *J* = 6.9 Hz, 3H) .

Step 7: 3-hydrazino-6-[(1-ethyl)-4-pyrazolyl]-1,2,4-triazine **(VII-6)**

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- [(1- ethyl)- 4- pyrazolyl]- 1, 2, 4- triazine **(VI- 6) .** As a result, compound VII- 6 was obtained as yellowish solid.

$^1$H- NMR (300Hz, DMSO- $d_6$) δ: 8.64 (s, 1H), 8.58 (br, 1H), 8.33 (s, 1H), 7.99 (s, 1H), 4.34 (br, 2H), 4.16 (m, *J* = 6.9 Hz, 2H), 1.49 (t, *J* = 6.9 Hz, 3H) .

Step 8: 6-[(1-ethyl)-4-pyrazolyl]-3-(6-quinolylacetylhydrazino)-1,2,4-triazine (**VIII-6**)

All used starting materials, reagents and preparation method were the same as those used in step 6 of Example 1, except that 3- hydrazino- 6- phenyl- 1, 2, 4- triazine (VII- 1) was replaced with 3- hydrazino- 6- [(1- ethyl)- 4- pyrazolyl]- 1, 2, 4- triazine **(VII- 6) .** As a result, compound VIII- 6 was obtained as yellowish solid.

$^1$H- NMR (300Hz, DMSO- $d_6$) δ: 10.36 (s, 1H), 9.47 (s, 1H), 8.88 (d, *J* = 3.3 Hz, 1H), 8.75 (s, 1H), 8.39 (s, 1H), 8.35 (d, *J* = 9.0 Hz, 1H), 7.92- 8.04 (m, 3H), 7.77 (d, *J* = 7.2 Hz, 1H), 7.55 (m, 1H), 4.16 (m, *J* = 6.9 Hz, 2H), 3.77 (s, 2H), 1.49 (t, *J* = 6.9 Hz, 3H) .

Step 9: 3-(6-quinolylmethyl)-6-[(1-ethyl)-4-pyrazolyl][1,2,4]triazolo[4,3-b][1,2,4]triazine (**I-6**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- [(1- ethyl)- 4- pyrazolyl]- 3- (6- quinolylacetylhydrazino)- 1, 2, 4- triazine **(VIII- 6) .** As a result, compound I- 6 was obtained as white solid.

$^1$H- NMR (300Hz, DMSO- $d_6$) δ: 8.87 (dd, *J* = 1.2, 3.9 Hz, 1H), 8.70 (s, 1H), 8.03- 8.09 (m, 4H), 7.77- 7.82 (m, 2H), 7.39 (dd, *J* = 4.2, 8.1 Hz, 1H), 4.75 (s, 2H), 4.31 (m, *J* = 6.9 Hz, 2H), 1.59 (t, *J* = 6.9 Hz, 3H) .

Example 7:

3-(6-quinolylmethyl)-6-[(1-benzyl)-4-pyrazolyl][1,2,4]triazolo[4,3-b][1,2,4]triazine (**I-7**)

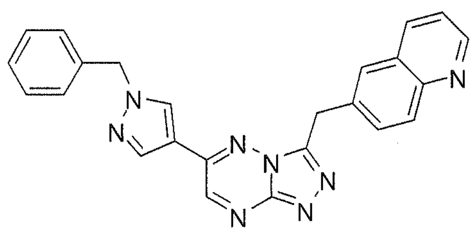

Step 1: 1-benzyl-1H-4-iodopyrazole

All used starting materials, reagents and preparation method were the same as those used in step 1 of Example 5, except that iodomethane was replaced with benzyl bromide. As a result, 1- benzyl- 4- iodopyrazole was obtained as colorless oil.

$^1$H- NMR (300Hz, $CDCl_3$) $\delta$: 7.54 (s, 1H), 7.39 (s, 1H), 7.36- 7.33 (m, 3H), 7.25- 7.23 (m, 2H), 5.30 (s, 2H)

Step 2: 1-[(1-benzyl)-4-pyrazolyl]ethanone

All used starting materials, reagents and preparation method were the same as those used in step 2 of Example 5, except that 1- methyl- 4- iodopyrazole was replaced with 1- benzyl- 4- iodopyrazole. As a result, 1- [(1- benzyl)- 4- pyrazolyl] ethanone was obtained as yellowish oil.

$^1$H- NMR (300Hz, $CDCl_3$) $\delta$: 7.93 (s, 1H), 7.84 (s, 1H), 7.38- 7.36 (m, 3H), 7.27- 7.24 (m, 2H), 5.31 (s, 2H), 2.40 (s, 3H) .

Step 3: 2-[(1-benzyl)-4-pyrazolyl]-2-oxoacetaldehyde (**III-7**)

All used starting materials, reagents and preparation method were the same as those used in step 1 of Example 1, except that acetophenone was replaced with 1- [(1- benzyl)- 1H- 4- pyrazolyl] ethanone. As a result, compound III- 7 was obtained as colorless oil.

Step 4: 2,2-diethoxy-1-[(1-benzyl)-1H-4-pyrazolyl]ethanone (**IV-7**)

All used starting materials, reagents and preparation method were the same as those used in step 2 of Example 1, except that 2- oxo- 2- phenylacetaldehyde **(III- 1)** was replaced with 2- [(1- benzyl)- 4- pyrazolyl]- 2- oxoacetaldehyde (**III- 7**) . As a result, compound IV- 7 was obtained as colorless oil.

Step 5: 3-methylthio-6-[(1-benzyl)-4-pyrazolyl]-1,2,4-triazine (**V-7**)

All used starting materials, reagents and preparation method were the same as those used in step 3 of Example 1, except that 2, 2- diethoxyacetophenone **(IV- 1)** was replaced with 2, 2- diethoxy- 1- [(1- benzyl)- 4- pyrazolyl] ethanone (**IV- 7**) . As a result, compound V- 7 was obtained as yellowish solid.

$^1$H- NMR (300Hz, $CDCl_3$) $\delta$: 8.52 (s, 1H), 8.08 (s, 1H), 8.05 (s, 1H), 7.39- 7.28 (m, 5H), 5.38 (s, 2H), 2.68 (s, 3H) .

Step 6: 3-methylsulfinyl-6-[(1-benzyl)-4-pyrazolyl]-1,2,4-triazine **(VI-7)**

All used starting materials, reagents and preparation method were the same as those used in step 4 of Example 1, except that 3- methylthio- 6- phenyl- 1, 2, 4- triazine (**V- 1**) was replaced with 3- methylthio- 6- [(1- benzyl)- 1H- 4- pyrazolyl]- 1, 2, 4- triazine (**V- 7**) . As a result, compound VI- 7 was obtained as yellowish solid.

Step 7: 3-hydrazino-6-[(1-benzyl)-4-pyrazolyl]-1,2,4-triazine **(VII-7)**

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- [(1- benzyl)- 4- pyrazolyl]- 1, 2, 4- triazine **(VI- 7) .** As a result, compound VII- 7 was obtained as yellowish solid.

$^1$H- NMR (300Hz, DMSO- $d_6$) $\delta$: 8.65 (s, 1H), 8.63 (s, 1H), 8.44 (s, 1H), 8.05 (s, 1H), 7.39- 7.28 (m, 5H), 5.39 (s, 2H), 4.40 (br, 2H) .

Step 8: 6-[(1-benzyl)-4-pyrazolyl]-3-(6-quinolylacetylhydrazino)-1,2,4-triazine **(VIII-7)**

All used starting materials, reagents and preparation method were the same as those used in step 6 of Example 1, except that 3- hydrazino- 6- phenyl- 1, 2, 4- triazine (**VII- 1**) was replaced with 3- hydrazino- 6- [(1- benzyl)- 4- pyrazolyl]- 1, 2, 4- triazine **(VII- 7) .** As a result, compound VIII- 7 was obtained as yellowish solid.

$^1$H- NMR (300Hz, $CDCl_3$) $\delta$: 8.86 (d, *J* = 3.3, 1H), 8.38 (s, 1H), 8.13- 8.07 (m, 2H), 7.97 (s, 1H), 7.94 (s, 1H), 7.78 (s, 1H), 7.70- 7.64 (m, 2H), 7.41- 7.30 (m, 5H), 5.34 (s, 2H), 5.30 (s, 1H), 3.91 (s, 2H), 3.59 (s, 1H) .

Step 9: 3-(6-quinolylmethyl)-6-[(1-benzyl)-4-pyrazolyl][1,2,4]triazolo[4,3-b][1,2,4]triazine **(1-7)**

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- [(1- benzyl)- 4- pyrazolyl]- 3- (6- quinolylacetylhydrazino)- 1, 2, 4- triazine **(VIII- 7)**. As a result, compound 1- 7 was obtained as white solid.
$^1$H- NMR (300Hz, $CDCl_3$) δ: 8.87- 8.85 (m, 1H), 8.68 (s, 1H), 8.11 (s, 1H), 8.07- 8.02 (m, 3H), 7.81 (s, 1H), 7.78- 7.74 (m, 1H), 7.40- 7.28 (m, 6H), 5.40 (s, 2H), 4.73 (s, 2H) .

Example 8: 3-(6-quinolylmethyl)-6-(3-quinolyl)[1,2,4]triazolo[4,3-b][1,2,4]triazine (**I-8**)

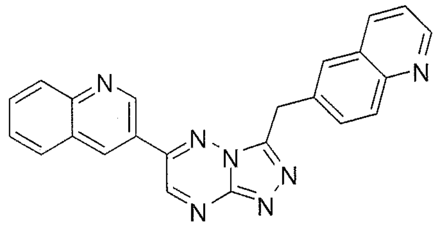

Step 1: 3-quinolylethanone

All used starting materials, reagents and preparation method were the same as those used in step 2 of Example 5, except that 1- methyl- 1H- 4- iodopyrazole was replaced with 3- bromoquinoline. As a result, 3- quinolylethanone was obtained as yellowish solid.
$^1$H- NMR (300Hz, $CDCl_3$) δ: 9.45 (d, $J$ = 2.7 Hz, 1H), 8.74 (d, $J$ = 1.8 Hz, 1H), 8.19 (d, $J$ = 8.7 Hz, 1H), 7.98 (d, $J$ = 7.8 Hz, 1H), 7.67 (m, 1H), 7.68 (m, 1H), 2.76 (s, 3H) .

Step 2: 2-oxo-2-(3-quinolyl)acetaldehyde (**III-8**)

All used starting materials, reagents and preparation method were the same as those used in step 1 of Example 1, except that acetophenone was replaced with 3-quinolylethanone. As a result, compound III-8 was obtained as yellow solid.
$^1$H- NMR (300Hz, DMSO- $d_6$) δ: 9.35 (d, $J$ = 1.8 Hz, 1H), 9.09 (d, $J$ = 1.8 Hz, 1H), 8.30 (d, $J$ = 8.4 Hz, 1H), 8.16 (d, $J$ = 8.4 Hz, 1H), 8.02 (m, 1H), 7.79 (m, 1H) .

Step 3: 2,2-diethoxy-1-(3-quinolyl)ethanone (**IV-8**)

All used starting materials, reagents and preparation method were the same as those used in step 2 of Example 1, except that 2- oxo- 2- phenylacetaldehyde (**III- 1**) was replaced with 2- oxo- 2- (3- quinolyl) acetaldehyde (**III- 8**) . As a result, compound IV- 8 was obtained.
$^1$H- NMR (300Hz, $CDCl_3$) δ: 9.57 (d, $J$ = 2.4 Hz, 1H), 9.05 (d, $J$ = 1.8 Hz, 1H), 8.17 (d, $J$ = 8.7 Hz, 1H), 7.98 (d, $J$ = 7.5 Hz, 1H), 7.87 (m, 1H), 7.63 (m, 1H), 5.24 (s, 1H), 3.65- 3.91 (m, 4H), 1.27 (t, $J$ = 6.3 Hz, 6H) .

Step 4: 3-methylthio-6-(3-quinolyl)1,2,4-triazine (**V-8**)

All used starting materials, reagents and preparation method were the same as those used in step 3 of Example 1, except that 2, 2- diethoxyacetophenone (**IV- 1**) was replaced with 2, 2- diethoxy- 1- (3- quinolyl) ethanone (**IV- 8**) . As a result, compound V- 8 was obtained as yellowish solid.
$^1$H- NMR (300Hz, $CDCl_3$) δ: 9.61 (d, $J$ = 2.1 Hz, 1H), 8.96 (s, 1H), 8.83 (d, $J$ = 2.1 Hz, 1H), 8.21 (d, $J$ = 8.4 Hz, 1H), 7.98 (d, $J$ = 8.4 Hz, 1H), 7.83 (m, 1H), 7.65 (m, 1H), 2.77 (s, 3H) .

Step 5: 3-methylsulfinyl-6-(3-quinolyl)1,2,4-triazine **(VI-8)**

All used starting materials, reagents and preparation method were the same as those used in step 4 of Example 1, except that 3- methylthio- 6- phenyl- 1, 2, 4- triazine (**V- 1**) was replaced with 3- methylthio- 6- (3- quinolyl) 1, 2, 4- triazine (**V- 8**) . As a result, compound VI- 8 was obtained as yellowish solid.

Step 6: 3-hydrazino-6-(3-quinolyl)-1,2,4-triazine(**VII-8**)

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- (3- quinolyl)- 1, 2, 4- triazine **(VI- 8) .** As a result, compound VII- 8 was obtained as yellowish solid.

$^1$H- NMR (300Hz, DMSO- $d_6$) δ: 9.58 (d, *J* = 2.4 Hz, 1H), 9.09 (s, 1H), 9.06 (br, 1H), 8.97 (d, *J* = 1.8 Hz, 1H), 8.09 (m, 2H), 7.83 (m, 1H), 7.68 (m, 1H), 4.53 (br, 2H) .

Step 7: 6-(3-quinolyl)-3-(6-quinolylacetylhydrazino)-1,2,4-triazine (**VIII-8**)

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- quinolyl)- 1, 2, 4- triazine **(VII- 8) .** As a result, compound VIII- 8 was obtained as yellowish solid.

$^1$H- NMR (300Hz, DMSO- $d_6$) δ: 10.28 (s, 1H), 9.87 (br, 1H), 9.56 (d, *J* = 2.4 Hz, 1H), 9.20 (s, 1H), 9.02 (d, *J* = 2.1 Hz, 1H), 8.89 (m, 2H), 8.35 (m, 2H), 8.03- 8.11 (m, 3H), 7.80 (m, 3H), 3.82 (s, 2H) .

Step 8: 3-(6-quinolylmethyl)-6-(3-quinolyl)[1,2,4]triazolo[4,3-b][1,2,4]triazine (**I-8**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (3- quinolyl)- 3- (6- quinolylacetylhydrazino)- 1, 2, 4- triazine **(VIII- 8) .** As a result, compound I- 8 was obtained as white solid.

$^1$H- NMR (300Hz, DMSO- $d_6$) δ: 9.59 (d, *J* = 2.4 Hz, 1H), 9.55 (s, 1H), 9.26 (d, *J* = 1.8 Hz, 1H), 8.87 (m, 1H), 8.43 (d, *J* = 2.4 Hz, 1H), 8.16 (d, *J* = 9.9 Hz, 2H), 7.98 (d, *J* = 9.9 Hz, 2H), 7.61 (m, 2H), 7.42 (m, 2H), 4.86 (s, 2H) .

Example 9:

3-(3-indolylmethyl)-6-[(1-propyl)-4-pyrazolyl][1,2,4]triazolo[4,3-b][1,2,4]triazine **(1-9)**

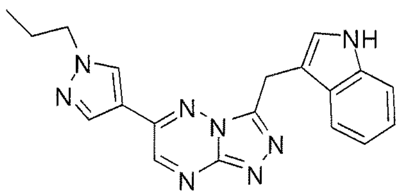

Step 1: 1-propyl-4-iodopyrazole

All used starting materials, reagents and preparation method were the same as those used in step 1 of Example 5, except that iodomethane was replaced with propyl bromine. As a result, 1- propyl- 1H- 4- iodopyrazole was obtained as colorless oil.

$^1$H- NMR (300Hz, $CDCl_3$) δ: 7.50 (s, 1H), 7.42 (s, 1H) . 4.09 (t, *J* = 6.9 Hz, 3H), 1.87 (m, 2H), 0.91 (t, *J* = 7.2 Hz, 2H) .

Step 2: 1-[(1-propyl)-4-pyrazolyl]ethanone

All used starting materials, reagents and preparation method were the same as those used in step 2 of Example 5, except that 1- methyl- 4- iodopyrazole was replaced with 1- propyl- 4- iodopyrazole. As a result, 1- [(1- propyl)- 4- pyrazolyl] ethanone was obtained as yellowish oil.

$^1$H- NMR (300Hz, $CDCl_3$) δ: 7.90 (s, 1H), 7.88 (s, 1H) . 4.10 (t, *J*= 7.5 Hz, 2H), 2.43 (s, 3H), 1.88- 1.95 (m, 2H), 0.93 (t, *J* = 7.2 Hz, 3H) .

Step 3: 2-[(1-propyl)-4-pyrazolyl]-2-oxoacetaldehyde (**III-9**)

All used starting materials, reagents and preparation method were the same as those used in step 1 of Example 1, except that acetophenone was replaced with 1- [(1- propyl)- 4- pyrazolyl] ethanone. As a result, compound III- 9 was obtained as colorless oil.

$^1H$- NMR (300Hz, $CDCl_3$) $\delta$: 9.46 (s, 1H), 8.15 (s, 1H), 8.12 (s, 1H), 4.11 (t, *J* = 6.6 Hz, 2H), 1.85- 2.02 (m, 2H), 0.93 (t, *J* = 7.2 Hz, 3H) .

Step 4: 2,2-diethoxy-1-[(1-propyl)-4-pyrazolyl]ethanone (**IV-9**)

All used starting materials, reagents and preparation method were the same as those used in step 2 of Example 1, except that 2- oxo- 2- phenylacetaldehyde (**III- 1**) was replaced with 2- [(1- propyl)- 4- pyrazolyl]- 2- oxoacetaldehyde (**III- 9**) . As a result, compound IV- 9 was obtained as colorless oil.

$^1H$- NMR (300Hz, $CDCl_3$) $\delta$: 8.10 (d, *J* = 1.2 Hz, 2H), 4.92 (s, 1H), 4.10 (t, *J* =7.2 Hz, 2H), 3.58- 3.80 (m, 4H), 1.86- 1.96 (m, 2H), 1.26 (t, *J* = 7.5 Hz, 6H), 0.94 (t, *J* = 6.9 Hz, 3H) .

Step 5: 3-methylthio-6-[(1-propyl)-4-pyrazolyl]-1,2,4-triazine (**V-9**)

All used starting materials, reagents and preparation method were the same as those used in step 3 of Example 1, except that 2, 2- diethoxyacetophenone (**IV- 1**) was replaced with 2, 2- diethoxy- 1- [(1- propyl)- 4- pyrazolyl] ethanone (**IV- 9**) . As a result, compound V- 9 was obtained as yellowish solid.

$^1H$- NMR (300Hz, $CDCl_3$) $\delta$: 8.55 (s, 1H), 8.09 (s, 1H), 8.04 (s, 1H), 4.17 (t, *J* = 4.8 Hz, 2H), 2.70 (s, 3H), 1.90- 2.00 (m, 2H), 0.97 (t, *J* = 7.5 Hz, 3H) .

Step 6: 3-methylsulfinyl-6-[(1-propyl)-4-pyrazolyl]-1,2,4-triazine **(VI-9)**

All used starting materials, reagents and preparation method were the same as those used in step 4 of Example 1, except that 3- methylthio- 6- phenyl- 1, 2, 4- triazine (**V- 1**) was replaced with 3- methylthio- 6- [(1- propyl)- 4- pyrazolyl]- 1, 2, 4- triazine (**V- 9**) . As a result, compound VI- 9 was obtained as yellowish solid.

Step 7: 3-hydrazino-6-[(1-propyl)-4-pyrazolyl]-1,2,4-triazine **(VII-9)**

All used starting materials, reagents and preparation method were the same as those used in step 5 of Example 1, except that 3- methylsulfinyl- 6- phenyl- 1, 2, 4- triazine (VI- 1) was replaced with 3- methylsulfinyl- 6- [(1- propyl)- 4- pyrazolyl]- 1, 2, 4- triazine **(VI- 9) .** As a result, compound VII- 9 was obtained as yellowish solid.

$^1H$- NMR (300Hz, DMSO- $d_6$) $\delta$: 8.65 (s, 1H), 8.60 (br s, 1H), 8.33 (s, 1H), 8.01 (s, 1H), 4.34 (br s, 2H), 4.12 (t, *J* = 7.2 Hz, 2H), 1.79- 1.86 (m, 2H), 0.86 (t, *J*= 7.2 Hz, 3H) .

Step 8:6-[(1-propyl)-4-pyrazolyl]-3-(3-indolylacetylhydrazino)-1,2,4-triazine **(VIII-9)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- [(1- propyl)- 4- pyrazolyl]- 1, 2, 4- triazine **(VII- 9),** and 6- quinoline acetic acid was replaced with 3- indole acetic acid. As a result, compound VIII- 9 was obtained as yellowish solid.

$^1H$- NMR (300Hz, DMSO- $d_6$) $\delta$: 10.88 (br s, 1H), 10.13 (s, 1H), 9.39 (br s, 1H), 8.73 (s, 1H), 8.38 (s, 1H), 8.04 (s, 1H), 7.64 (d, *J* = 7.2 Hz, 1H), 7.34 (d, *J* = 8.1 Hz, 1H), 7.26 (d, *J* = 2.1 Hz, 1H), 6.97- 7.10 (m, 2H), 4.13 (t, *J* = 6.9 Hz, 2H), 3.62 (s, 2H), 1.76- 1.89 (m, 2H), 0.86 (t, *J* = 7.5 Hz, 3H) .

Step 9: 3-(3-indolylmethyl)-6-[(1-propyl)-4-pyrazolyl][1,2,4]triazolo[4,3-b][1,2,4]triazine (**I-9**)

All used starting materials, reagents and preparation method were the same as those used in step 7 of Example 1, except that 6- phenyl- 3- (6- quinolylacetylhydrazino)- 1, 2, 4- triazine (VIII- 1) was replaced with 6- [(1- propyl)- 4- pyrazolyl]- 3- (3- indolylacetylhydrazino)- 1, 2, 4- triazine **(VIII- 9) .** As a result, compound 1- 9 was obtained as white solid.

$^1H$- NMR (300Hz, DMSO- $d_6$) $\delta$: 10.96 (br s, 1H), 9.08 (s, 1H), 8.69 (s, 1H), 8.28 (s, 1H), 7.67 (d, *J* = 7.5 Hz, 1H), 7.35 (t, *J* = 8.4, 8.1 Hz, 2H), 6.99- 7.09 (m, 2H), 4.57 (s, 2H), 4.18 (t, *J* = 6.9 Hz, 2H), 1.28- 1.89 (m, 2H), 0.87 (t, *J* = 7.2 Hz, 3H) .

Example 10: 3-(3-indolylmethyl)-6-(4-bromophenyl)[1,2,4]triazolo[4,3-b][1,2,4]triazine **(1-10)**

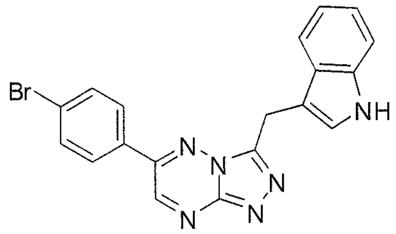

Step 1: 2-(4-bromophenyl)-2-oxoacetaldehyde (**III-10**)

All used starting materials, reagents and preparation method were the same as those used in step 1 of Example 1, except that acetophenone was replaced with 4-bromoacetophenone. As a result, compound III-10 was obtained as yellow solid.

$^1$H- NMR (300Hz, $CDCl_3$) $\delta$: 8.01 (d, *J* = 8.4 Hz, 2H), 7.68 (d, *J* = 8.4 Hz, 2H) .

Step 2: 1-(4-bromophenyl)-2,2-diethoxyethanone (**IV-10**)

All used starting materials, reagents and preparation method were the same as those used in step 2 of Example 1, except that 2- oxo- 2- phenyl acetaldehyde (**III- 1)** was replaced with 2- (4- bromophenyl)- 2- oxoacetaldehyde (**III-10**) . As a result, compound IV- 10 was obtained as yellowish oil.

$^1$H- NMR (300Hz, $CDCl_3$) $\delta$: 8.02 (d, *J* = 8.7 Hz, 2H), 7.57 (d, *J* = 8.7 Hz, 2H), 5.15 (s, 1H), 3.56- 3.80 (m, 4H), 1.22 (t, *J* = 7.4 Hz, 6H) .

Step 3: 3-methylthio-6-(4-bromophenyl)-1,2,4-triazine (**V-10**)

All used starting materials, reagents and preparation method were the same as those used in step 3 of Example 1, except that 2, 2- diethoxyacetophenone (**IV- 1**) was replaced with 1- (4- bromophenyl)- 2, 2- diethoxyethanone (**IV-10**) . As a result, compound V- 10 was obtained as yellowish solid.

$^1$H- NMR (300Hz, $CDCl_3$) $\delta$: 8.75 (s, 1H), 7.93 (d, *J*=9.0 Hz, 2H), 7.68 (d, *J*=9.0 Hz, 2H), 2.73 (s, 3H) .

Step 4: 3-methylsulfinyl-6-(4-bromophenyl)-1,2,4-triazine **(VI-10)**

All used starting materials, reagents and preparation method were the same as those used in step 4 of Example 1, except that 3- methylthio- 6- phenyl- 1, 2, 4- triazine (**V- 1**) was replaced with 3- methylthio- 6- (4- bromophenyl)- 1, 2, 4- triazine (**V- 10**) . As a result, compound VI- 10 was obtained as yellowish solid.

Step 5: 3-hydrazino-6-(4-bromophenyl)-1,2,4-triazine (**VII-10**)

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- (4- bromophenyl)- 1, 2, 4- triazine **(VI- 10) .** As a result, compound VII- 10 was obtained as yellowish solid.

$^1$H- NMR (300Hz, DMSO- $d_6$) $\delta$: 8.96 (br, 1H), 8.89 (s, 1H), 7.99 (d, *J* = 8.4 Hz, 2H), 7.71 (d, *J* = 8.4 Hz, 2H), 4.47 (s, 2H) .

Step 6: 6-(4-bromophenyl)-3-(3-indolylacetylhydrazino)-1,2,4-triazine (**VIII-10**)

All used starting materials, reagents and preparation method were the same as those used in step 6 of Example 1, except that 3- hydrazino- 6- phenyl- 1, 2, 4- triazine (**VII- 1**) was replaced with 3- hydrazino- 6- (4- bromophenyl)- 1, 2, 4- triazine (**VII- 10**), and 6- quinoline acetic acid was replaced with 3- indole acetic acid. As a result, compound VIII-10 was obtained as yellowish solid.

$^1$H- NMR (300Hz, DMSO- $d_6$) $\delta$: 10.90 (s, 1H), 10.24 (s, 1H), 9.72 (br s, 1H), 8.98 (s, 1H), 8.01 (d, *J* = 8.4 Hz, 2H), 7.73 (d, *J* = 8.4 Hz, 2H), 7.65 (d, *J* = 7.5 Hz, 1H), 7.35 (d, *J* = 8.1 Hz, 1H), 7.28 (d, *J* = 2.1 Hz, 1H), 6.98- 7.10 (m, 2H), 3.65 (s, 2H) .

Step 7: 3-(3-indolylmethyl)-6-(4-bromophenyl)[1,2,4]triazolo[4,3-b][1,2,4]triazine (**I-10**)

All used starting materials, reagents and preparation method were the same as those used in step 7 of Example

1, except that 6- phenyl- 3- (6- quinolylacetylhydrazino)- 1, 2, 4- triazine (**VIII- 1**) was replaced with 6- (4- bromophenyl)- 3- (3- indolylacetylhydrazino)- 1, 2, 4- triazine **(VIII- 10) .** As a result, compound I- 10 was obtained as white solid.

$^1H$- NMR (300Hz, DMSO- $d_6$) δ: 10.96 (s, 1H), 9.32 (s, 1H), 8.12 (d, *J* = 8.7 Hz, 2H), 7.84 (d, *J* = 8.7 Hz, 2H), 7.66 (d, *J* = 8.4 Hz, 1H), 7.34 (t, *J* = 2.1, 5.1 Hz, 2H), 6.97- 7.10 (m, 2H), 4.64 (s, 2H) .

Example 11: 3-(3-indolylmethyl)-6-(3-hydroxylphenyl)[1,2,4]triazolo[4,3-b][1,2,4]triazine **(I-11)**

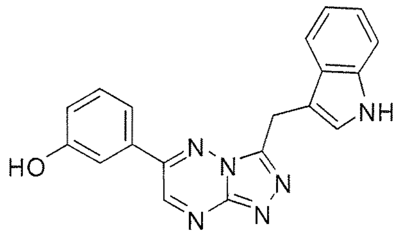

Step1: 3-(2-oxoacetyl)phenylbenzoate **(III-11)**

All used starting materials, reagents and preparation method were the same as those used in step 1 of Example 1, except that acetophenone was replaced with 3-acetylphenyl benzoate. As a result, compound III-11 was obtained.

$^1H$- NMR (300Hz, $CDCl_3$) δ: 8.22 (s, 1H), 8.19 (d, *J* = 9.6 Hz, 1H), 7.96 (dt, *J* = 1.2, 2.1, 9.6 Hz, 1H), 7.90 (t, *J* = 1.2, 2.1 Hz, 1H), 7.49- 7.68 (m, 5H) .

Step 2: 3-[2,2-diethoxy)acetyl]phenylbenzoate (**IV-11**)

All used starting materials, reagents and preparation method were the same as those used in step 2 of Example 1, except that 2- oxo- 2- phenylacetaldehyde **(III- 1)** was replaced with 3- (2- oxoacetyl) phenyl benzoate **(III- 11) .** As a result, compound IV- 11 was obtained as yellowish oil.

$^1H$- NMR (300Hz, $CDCl_3$) δ: 8.23 (s, 1H), 8.20 (d, *J* = 1.5 Hz, 1H), 8.11 (dt, *J* = 1.2, 1.5, 8.1 Hz, 1H), 8.01 (t, *J* = 1.2, 1.5 Hz, 1H), 7.46- 7.66 (m, 5H), 5.26 (s, 1H), 3.63- 3.80 (m, 4H), 1.26 (t, *J* = 7.2 Hz, 6H) .

Step 4: 3-[6-(3-methylthio)-1,2,4-triazinyl]phenylbenzoate (**V-11**)

All used starting materials, reagents and preparation method were the same as those used in step 3 of Example 1, except that 2, 2- diethoxy acetophenone (**IV- 1**) was replaced with 3- [2, 2- diethoxy) acetyl] phenylbenzoate (**IV- 11**) . As a result, compound V- 11 was obtained as yellowish solid.

$^1H$- NMR (300Hz, $CDCl_3$) δ: 8.79 (s, 1H), 8.23 (d, *J* = 8.4 Hz, 2H), 7.98 (s, 1H), 7.95 (s, 1H), 7.51- 7.67 (m, 4H), 7.41 (d, *J* = 7.4 Hz, 1H), 2.74 (s, 3H) .

Step 5: 3-[6-(3-methylsulfinyl)-1,2,4-triazinyl]phenylbenzoate(**VI-11**)

All used starting materials, reagents and preparation method were the same as those used in step 4 of Example 1, except that 3- methylthio- 6- phenyl- 1, 2, 4- triazine (**V- 1**) was replaced with 3- [6- (3- methylthio)- 1, 2, 4- triazinyl] phenyl benzoate (**V- 11**) . As a result, compound VI- 11 was obtained as yellowish solid.

Step 6: 3-hydrazino-6-(3-hydroxylphenyl)-1,2,4-triazine (**VII-11**)

All used starting materials, reagents and preparation method were the same as those used in step 5 of Example 1, except that 3- methylsulfinyl- 6- phenyl- 1, 2, 4- triazine (**VI- 1**) was replaced with 3- [6- (3- methylsulfinyl)- 1, 2, 4- triazinyl] phenyl benzoate **(VI- 11) .** As a result, compound VII- 11 was obtained as yellowish solid.

$^1H$- NMR (300Hz, DMSO- $d_6$) δ: 9.64 (br, 1H), 8.85 (br, 1H), 8.79 (s, 1H), 7.41 (d, *J* = 8.2 Hz, 1H), 7.29 (t, *J* = 7.5, 8.2 Hz, 1H), 6.84 (dd, *J* = 1.8, 7.5 Hz, 1H), 4.44 (s, 2H) .

Step 7: 6-(3-hydroxylphenyl)-3-(3-indolylacetylhydrazino)-1,2,4-triazine(**VIII-11**)

All used starting materials, reagents and preparation method were the same as those used in step 6 of Example

1, except that 3- hydrazino- 6- phenyl- 1, 2, 4- triazine **(VII- 1)** was replaced with 3- hydrazino- 6- (3- hydroxylphenyl)- 1, 2, 4- triazine (**VII- 11**), and 6- quinoline acetic acid was replaced with 3- indole acetic acid. As a result, compound VIII- 11 was obtained as yellowish solid.

$^1H$- NMR (300Hz, DMSO- $d_6$) $\delta$: 10.89 (br s, 1H), 10.20 (s, 1H), 9.67 (s, 1H), 9.61 (br s, 1H), 8.88 (s, 1H), 7.65 (d, *J* = 7.2 Hz, 1H), 7.44 (d, *J* = 9 Hz, 2H), 7.34 (t , *J* = 6.9, 6.6 Hz, 2H), 7.28 (d, *J* = 2.1 Hz, 1H), 6.98- 7.10 (m, 2H), 6.87 (d, *J* = 6.6 Hz, 1H), 3.65 (s, 2H) .

Step 8: 3-(3-indolylmethyl)-6-(3-hydroxylphenyl)[1,2,4]triazolo[4,3-b][1,2,4]triazine **(I-11)**

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (3- hydroxylphenyl)- 3- (3- indolylacetylhydrazino)- 1, 2, 4- triazine (**VIII- 11**) . As a result, compound I- 11 was obtained as white solid.

$^1H$- NMR (300Hz, DMSO- $d_6$) $\delta$: 10.96 (br s, 1H), 9.92 (s, 1H), 9.26 (s, 1H), 7.57- 7.67 (m, 3H), 7.42 (t, *J* = 7.2, 9 Hz, 1H), 7.34 (t, *J* = 3.6, 1.8 Hz, 2H), 6.96- 7.09 (m, 3H), 4.63 (s, 2H) .

Example 12:

3-(6-quinolylmethyl)-6-[(1-propyl)-4-pyrazolyl][1,2,4]triazolo[4,3-b][1,2,4]triazine (**I-12**)

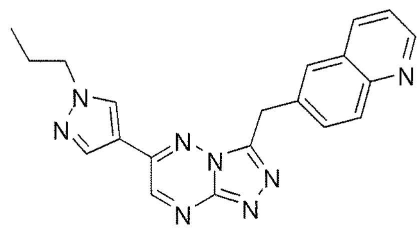

Step 1: 6-[(1-propyl)-4-pyrazolyl]-3-(6-quinolylacetylhydrazino)-1,2,4-triazine (**VIII-12**)

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- [(1- propyl)- 4- pyrazolyl]- 1, 2, 4- triazine **(VII- 9) .** As a result, compound VIII- 12 was obtained as yellowish solid.

$^1H$- NMR (300Hz, DMSO- $d_6$) $\delta$: 10.34 (s, 1H), 9.48 (s, 1H), 8.88 (dd, *J* = 1.8, 4.5 Hz, 1H), 8.76 (s , 1H), 8.39 (s, 1H), 8.35 (d, *J* = 8.7 Hz, 1H), 8.05 (s, 1H), 7.99 (d, *J* = 8.4 Hz, 1H), 7.92 (s, 1H), 7.76 (dd, *J* = 1.8, 9 Hz, 1H), 7.53 (q, *J* = 4.2, 8.4 Hz, 1H), 4.12 (t, *J* = 7.2 Hz, 2H), 3.77 (s, 2H), 1.77- 1.89 (m, 2H), 0.85 (t, *J* = 7.2 Hz, 3H) .

Step 2: 3-(6-quinolylmethyl)-6-[(1-propyl)-4-pyrazolyl][1,2,4]triazolo[4,3-b][1,2,4]triazine **(I-12)**

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- [(1- propyl)- 4- pyrazolyl]- 3- (6- quinolylacetylhydrazino)- 1, 2, 4- triazine (**VIII- 12**) . As a result, compound I- 12 was obtained as white solid.

$^1H$- NMR (300Hz, DMSO- $d_6$) $\delta$: 8.88 (d, *J* = 4.5 Hz, 1H), 8.71 (s, 1H), 8.08 (t , *J* = 9.0, 8.7 Hz, 3H), 8.01 (s, 1H), 7.82 (t, *J* = 4.5, 8.7 Hz, 2H), 7.39 (q, *J* = 4.2, 8.4 Hz, 1H), 4.77 (s, 2H), 4.19 (t, *J* = 7.2 Hz, 2H), 1.91- 2.05 (m, 2H), 0.97 (t, *J* = 7.2 Hz, 3H) .

Examples 13 and 14:

3-(6-quinolylmethyl)-6-[1-(1-tert-butoxycarbonyl-4-piperidinyl)-4-pyrazolyl][1,2,4]triazolo[4,3 -b][1,2,4]triazine **(1-13)** and

3-(6-quinolylmethyl)-6-[1-(4-piperidinyl)-4-pyrazolyl][1,2,4]triazolo[4,3-b][1,2,4]triazine **(1-14)**

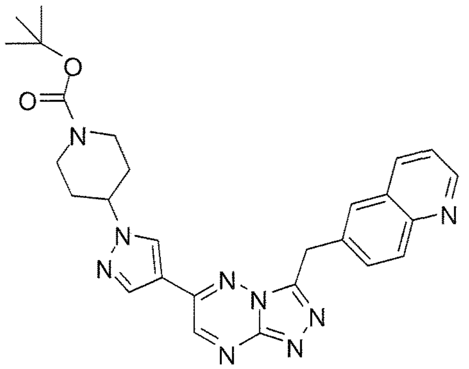

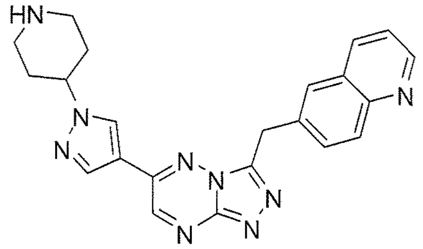

Step 1: 1-tert-butoxycarbonyl-4-methyl sulfonyloxy piperidine

1- tert- butoxycarbonyl- 4- hydroxyl piperidine (7.94 g) was dissolved in 100 mL of dichloromethane, then 48 mg of dimethylamino pyridine was added therein. The mixture was agitated in an ice- water bath, while 5.54 mL of triethylamine and 3.06 mL of methane sulfonyl chloride were added in dropwise. After that, the mixture was warmed to room temperature and agitated over night. 50 mL of water was further added therein, then the resulted reaction solution was separated and extracted by dichloromethane (3x300 mL) . The parts of dichloromethane were combined, washed by saturated saline solution, dried over anhydrous sodium sulfate, filtrated and ovendried to produce 1- tert- butoxycarbonyl- 4- methyl sulfonyloxy piperidine as white solid (10.649g, yield: 96.6%) .

$^1$H- NMR (300Hz, $CDCl_3$) δ: 4.88 (m, 1H), 3.70 (m, 2H), 3.34 (m, 2H), 3.04 (s, 3H), 1.97 (m, 2H), 1.84 (m, 2H), 1.46 (s, 9H) .

Step 2: 1-(1-tert-butoxycarbonyl-4-piperidinyl)-4-iodopyrazole

4- iodopyrazole (5.54 g) and anhydrous N, N- dimethylformamide (90 mL) were added in a round bottomed flask (250 mL) and 1.371 g of sodium hydride (60%) was further added with agitating in an ice- water bath. Then, 8.776 g of 1- tert- butoxycarbonyl- 4- methylsulfonyloxy piperidine was added in the mixture when it was agitated in an ice-water bath for another 2h, and it was warmed to 100°C, agitated for 12h and cooled in an ice- water bath. The resulted mixture was quenched by adding 300 mL of water. The reaction solution was extracted by ethyl acetate (3x100 mL), the parts of ethyl acetate were combined, washed by saturated saline solution, dried over anhydrous sodium sulfate, and purified by column chromatography (ethyl acetate: petroleum ether=1: 5) to produce 1- (1- tert- butoxycarbonyl- 4-piperidinyl)- 4- iodopyrazole as white solid (7.43 g, yield: 68.7%) .

$^1$H- NMR (300Hz, $CDCl_3$) δ: 7.51 (s, 1H), 7.46 (s, 1H), 4.30 (m, 3H), 2.87 (m, 2H), 2.12 (m, 2H), 1.89 (m, 2H), 1.47 (s, 9H) .

Step 3: 1-(1-tert-butoxycarbonyl-4-piperidinyl)-4-acetylpyrazole

All used starting materials, reagents and preparation method were the same as those used in step 2 of Example 5, except that 1- methyl- 4- iodopyrazole was replaced with 1- (1- tert- butoxycarbonyl- 4- piperidinyl)- 4- iodopyrazole. As a result, 1- (1- tert- butoxycarbonyl- 4- piperidinyl)- 4- acetyl pyrazole was obtained as yellowish oil.

$^1$H- NMR (300Hz, $CDCl_3$) δ: 7.93 (s, 1H), 7.91 (s, 1H), 4.31 (m, 3H), 2.89 (m, 2H), 2.43 (s, 3H), 2.17 (m, 2H), 1.91 (m, 2H), 1.47 (s, 9H) .

Step 4: 2-[(1-tert-butoxycarbonyl-4-piperidinyl)-4-pyrazolyl]-2-oxoacetaldehyde (**III-13**)

All used starting materials, reagents and preparation method were the same as those used in step 1 of Example 1, except that acetophenone was replaced with 1- (1- tert- butoxycarbonyl- 4- piperidinyl)- 4- acetylpyrazole. As a result, compound III- 13 was obtained as colorless oil.

Step 5: 2,2-diethoxy-1-[1-(1-tert-butoxycarbonyl-4-piperidinyl)-4-pyrazolyl] ethanone (**IV-13**)

All used starting materials, reagents and preparation method were the same as those used in step 2 of Example 1, except that 2- oxo- 2- phenylacetaldehyde (**III- 1)** was replaced with 2- [1- (1- tert- butoxycarbonyl- 4- piperidinyl)- 4-pyrazolyl]- 2- oxoacetaldehyde (**III- 13**) . As a result, compound IV- 13 was obtained as colorless oil.

$^1$H- NMR (300Hz, $CDCl_3$) δ: 8.15 (s, 1H), 8.11 (s, 1H), 4.91 (s, 1H), 4.28 (m, 3H), 3.78- 3.61 (m, 4H), 2.89 (m,

2H), 2.17 (m, 2H), 1.96 (m, 2H), 1.47 (s, 9H), 1.26 (t, J= 6.9 Hz, 6H) .

Step 6: 3-methylthio-6-[1-(1-tert-butoxycarbonyl-4-piperidinyl)-4-pyrazolyl]-1,2,4-triazine (**V-13**)

All used starting materials, reagents and preparation method were the same as those used in step 3 of Example 1, except that 2, 2- diethoxyacetophenone (**IV- 1**) was replaced with 2, 2- diethoxy- 1- [1- (1- tert- butoxycarbonyl- 4- piperidinyl)- 4- pyrazolyl] ethanone (**IV- 13**) . As a result, compound V- 13 was obtained as yellowish solid.
$^1$H- NMR (300Hz, $CDCl_3$) δ: 8.54 (s, 1H), 8.14 (s, 1H), 8.05 (s, 1H), 4.35 (m, 3H), 2.92 (m, 2H), 2.70 (s, 3H), 2.17 (m, 2H), 1.99 (m, 2H), 1.48 (s, 9H) .

Step 7:

3-methylsulfinyl-6-[1-(1-tert-butoxycarbonyl-4-piperidinyl)-4-pyrazolyl]-1,2,4-triazine **(VI-13)**

All used starting materials, reagents and preparation method were the same as those used in step 4 of Example 1, except that 3- methylthio- 6- phenyl- 1, 2, 4- triazine (**V- 1**) was replaced with 3- methylthio- 6- [1- (1- tert- butoxycarbonyl- 4- piperidinyl)- 4- pyrazolyl]- 1, 2, 4- triazine **(V- 13) .** As a result, compound VI- 13 was obtained as yellowish solid.

Step 8: 3-hydrazino-6-[1-(1-tert-butoxycarbonyl-4-piperidinyl)-4-pyrazolyl]-1,2,4-triazine (**VII-13**)

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- [2- (1- tert-butoxycarbonyl- 4- piperidinyl)- 4- pyrazolyl]- 1, 2, 4- triazine **(VI- 13) .** As a result, compound VII- 13 was obtained as yellowish solid.
$^1$H- NMR (300Hz, DMSO- $d_6$) δ: 8.65 (s, 1H), 8.42 (s, 1H), 8.02 (s, 1H), 4.38 (m, 3H), 2.93 (m, 2H), 2.06 (m, 2H), 1.83 (m, 2H), 1.42 (s, 9H) .

Step 9:

6-[1-(1-tert-butoxycarbonyl-4-piperidinyl)-4-pyrazolyl]-3-(6-quinolylacetylhydrazino)-1,2,4-tri azine **(VIII-13)**

All used starting materials, reagents and preparation method were the same as those used in step 6 of Example 1, except that 3- hydrazino- 6- phenyl- 1, 2, 4- triazine (**VII- 1**) was replaced with 3- hydrazino- 6- [1- (1- tert- butoxycarbonyl- 4- piperidinyl)- 4- pyrazolyl]- 1, 2, 4- triazine (**VII- 13**) . As a result, compound VIII- 13 was obtained as yellowish solid.
$^1$H- NMR (300Hz, $CDCl_3$) δ: 8.90- 8.89 (m, 1H), 8.43 (s, 1H), 8.18 (s, 1H), 8.15 (m, 1H), 8.10 (s, 1H), 8.03 (s, 1H), 7.96 (s, 1H), 7.82 (s, 1H), 7.74- 7.68 (m, 2H), 7.45- 7.40 (m, 1H), 4.36- 4.24 (m, 3H), 3.90 (s, 2H), 2.98- 2.86 (m, 2H), 2.18- 2.15 (m, 2H), 2.01- 1.93 (m, 2H), 1.48 (s, 9H) .

Step 10:

3-(6-quinolylmethyl)-6-[1-(1-tert-butoxycarbonyl-4-piperidinyl)-4-pyrazolyl][1,2,4]triazolo[4,3 -b][1,2,4]triazine **(I-13)** and

3-(6-quinolylmethyl)-6-[1-(4-piperidinyl)-4-pyrazolyl][1,2,4]triazolo[4,3-b][1,2,4]triazine (**I-14**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- [1- (1- tert-butoxycarbonyl- 4- piperidinyl)- 4- pyrazolyl]- 3- (6- quinolylacetylhydrazino)- 1, 2, 4- tri azine **(VIII- 13) .** As a result, compound I- 13 and I- 14 were obtained as white solid. Compound 1- 13: $^1$H- NMR (300Hz, $CDCl_3$) δ: 8.88 (d, *J* = 4.2 Hz, 1H), 8.71 (s, 1H), 8.11- 8.03 (m, 4H), 7.82- 7.77 (m, 2H), 7.41- 7.36 (m, 1H), 4.76 (s, 2H), 4.36- 4.31 (m, 3H), 2.91 (m, 2H), 2.19- 2.10 (m, 2H), 2.03- 1.92 (m, 2H), 1.48 (s, 9H) .
Compound **I- 14**: $^1$H- NMR (300Hz, $CDCl_3$) δ: 8.89- 8.87 (m, 1H), 8.71 (s, 1H), 8.12- 8.07 (m, 3H), 8.03 (s, 1H), 7.83- 7.78 (m, 2H), 7.42- 7.38 (m, 1H), 4.77 (s, 2H), 4.45 (m, 1H), 4.03 (m, 1H), 3.32 (m, 1H), 2.79 (m, 1H), 2.36- 2.20 (m, 2H), 2.10- 1.92 (m, 3H) .

Example 15:

3-(6-quinolylmethyl)-6-(3-nitrophenyl)[1,2,4]triazolo[4,3-b][1,2,4]triazine(**I-15**)

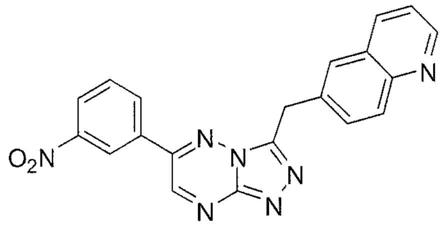

Step 1: 2-oxo-2-(3-nitrophenyl)acetaldehyde (**III-15**)

All used starting materials, reagents and preparation method were the same as those used in step 1 of Example 1, except that acetophenone was replaced with 3-nitroacetophenone. As a result, compound III-15 was obtained as colorless oil.

Step 2: 2,2-diethoxy-1-(3-nitrophenyl)ethanone (**IV-15**)

All used starting materials, reagents and preparation method were the same as those used in step 2 of Example 1, except that 2- oxo- 2- phenylacetaldehyde (**III- 1**) was replaced with 2- oxo- 2- (3- nitrophenyl) acetaldehyde (**III- 15**) . As a result, compound IV- 15 was obtained as yellowish oil.

$^1$H- NMR (300Hz, $CDCl_3$) δ: 9.03 (s, 1H), 8.52 (d, *J* = 7.8 Hz, 1H), 8.43 (dd, *J* = 2.4, 7.8 Hz, 1H), 7.69 (t, *J* = 7.8 Hz, 1H), 5.17 (s, 1H), 3.87- 3.64 (m, 4H), 1.26 (t, *J* = 6.6 Hz, 6H)

Step 3: 3-methylthio-6-(3-nitrophenyl)-1,2,4-triazine (**V-15**)

All used starting materials, reagents and preparation method were the same as those used in step 3 of Example 1, except that 2, 2- diethoxyacetophenone (**IV- 1**) was replaced with 2, 2- diethoxy- 1- (3- nitrophenyl) ethanone (**IV- 15**) . As a result, compound V- 15 was obtained as yellowish solid.

$^1$H- NMR (300Hz, $CDCl_3$) δ: 8.92 (s, 1H), 8.46 (d, *J* = 8.7 Hz, 1H), 8.41 (dd, *J* =. 4, 8.7 Hz, 1H), 7.79 (t, *J* = 8.7 Hz, 1H), 2.75 (s, 3H) .

Step 4: 3-methylsulfinyl-6-(3-nitrophenyl)-1,2,4-triazine **(VI-15)**

All used starting materials, reagents and preparation method were the same as those used in step 4 of Example 1, except that 3- methylthio- 6- phenyl- 1, 2, 4- triazine (**V- 1**) was replaced with 3- methylthio- 6- (3- nitrophenyl)- 1, 2, 4- triazine (**V- 15**) . As a result, compound VI- 15 was obtained as yellowish solid.

Step 5: 3-hydrazino-6-(3-nitrophenyl)-1,2,4-triazine(**VII-15**)

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- (3- nitrophenyl)- 1, 2, 4- triazine **(VI- 15) .** As a result, compound VII- 15 was obtained as yellowish solid.

$^1$H- NMR (300Hz, DMSO- $d_6$) δ: 9.12 (br, 1H), 9.02 (s, 1H), 8.84 (s, 1H), 8.49 (d, *J* = 8.4 Hz, 1H), 8.30 (dd, *J* = 1.5, 8.4 Hz, 1H), 7.83 (t, *J* = 8.4 Hz, 1H), 4.52 (br, 2H) .

Step 6: 6-(3-nitrophenyl)-3-(6-quinolylacetylhydrazino)-1,2,4-triazine **(VIII-15)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- nitrophenyl)- 1, 2, 4- triazine (**VII- 15**) . As a result, compound VIII- 15 was obtained as yellow solid.

Step 7: 3-(6-quinolylmethyl)-6-(3-nitrophenyl)[1,2,4]triazolo[4,3-b][1,2,4]triazine (**I-15**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (3- nitrophenyl)- 3- (6- quinolylacetylhydrazino)- 1, 2, 4- triazine **(VIII- 15) .** As a result, compound I- 15 was obtained as white solid.
$^1$H- NMR (300Hz, DMSO- $d_6$) δ: 9.46 (s, 1H), 8.47- 8.91 (m, 2H), 8.61 (d, *J* = 8.1 Hz, 1H), 8.48 (dd, *J* = 1.5, 4.8 Hz, 1H), 8.34 (d, *J* = 8.1 Hz, 1H), 7.81- 8.01 (m, 4H), 7.53 (dd, *J* = 3.9, 8.4 Hz, 1H), 4.83 (s, 2H) .

Example 16: 3-(6-quinolylmethyl)-6-(4-chlorophenyl)[1,2,4]triazolo[4,3-b][1,2,4]triazine **(1-16)**

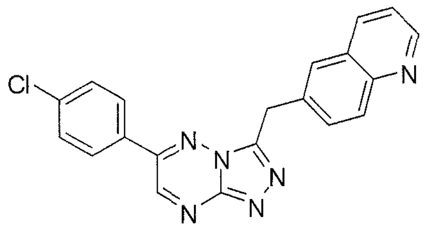

Step 1: 2-oxo-2-(4-chlorophenyl)acetaldehyde (**III-16**)

All used starting materials, reagents and preparation method were the same as those used in step 1 of Example 1, except that acetophenone was replaced with 4-chloroacetophenone. As a result, compound III-16 was obtained as colorless oil.
$^1$H- NMR (300Hz, $CDCl_3$) δ: 8.06 (d, *J* = 9.0 Hz, 2H), 7.46 (d, *J* = 9.0 Hz, 2H), 6.29 (s, 1H) .

Step 2: 2,2-diethoxy-1-(4-chlorophenyl)ethanone (**IV-16**)

All used starting materials, reagents and preparation method were the same as those used in step 2 of Example 1, except that 2- oxo- 2- phenylacetaldehyde (**III- 1**) was replaced with 2- oxo- 2- (4- chlorophenyl) acetaldehyde (**III-16**) . As a result, compound IV- 16 was obtained as yellowish oil.
$^1$H- NMR (300Hz, $CDCl_3$) δ: 8.12 (d, *J* = 8.4 Hz, 2H), 7.42 (d, *J* = 8.4 Hz, 2H), 5.17 (s, 1H), 3.79- 3.60 (m, 4H), 1.24 (t, *J* = 6.9 Hz, 6H) .

Step 3: 3-methylthio-6-(4-chlorophenyl)-1,2,4-triazine (**V-16**)

All used starting materials, reagents and preparation method were the same as those used in step 3 of Example 1, except that 2, 2- diethoxyacetophenone (**IV- 1**) was replaced with 2, 2- diethoxy- 1- (4- chlorophenyl) ethanone (**IV-16**) . As a result, compound V- 16 was obtained as yellowish solid.

Step 4: 3-methylsulfinyl-6-(4-chlorophenyl)-1,2,4-triazine **(VI-16)**

All used starting materials, reagents and preparation method were the same as those used in step 4 of Example 1, except that 3- methylthio- 6- phenyl- 1, 2, 4- triazine (**V- 1**) was replaced with 3- methylthio- 6- (4- chlorophenyl)- 1, 2, 4- triazine (**V- 16**) . As a result, compound VI- 16 was obtained as yellowish solid.

Step 5: 3-hydrazino-6-(4-chlorophenyl)-1,2,4-triazine **(VII-16)**

All used starting materials, reagents and preparation method were the same as those used in step 5 of Example 1, except that 3- methylsulfinyl- 6- phenyl- 1, 2, 4- triazine (**VI- 1**) was replaced with 3- methylsulfinyl- 6- (4- chlorophenyl)- 1, 2, 4- triazine **(VI- 16) .** As a result, compound VII- 16 was obtained as yellowish solid.
$^1$H- NMR (300Hz, DMSO- $d_6$) δ: 8.92 (s, 1H), 8.86 (s, 1H), 8.03 (d, *J* = 8.7 Hz, 2H), 7.55 (d, *J* = 8.7 Hz, 2H), 4.44 (s, 2H) .

Step 6: 6-(4-chlorophenyl)-3-(6-quinolylacetylhydrazino)-1,2,4-triazine (**VIII-16**)

All used starting materials, reagents and preparation method were the same as those used in step 6 of Example 1, except that 3- hydrazino- 6- phenyl- 1, 2, 4- triazine (**VII- 1**) was replaced with 3- hydrazino- 6- (4- chlorophenyl)- 1, 2, 4- triazine **(VII- 16) .** As a result, compound VIII- 16 was obtained as yellow solid.

Step 7: 3-(6-quinolylmethyl)-6-(4-chlorophenyl)[1,2,4]triazolo[4,3-b][1,2,4] triazine (**I-16**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (4- chlorophenyl)- 3- (6- quinolylacetylhydrazino)- 1, 2, 4- triazine **(VIII- 16) .** As a result, compound I- 16 was obtained as white solid.

$^1$H- NMR (300Hz, $CDCl_3$) δ: 8.94 (s, 1H), 8.88 (dd, *J* = 1.2, 3.9 Hz, 1H), 8.11 (m, 2H), 7.79- 7.90 (m, 4H), 7.56 (d, *J* = 6.9 Hz, 2H), 7.41 (dd, *J* = 4.2, 8.4 Hz, 1H), 4.81 (s, 2H) .

Example 17: 3-(6-quinolylmethyl)-6-(4-bromophenyl)[1,2,4]triazolo[4,3-b][1,2,4]triazine (1-17)

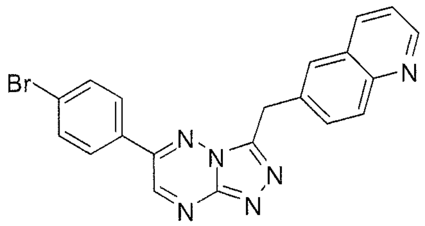

Step 1: 6-(4-bromophenyl)-3-(6-quinolylacetylhydrazino)-1,2,4-triazine(**VIII-17**)

All used starting materials, reagents and preparation method were the same as those used in step 6 of Example 1, except that 3- hydrazino- 6- phenyl- 1, 2, 4- triazine (**VII- 1**) was replaced with 3- hydrazino- 6- (4- bromophenyl)- 1, 2, 4- triazine **(VII- 10) .** As a result, compound VIII- 17 was obtained as yellow solid.

$^1$H- NMR (300Hz, DMSO- $d_6$) δ: 10.44 (s, 1H), 9.81 (br, 1H), 9.00 (s, 1H), 8.88 (d, *J* = 2.1 Hz, 1H), 8.35 (d, *J* = 7.2 Hz, 1H), 7.99- 8.03 (m, 3H), 7.93 (s, 1H), 7.75 (t, *J* = 8.7, 9.6 Hz, 3H), 7.54 (q, *J* = 2.1, 8.4 Hz, 1H), 3.79 (s, 2H) .

Step 2: 3-(6-quinolylmethyl)-6-(4-bromophenyl)[1,2,4]triazolo[4,3-b][1,2,4]triazine (**I-17**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (4- bromophenyl)- 3- (6- quinolylacetylhydrazino)- 1, 2, 4- triazine (**VIII- 17**) . As a result, compound I- 17 was obtained as white solid.

$^1$H- NMR (300Hz, DMSO- $d_6$) δ: 9.36 (s, 1H), 8.86 (d, *J* = 3.9 Hz, 1H), 8.32 (d, *J* = 8.1 Hz, 1H), 8.11 (d, *J* = 7.5 Hz, 2H), 7.99 (d, *J* = 8.7 Hz, 2H), 7.83 (t, *J* = 8.1, 9.9 Hz, 3H), 7.51 (q, *J* = 3.9, 8.4 Hz, 1H), 4.79 (s, 2H) .

Example 18:

3-(3-indolylmethyl)-6-(4-benzyloxyphenyl)[1,2,4]triazolo[4,3-b][1,2,4]triazine (**I-18**)

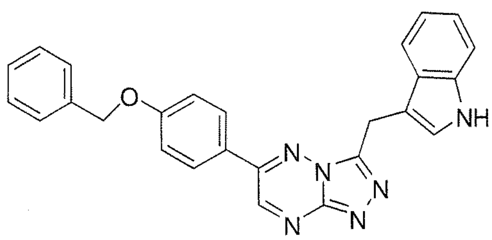

Step 1: 6-(4-benzyloxyphenyl)-3-(3-indolylacetylhydrazino)-1,2,4-triazine (**VIII-18**)

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- benzyloxyphenyl)- 1, 2, 4- triazine (**VII- 2**), and 6- quinoline acetic acid was replaced with 3- indoleacetic acid. As a result, compound VIII-18 was obtained as yellow solid.

$^{1}$H- NMR (300Hz, DMSO- $d_6$) δ: 10.90 (br s, 1H), 10.19 (s, 1H), 9.53 (br s, 1H), 8.92 (s, 1H), 7.99 (d, *J* = 9.0 Hz, 2H), 7.65 (d, *J* = 7.2 Hz, 1H), 7.33- 7.49 (m, 6H), 7.28 (d, *J* = 2.1 Hz, 1H) 7.17 (d, *J* = 9.0 Hz, 2H), 7.00- 7.10 (m, 2H), 5.19 (s, 2H), 3.64 (s, 2H) .

Step 2: 3-(3-indolylmethyl)-6-(4-benzylphenyl)[1,2,4]triazolo[4,3-b][1,2,4]triazine **(1-18)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- benzyloxyphenyl)- 3- (3- indolylacetylhydrazino)- 1, 2, 4- triazine **(VIII- 18) .** As a result, compound I- 18 was obtained as white solid.

$^{1}$H- NMR (300Hz, DMSO- $d_6$) δ: 10.95 (br s, 1H), 9.29 (s, 1H), 8.15 (d, *J* = 9.0 Hz, 2H), 7.66 (d, *J* = 8.1 Hz, 1H), 7.32- 7.50 (m, 7H), 7.24 (d, *J* = 9.0 Hz, 2H), 6.96- 7.09 (m, 2H), 5.25 (s, 2H), 4.62 (s, 2H) .

Example 19:

3-[(6-quinolyl)difluoromethyl]-6-[(1-methyl)-4-pyrazolyl][1,2,4]triazolo[4,3-b][1,2,4]triazine(**I -19)**

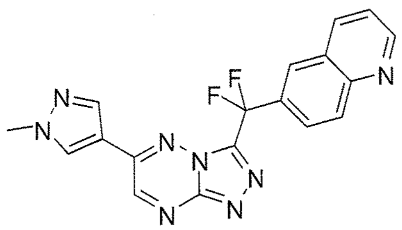

Step 1:

6-[(1-methyl)-4-pyrazolyl]-3-[2,2-difluoro-2-(6-quinolyl)acetylhydrazino]-1,2,4-triazine (**VIII-19**)

All used starting materials, reagents and preparation method were the same as those used in step 7 of Example 1, except that 3- hydrazino- 6- phenyl- 1, 2, 4- triazine (**VII- 1**) was replaced with 3- hydrazino- 6- [(1- methyl)- 4- pyrazolyl]- 1, 2, 4- triazine **(VII- 5),** and 6- quinolineacetic acid was replaced with 2, 2- difluoro- 2- (6- quinolyl) acetic acid. As a result, compound VIII- 19 was obtained as white solid.

$^{1}$H- NMR (300Hz, DMSO- $d_6$) δ: 9.73 (s, 1H), 9.04 (d, *J* = 2.1 Hz, 1H), 8.78 (s, 1H), 8.59 (dd, *J* = 9.6, 2.1 Hz, 1H), 8.46 (d, *J* = 3.0 Hz, 1H), 8.34 (s, 1H), 8.22 (dd, *J* = 5.7, 1.2 Hz, 1H), 8.03 (s, 1H), 7.83 (d, *J* = 8.1 Hz, 1H), 7.69 (dd, *J* = 8.1, 4.2 Hz, 1H), 7.55 (d, *J* = 7.8 Hz, 1H), 3.90 (s, 3H) .

Step 2:

3-[(6-quinolyl)difluoromethyl]-6-[(1-methyl)-4-pyrazolyl][1,2,4]triazolo[4,3-b][1,2,4]triazine (**I-19**)

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- [(1- methyl)- 4- pyrazolyl]- 3- [2, 2- difluoro- 2- (6quinolyl) acetylhydrazino]- 1, 2, 4- triazine **(VIII- 19) .** As a result, compound I- 19 was obtained as white solid.

$^{1}$H- NMR (300Hz, $CDCl_3$) δ: 9.02 (s, 1H), 8.84 (s, 1H), 8.27 (m, 3H), 8.03- 8.11 (m, 3H), 7.53 (dd, *J* = 8.4 Hz, 1H), 4.05 (s, 3H) .

Examples 20 and 21:

3-[(6-quinolyl)difluoromethyl]-6-[1-(1-tert-butoxycarbonyl-4-piperidinyl)-4-pyrazolyl][1,2,4]tri azolo[4,3-b][1,2,4]triazine (**I-20**) and

3-[(6-quinolyl)difluoromethyl]-6-[1-(4-piperidinyl)-4-pyrazolyl][1,2,4]triazolo[4,3-b][1,2,4]tria zine (**I-21**)

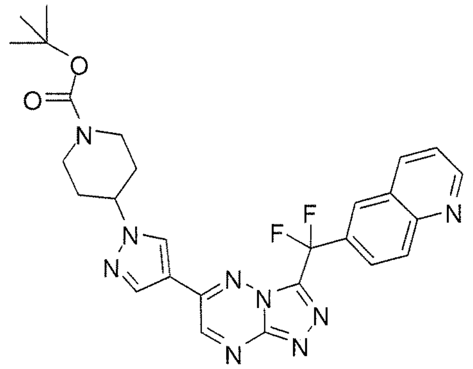

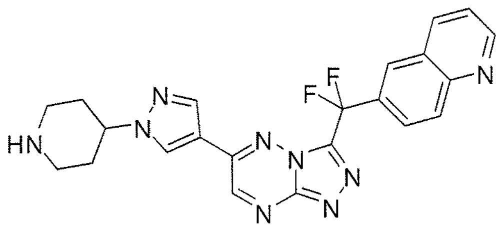

Step 1:

6-[1-(1-tert-butoxycarbonyl-4-piperidinyl)-4-pyrazolyl]-3-[2,2-difluoro-2-(6-quinolyl)acetylhyd razino]-1,2,4-triazine(**VIII-20**)

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- [1- (1- tert- butoxycarbonyl- 4- piperidinyl)- 4- pyrazolyl]- 1, 2, 4- triazine (**VII- 13**), and 6- quinolineacetic acid was replaced with 2, 2- difluoro- 2- (6- quinolyl) acetic acid. As a result, compound VIII- 20 was obtained as yellowish solid.
$^1$H- NMR (300Hz, DMSO- $d_6$) δ: 11.31 (s, 1H), 9.74 (s, 1H), 9.04 (d, *J* = 3.0 Hz, 1H), 8.78 (s, 1H), 8.58 (d, *J* = 9.0 Hz, 1H), 8.46 (s, 2H), 8.22 (d, *J* = 9.0 Hz, 1H), 8.07 (s, 1H), 8.02 (m, 1H), 7.68 (m, 1H), 4.46 (m, 1H), 3.97- 4.04 (m, 4H), 2.48 (m, 2H), 1.81 (m, 2H), 1.42 (s, 9H) .

Step 2:

3-[(6-quinolyl)difluoromethyl]-6-[1-(1-tert-butoxycarbonyl-4-piperidinyl)-4-pyrazolyl][1,2,4]tri azolo[4,3-b][1,2,4]triazine **(1-20)** and

3-[(6-quinolyl)difluoromethyl]-6-[1-(4-piperidinyl)-4-pyrazolyl][1,2,4]triazolo[4,3-b][1,2,4]tria zine (**I-21**)

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- [1- (1- tert- butoxycarbonyl- 4- piperidinyl)- 4- pyrazolyl]- 3- [2, 2- difluoro- 2- (6- quinolyl) acetylhyd razino]- 1, 2, 4- triazine **(VIII- 20) .** As a result, compound 1- 20 and 1- 21 were obtained as white solids.
Compound **1- 20:** $^1$H- NMR (300Hz, $CDCl_3$) δ: 9.03 (d, *J* = 4.2 Hz, 1H), 8.85 (s, 1H), 8.22- 8.26 (m, 3H), 8.13 (s, 2H), 8.04 (d, *J* = 9.0 Hz, 1H), 7.52 (m, 1H), 4.29- 4.37 (m, 3H), 2.92 (m, 2H), 2.20 (m, 2H), 1.94- 2.04 (m, 2H), 1.44 (s, 9H) .
Compound **I- 21**: $^1$H- NMR (300Hz, DMSO- $d_6$) δ: 9.22 (s, 1H), 9.00 (d, *J* = 1.8 Hz, 1H), 8.77 (s, 1H), 8.52 (s, 1H), 8.15 (s, 1H), 8.04 (m, 1H), 7.45- 7.65 (m, 2H), 7.07 (m, 1H), 4.50 (m, 1H), 4.14 (m, 2H), 3.15 (m, 2H), 2.48 (m, 2H), 1.94- 2.04 (m, 2H) .

Example 22: 3-[(6-quinolyl)difluoromethyl]-6-phenyl[1,2,4]triazolo[4,3-b][1,2,4]triazine (**I-22**)

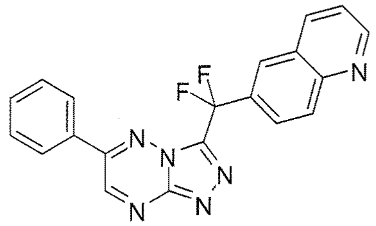

Step 1: 6-phenyl-3-[2,2-difluoro-2-(6-quinolyl)acetylhydrazino]-1,2,4-triazine (**VIII-22**)

All used starting materials, reagents and preparation method were the same as those used in step 6 of Example 1. except that 6- quinolineacetic acid was replaced with 2, 2- difluoro- 2- (6- quinolyl) acetic acid. As a result, compound VIII- 22 was obtained as yellowish solid.

$^1$H- NMR (300Hz, DMSO- $d_6$) δ: 11.34 (s, 1H), 9.95 (br, 1H), 8.97 (s, 2H), 8.39- 8.52 (m, 2H), 8.15 (m, 1H), 7.89- 7.99 (m, 3H), 7.50- 7.63 (m, 1H), 7.38- 7.47 (m, 3H) .

Step 2: 3-[(6-quinolyl)difluoromethyl]-6-phenyl[1,2,4]triazolo[4,3-b][1,2,4]triazine (1-22)

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- phenyl- 3- [2, 2- difluoro- 2- (6quinolyl) acetylhydrazino]- 1, 2, 4- triazine **(VIII- 22)** **.** As a result, compound 1- 22 was obtained as white solid.

$^1$H- NMR (300Hz, $CDCl_3$) δ: 9.14 (s, 1H), 9.03 (d, *J* = 2.4 Hz, 1H), 8.25- 8.33 (m, 3H), 8.00- 8.10 (m, 3H), 7.60- 7.67 (m, 3H), 7.54 (dd, *J*= 3.9, 8.7 Hz, 1H) .

Example 23:

3-[(6-quinolyl)difluoromethyl]-6-(2-thienyl)[1,2,4]triazolo[4,3-b][1,2,4]triazine **(I-23)**

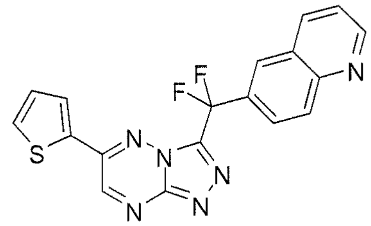

Step 1: 6-(2-thienyl)-3-[2,2-difluoro-2-(6-quinolyl)acetylhydrazino]-1,2,4-triazine **(VIII-23)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (2- thienyl)- 1, 2, 4- triazine (**VII- 3**) **,** and 6- quinolineacetic acid was replaced with 2, 2- difluoro- 2- (6- quinolyl) acetic acid. As a result, compound VIII- 23 was obtained as yellowish solid.

$^1$H- NMR (300Hz, DMSO- $d_6$) δ: 11.38 (s, 1H), 9.97 (br, 1H), 9.05 (m, 2H), 8.58 (d, *J* = 6.0 Hz, 1H), 8.46 (s, 1H), 8.22 (d, *J* = 9.0 Hz, 1H), 8.01 (d, *J* = 9.0 Hz, 1H), 7.82 (d, *J* = 3.3 Hz, 1H), 7.64- 7.68 (m, 2H), 7.18- 7.21 (m, 1H) .

Step 2: 3-[(6-quinolyl)difluoromethyl]-6-(2-thienyl)[1,2,4]triazolo[4,3-b][1,2,4]triazine **(I-23)**

All used starting materials, reagents and preparation method were the same as those used in step 7 of Example 1, except that 6- phenyl- 3- (6- quinolylacetyl hydrazino)- 1, 2, 4- triazine **(VIII- 1)** was replaced with 6- (2- thienyl)- 3- [2, 2- difluoro- 2- (6- quinolyl) acetylhydrazino]- 1, 2, 4- triazine (**VIII- 23**) **.** As a result, compound 1- 23 was obtained.

$^1$H- NMR (300Hz, $CDCl_3$) δ: 9.01 (s, 1H), 8.22- 8.32 (m, 3H), 8.09 (d, *J* = 8.7 Hz, 1H), 7.70 (d, *J*= 5.4 Hz, 1H), 7.51 (dd, *J*= 3.9, 8.7 Hz, 1H), 7.26 (s, 2H) .

Example 24:

3-[(6-quinolyl)difluoromethyl]-6-(4-benzyloxyphenyl)[1,2,4]triazolo[4,3-b][1,2,4]triazine **(I-24)**

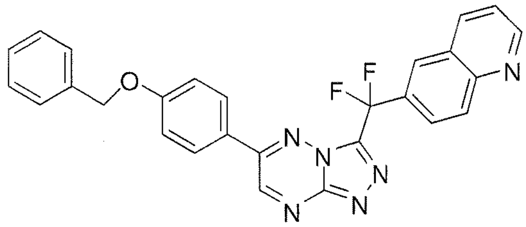

Step 1:

6-(4-benzyloxyphenyl)-3-[2,2-difluoro-2-(6-quinolyl)acetylhydrazino]-1,2,4-triazine **(VIII-24)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- benzyloxyphenyl)- 1, 2, 4- triazine **(VII- 2),** and 6- quinolineacetic acid was replaced with 2, 2- difluoro- 2- (6- quinolyl) acetic acid. As a result, compound VIII- 24 was obtained as yellowish solid.

$^1$H- NMR (300Hz, DMSO- $d_6$) $\delta$: 11.49 (br, 1H), 9.87 (s, 1H), 9.04 (d, *J*= 4.2 Hz, 1H), 8.97 (s, 1H), 8.59 (d, *J*= 9.3 Hz, 1H), 8.46 (s, 1H), 8.22 (d, *J*= 5.7 Hz, 1H), 7.97- 8.03 (m, 3H), 7.68 (dd, *J* = 3.9, 8.7 Hz, 1H), 7.16- 7.47 (m, 5H), 7.13 (d, *J*= 8.7 Hz, 2H), 5.17 (s, 2H) .

Step 2:

3-[(6-quinolyl)difluoromethyl]-6-(4-benzyloxyphenyl)[1,2,4]triazolo[4,3-b][1,2,4]triazine **(I-24)**

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (4- benzyloxyphenyl)- 3- [2, 2- difluoro- 2- (6- quinolyl) acetylhydrazino]- 1, 2, 4- triazine (VIII- 24) . As a result, compound 1- 24 was obtained.

$^1$H- NMR (300Hz, DMSO- $d_6$) $\delta$: 9.48 (s, 1H), 9.03 (d, *J*= 4.2 Hz, 1H), 8.58 (d, *J*= 8.4 Hz, 1H), 8.47 (s, 1H), 8.14- 8.20 (m, 2H), 7.99- 8.05 (m, 3H), 7.67 (dd, *J* = 3.9, 8.7 Hz, 1H), 7.25- 7.49 (m, 5H), 7.18- 7.21 (m, 2H), 5.22 (s, 2H) .

Example 25: 3-[(7-methoxy-4-quinolyl)oxymethyl]-6-(4-fluorophenyl)[1,2,4]triazolo[4,3-b] [1,2,4]triazine(**II-1**)

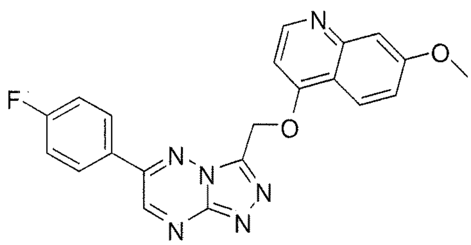

Step 1: 2-oxo-2-(4-fluorophenyl)acetaldehyde **(III-25)**

All used starting materials, reagents and preparation method were the same as those used in step 1 of Example 1, except that acetophenone was replaced with 4-fluoroacetophenone. As a result, compound III-25 was obtained as colorless oil.

Step 2: 2,2-diethoxy-1-(4-fluorophenyl)ethanone **(IV-25)**

All used starting materials, reagents and preparation method were the same as those used in step 2 of Example 1, except that 2- oxo- 2- phenylacetaldehyde **(III- 1)** was replaced with 2- oxo- 2- (4- fluorophenyl) acetaldehyde **(III- 25) .** As a result, compound IV- 25 was obtained as yellowish oil.
$^1$H- NMR (300Hz, $CDCl_3$) δ: 8.23 (m, 2H), 7.14 (m, 2H), 5.17 (s, 1H), 3.62- 3.79 (m, 4H), 1.27 (t, J = 7.2 Hz, 6H) .

Step 3: 3-methylthio-6-(4-fluorophenyl)-1,2,4-triazine **(V-25)**

All used starting materials, reagents and preparation method were the same as those used in step 3 of Example 1, except that 2, 2- diethoxyacetophenone **(IV- 1)** was replaced with 2, 2- diethoxy- 1- (4- fluorophenyl) ethanone **(IV- 25) .** As a result, compound V- 25 was obtained as yellowish solid.
$^1$H- NMR (300Hz, $CDCl_3$) δ: 8.75 (s, 1H), 8.07 (m, 2H), 7.26 (m, 2H), 2.72 (s, 3H) .

Step 4: 3-methylsulfinyl-6-(4-fluorophenyl)-1,2,4-triazine **(VI-25)**

All used starting materials, reagents and preparation method were the same as those used in step 4 of Example 1, except that 3- methylthio- 6- phenyl- 1, 2, 4- triazine **(V- 1)** was replaced with 3- methylthio- 6- (4- fluorophenyl)- 1, 2, 4- triazine **(V- 25) .** As a result, compound VI- 25 was obtained as yellowish solid.
$^1$H- NMR (300Hz, $CDCl_3$) δ: 9.15 (s, 1H), 8.20 (m, 2H), 7.33 (m, 2H), 3.13 (s, 3H) .

Step 5: 3-hydrazino-6-(4-fluorophenyl)-1,2,4-triazine **(VII-25)**

All used starting materials, reagents and preparation method were the same as those used in step 5 of Example 1, except that 3- methylsulfinyl- 6- phenyl- 1, 2, 4- triazine **(VI- 1)** was replaced with 3- methylsulfinyl- 6- (4- fluorophenyl)- 1, 2, 4- triazine **(VI- 25) .** As a result, compound VII- 25 was obtained as yellowish solid.

Step 6: 6-(4-fluorophenyl)-3-[(7-methoxy-4-quinolyl)oxyacetylhydrazino]-1,2,4-triazine **(VIII-25)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- fluorophenyl)- 1, 2, 4- triazine (**VII- 25**), and 6- quinolineacetic acid was replaced with 7- methoxy- 4- quinolyloxyacetic acid. As a result, compound VIII- 25 was obtained as yellow solid.
$^1$H- NMR (300Hz, DMSO- $d_6$) δ: 10.52 (s, 1H), 9.83 (br, 1H), 8.99 (s, 1H), 8.69 (d, *J*= 5.1 Hz, 1H), 8.32 (d, *J* = 9.3 Hz, 1H), 8.11 (m, 2H), 7.32- 7.39 (m, 3H), 7.22 (dd, *J* = 2.4, 9.3 Hz, 1H), 6.94 (d, *J* = 5.4 Hz, 1H), 4.93 (s, 1H), 3.89 (s, 3H) .

Step 7:

3-[(7-methoxy-4-quinolyl)oxymethyl]-6-(4-fluorophenyl)[[1,2,4]triazolo[4,3-b][1,2,4]triazine **(II-1)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- fluorophenyl)- 3- [(7- methoxy- 4- quinolyl) oxyacetylhydrazino]- 1, 2, 4- triazine **(VIII- 25) .** As a result, compound II- 1 was obtained as white solid.
$^1$H- NMR (300Hz, DMSO- $d_6$) δ: 9.44 (s, 1H), 8.73 (d, *J*= 5.1 Hz, 1H), 8.22 (m, 2H), 7.99 (d, *J*= 8.7 Hz, 1H), 7.40- 7.46 (m, 2H), 7.32 (m, 2H), 7.14 (dd, *J*= 2.7, 9.3 Hz, 1H) .

Example 26:

3-[(7-methoxy-4-quinolyl)oxymethyl]-6-(4-chlorophenyl)[1,2,4]triazolo[4,3-b][1,2,4]triazine **(II-2)**

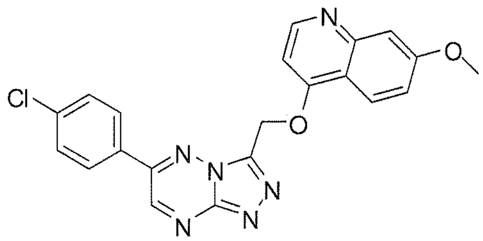

Step 1: 6-{4-chlorophenyl)-3-[(7-methoxy-4-quinolyl)oxyacetylhydrazino]-1,2,4-triazine **(VIII-26)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- chlorophenyl)- 1, 2, 4- triazine **(VII- 16),** and 6- quinolineacetic acid was replaced with 7- methoxy- 4- quinolyloxy acetic acid. As a result, compound VIII- 26 was obtained as yellow solid.

$^1$H- NMR (300Hz, DMSO- $d_6$) δ: 10.53 (br, 1H), 9.88 (br, 1H), 9.01 (s, 1H), 8.68 (d, *J*= 5.4 Hz, 1H), 8.29 (d, *J* = 7.3 Hz, 1H), 8.07 (d, *J* = 8.7 Hz, 2H), 7.58 (d, *J* = 8.7 Hz, 2H), 7.32 (d, *J* = 2.4 Hz, 1H), 7.21 (dd, *J*= 2.4, 9.3 Hz, 1H), 6.94 (d, *J*= 5.4 Hz, 1H), 4.97 (s, 2H), 3.89 (s, 3H) .

Step 2:

3-[(7-methoxy-4-quinolyl)oxymethyl]-6-(4-chlorophenyl)[1,2,4]triazolo[4,3-b][1,2,4]triazine **(II-2)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- chlorophenyl)- 3- [(7- methoxy- 4- quinolyl) oxyacetylhydrazino]- 1, 2, 4- triazine **(VIII- 26) .** As a result, compound II- 2 was obtained as white solid.

$^1$H- NMR (300Hz, DMSO- $d_6$) δ: 9.43 (s, 1H), 8.72 (d, *J*= 5.4 Hz, 1H), 8.17 (d, *J*= 8.7 Hz, 2H), 7.98 (d, *J*= 9.3 Hz, 1H), 7.67 (d, *J*= 8.7 Hz, 2H), 7.32 (d, *J*= 2.7 Hz, 1H), 7.29 (d, *J*= 5.4 Hz, 1H), 7.13 (dd, *J*= 2.7, 9.3 Hz, 1H), 5.97 (s, 2H), 3.872 (s, 2H)

Example 27:

3-[(7-methoxy-4-quinolyl)oxymethyl]-6-(4-bromophenyl)[1,2,4]triazolo[4,3-b][1,2,4]triazine **(II-3)**

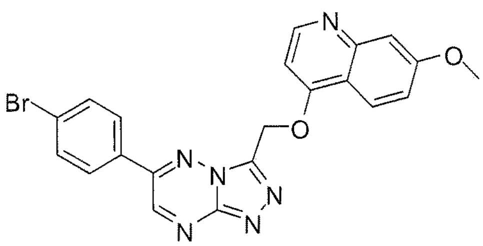

Step 1: 6-(4-bromophenyl)-3-[(7-methoxy-4-quinolyl)oxyacetylhydrazino]-1,2,4-triazine **(VIII-27)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- bromophenyl)- 1, 2, 4- triazine **(VII- 15),** and 6- quinolineacetic acid was replaced with 7- methoxy- 4- quinolyloxy acetic acid. As a result, compound VIII- 27 was obtained as yellow solid.

$^1$H- NMR (300Hz, DMSO- $d_6$) δ: 10.55 (s, 1H), 9.91 (br, 1H), 9.03 (s, 1H), 8.70 (d, *J*= 5.4 Hz, 1H), 8.32 (d, *J*= 9.3 Hz, 1H), 8.02 (d, *J*= 8.4 Hz, 2H), 7.74 (d, *J*= 8.4 Hz, 2H), 7.34 (d, *J*= 2.1 Hz, 1H), 7.22 (dd, *J*= 2.1, 9.3 Hz, 1H), 3.92 (s, 3H), 6.95 (d, *J*= 5.4 Hz, 1H), 4.99 (s, 2H) .

Step 2:

3-[(7-methoxy-4-quinolyl)oxymethyl]-6-(4-bromophenyl)[1,2,4]triazolo[4,3-b][1,2,4]triazine **(II-3)**

All used starting materials, reagents and preparation method were the same as those used in step 6 of Example 1, except that 3- hydrazino- 6- phenyl- 1, 2, 4- triazine **(VII- 1)** was replaced with 6- (4- bromophenyl)- 3- [(7- methoxy- 4- quinolyl) oxyacetylhydrazino]- 1, 2, 4- triazine **(VIII- 27),** and 6- quinolineacetic acid was replaced with 7- methoxy- 4- quinolyloxy acetic acid. As a result, compound II- 3 was obtained as yellow solid.

$^1$H- NMR (300Hz, DMSO- $d_6$) $\delta$: 9.45 (s, 1H), 8.73 (d, *J*= 5.1 Hz, 1H), 8.09 (d, *J*= 8.7 Hz, 2H), 7.99 (d, *J* = 9.3 Hz, 1H), 7.82 (d, *J* = 8.7 Hz, 2H), 7.34 (d, *J* = 2.7 Hz, 1H), 7.31 (d, *J* = 5.1 Hz, 1H), 7.14 (dd, *J*= 2.7, 9.3 Hz, 1H), 5.99 (s, 2H), 3.89 (s, 3H) .

Example 28:

3-[(7-methoxy-4-quinolyl)oxymethyl]-6-(2-thienyl)[1,2,4]triazolo[4,3-b][1,2,4]triazine **(II-4)**

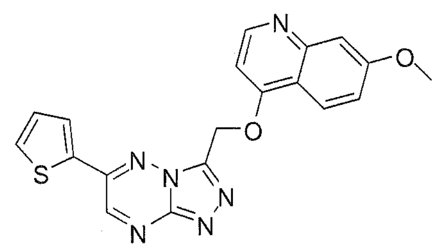

Step 1: 6-(2-thienyl)-3-[(7-methoxy-4-quinolyl)oxyacetylhydrazino]-1,2,4-triazine **(VIII-28)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (2- thienyl)- 1, 2, 4- triazine **(VII- 3),** and 6- quinolineacetic acid was replaced with 7- methoxy- 4- quinolyloxy acetic acid. As a result, compound VIII- 28 was obtained as yellow solid.

$^1$H- NMR (300Hz, DMSO- $d_6$) $\delta$: 10.51 (s, 1H), 9.82 (br, 1H), 9.04 (s, 1H), 8.69 (d, *J*= 5.1 Hz, 1H), 8.32 (d, *J*= 9.3 Hz, 2H), 7.81 *(d, J*= 3.3 Hz, 1H), 7.68 (d, *J*= 4.8 Hz, 1H), 7.33 (d, *J*= 2.4 Hz, 1H), 7.21 (m, 2H), 6.94 (d, *J*= 6.0 Hz, 1H), 4.96 (s, 2H), 3.89 (s, 3H) .

Step 2:

3-[(7-methoxy-4-quinolyl)oxymethyl]-6-(2-thienyl)[1,2,4]triazolo[4,3-b][1,2,4]triazine **(II-4)**

All used starting materials, reagents and preparation method were the same as those used in step 6 of Example 1, except that 3- hydrazino- 6- phenyl- 1, 2, 4- triazine **(VII- 1)** was replaced with 6- (2- thienyl)- 3- [(7- methoxy- 4- quinolyl) oxyacetylhydrazino]- 1, 2, 4- triazine **(VIII- 28),** and 6- quinolineacetic acid was replaced with 7- methoxy- 4- quinolyloxy acetic acid. As a result, compound II- 4 was obtained as yellow solid.

$^1$H- NMR (300Hz, DMSO- $d_6$) $\delta$: 9.45 (s, 1H), 8.72 (dd, *J*= 1.5, 5.1 Hz, 1H), 8.29 (d, *J* = 3.6 Hz, 1H), 8.00 (d, *J* = 9.0 Hz, 1H), 7.93 (dd, *J* = 1.5, 5.1 Hz, 1H), 7.28- 7.32 (m, 3H), 7.13 (dd, *J*= 1.5, 5.1 Hz, 1H), 5.90 (s, 2H), 3.87 (s, 3H) .

Example 29:

3-[(7-methoxy-4-quinolyl)oxymethyl]-6-(4-methoxyformylphenyl)[[1,2,4]triazolo[[4,3-b][[1,2,4]t riazine **(II-5)**

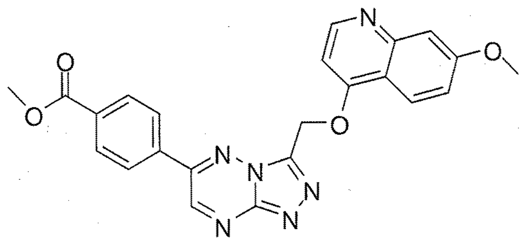

Step 1: 4-(2-oxoacetyl)methyl benzoate **(III-29)**

All used starting materials, reagents and preparation method were the same as those used in step 1 of Example 1, except that acetophenone was replaced with 4-acetyl methyl benzoate. As a result, compound III-29 was obtained as colorless oil.

$^1$H- NMR (300Hz, $CDCl_3$) δ: 9.662 (s, 1H), 8.284 (d, *J*= 8.7 Hz, 2H), 8.181 (d, *J*= 8.7 Hz, 2H), 3.965 (s, 3H) .

Step 2: 4-[(2,2-diethoxy)acetyl]methyl benzoate **(IV-29)**

All used starting materials, reagents and preparation method were the same as those used in step 2 of Example 1, except that 2- oxo- 2- phenylacetaldehyde **(III- 1)** was replaced with 4- (2- oxoacetyl) methyl benzoate **(III- 29) .** As a result, compound IV- 29 was obtained as yellowish oil.

$^1$H- NMR (300Hz, $CDCl_3$) δ: 8.203 (d, *J*= 9 Hz 2H), 8.087 (d, *J*= 9 Hz, 2H), 5.199 (s, 1H), 3.916 (s, 3H), 3.805-3.597 (m, 4H), 1.217 (t, *J* = 6.6 Hz, 6H)

Step 3: 3-methylthio-6-(4-methoxyformylphenyl)-1,2,4-triazine **(V-29)**

All used starting materials, reagents and preparation method were the same as those used in step 3 of Example 1, except that 2, 2- diethoxyacetophenone **(IV- 1)** was replaced with 4- [(2, 2- diethoxy) acetyl] methyl benzoate **(IV-29) .** As a result, compound V- 29 was obtained as yellowish solid.

$^1$H- NMR (300Hz, $CDCl_3$) δ: 8.817 (s, 1H), 8.227 (d, *J*= 8.7 Hz, 2H), 8.146 (d, *J*= 8.7 Hz, 2H), 3.967 (s, 3H), 2.741 (s, 3H)

Step 4: 3-methylsulfinyl-6-(4-methoxyformylphenyl)-1,2,4-triazine **(VI-29)**

All used starting materials, reagents and preparation method were the same as those used in step 4 of Example 1, except that 3- methylthio- 6- phenyl- 1, 2, 4- triazine **(V- 1)** was replaced with 3- methylthio- 6- (4- methoxyformylphenyl)-1, 2, 4- triazine **(V- 29) .** As a result, compound VI- 29 was obtained as yellowish solid.

Step 5: 3-hydrazino-6-(4-methoxyformylphenyl)-1,2,4-triazine **(VII-29)**

All used starting materials, reagents and preparation method were the same as those used in step 5 of Example 1, except that 3- methylsulfinyl- 6- phenyl- 1, 2, 4- triazine **(VI- 1)** was replaced with 3- methylsulfinyl- 6- (4- methoxyformylphenyl)- 1, 2, 4- triazine **(VI- 29) .** As a result, compound VII- 29 was obtained as yellowish solid.

$^1$H- NMR (300Hz, DMSO- $d_6$) δ: 9.057 (br, 1H), 8.929 (s, 1H), 8.182 (d, *J*= 8.4 Hz, 2H), 8.069 (d, *J*= 8.4 Hz, 2H), 4.496 (br, 2H), 3.868 (s, 3H) .

Step 6:

6-(4-methoxyformylphenyl)-3-[(7-methoxy-4-quinolyl)oxyacetylhydrazino]-1,2,4-triazine **(VIII-29)**

All used starting materials, reagents and preparation method were the same as those used in step 6 of Example 1, except that 6- quinolineacetic acid was replaced with 7- methoxy- 4- quinolyloxy acetic acid, and 3- hydrazino- 6-phenyl- 1, 2, 4- triazine **(VII- 1)** was replaced with 3- hydrazino- 6- (4- methoxyformylphenyl)- 1, 2, 4- triazine **(VII- 29) .** As a result, compound VIII- 29 was obtained as yellow solid.

$^1$H- NMR (300Hz, DMSO- $d_6$) δ: 10.539 (s, 1H), 10.005 (br, 1H), 9.101 (s, 1H), 8.722 (d, *J* = 5.4 Hz, 1H), 8.339 (d, *J* = 8.7 Hz, 1H), 8.245 (d, *J* = 8.4 Hz, 2H), 8.118 (d, *J*= 8.4 Hz, 2H), 7.351 (d, *J*= 2.4 Hz, 1H, 7.244 (dd, *J*= 2.4, 8.7 Hz, 1H), 6.970 (d, *J*= 5.4 Hz, 1H), 5.005 (s, 2H), 3.916 (s, 3H), 3.894 (s, 3H) .

Step 7:

3-[(7-methoxy-4-quinolyl)oxymethyl]-6-(4-methoxyformylphenyl)[1,2,4]triazolo[4,3-b][1,2,4]t riazine **(II-5)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- methoxyformylphenyl)- 3- [(7- methoxy- 4- quinolyl) oxyacetylhydrazino]- 1, 2, 4- triazine **(VIII- 29) .** As a result, compound II- 5 was obtained as white solid.

$^1$H- NMR (300Hz, DMSO- $d_6$) δ: 9.501 (s, 1H), 8.756 (d, *J*= 5.4 Hz, 1H), 8.299 (d, *J*= 6.6 Hz, 2H), 8.148 (d, *J* = 6.6 Hz, 2H, 8.018 (d, *J* = 10.2 Hz, 1H), 7.352 (d, *J* = 2.1 Hz, 1H), 7.330 (d, *J*= 5.4 Hz, 1H), 7.158 (dd, *J*= 2.1, 10.2 Hz, 1H), 6.007 (s, 2H), 3.905 (s, 3H), 3.891 (s, 3H) .

Example 30:

3-[(7-methoxy-4-quinolyl)oxymethyl]-6-(4-nitrophenyl)[1,2,4]triazolo[4,3-b][1,2,4]triazine **(II-6)**

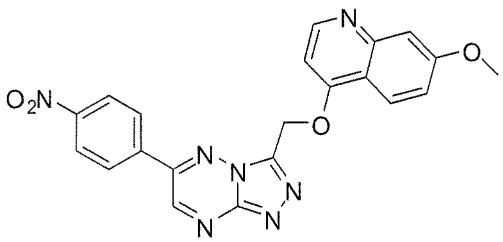

Step1: 2-oxo-2-(4-nitrophenyl)acetaldehyde **(III-30)**

All used starting materials, reagents and preparation method were the same as those used in step 1 of Example 1, except that acetophenone was replaced with nitroacetophenone. As a result, compound III-30 was obtained as yellow solid.

$^1$H- NMR (300Hz, $CDCl_3$) δ: 8.36 (d, *J* = 8.7 Hz, 2H), 8.23 (d, *J* = 8.7 Hz, 2H) .

Step 2: 2,2-diethoxy-1-(4-nitrophenyl)ethanone **(IV-30)**

All used starting materials, reagents and preparation method were the same as those used in step 2 of Example 1, except that 2- oxo- 2- phenylacetaldehyde **(III- 1)** was replaced with 2- oxo- 2- (4- nitrophenyl) acetaldehyde **(III- 30) .** As a result, compound IV- 30 was obtained as yellowish oil.

$^1$H- NMR (300Hz, $CDCl_3$) δ: 8.35 (d, *J* = 8.7 Hz, 2H), 8.28 (d, *J*= 8.7 Hz, 2H), 5.13 (s, 1H), 3.62- 3.84 (m, 4H), 1.27 (t, *J*= 7.8 Hz, 6H) .

Step 3: 3-methylthio-6-(4-nitrophenyl)-1,2,4-triazine **(V-30)**

All used starting materials, reagents and preparation method were the same as those used in step 3 of Example 1, except that 2, 2- diethoxyacetophenone **(IV- 1)** was replaced with 2, 2- diethoxy- 1- (4- nitrophenyl) ethanone **(IV- 30) .** As a result, compound V- 30 was obtained as yellowish solid.

Step 4: 3-methylsulfinyl-6-(4-nitrophenyl)-1,2,4-triazine **(VI-30)**

All used starting materials, reagents and preparation method were the same as those used in step 4 of Example 1, except that 3- methylthio- 6- phenyl- 1, 2, 4- triazine **(V- 1)** was replaced with 3- methylthio- 6- (4- nitrophenyl)- 1, 2, 4- triazine **(V- 30) .** As a result, compound VI- 30 was obtained as yellowish solid.

Step 5: 3-hydrazino-6-(4-nitrophenyl)-1,2,4-triazine **(VII-30)**

All used starting materials, reagents and preparation method were the same as those used in step 5 of Example 1, except that 3- methylsulfinyl- 6- phenyl- 1, 2, 4- triazine **(VI- 1)** was replaced with 3- methylsulfinyl- 6- (4- nitrophenyl)-1, 2, 4- triazine **(VI- 30)**. As a result, compound VII- 30 was obtained as yellowish solid.

$^1$H- NMR (300Hz, DMSO- $d_6$) δ: 9.15 (br, 1H), 8.99 (s, 1H), 8.27- 8.39 (m, 4H), 4.58 (br, 2H)

Step 6: 6-(4-nitrophenyl)-3-[(7-methoxy-4-quinolyl)oxyacetylhydrazino]-1,2,4-triazine **(VIII-30)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- nitrophenyl)- 1, 2, 4- triazine **(VII- 30),** and 6- quinoline acetic acid was replaced with 7- methoxy- 4- quinolyloxy acetic acid. As a result, compound VIII- 30 was obtained as yellow solid.

$^1$H- NMR (300Hz, DMSO- $d_6$) δ: 10.61 (s, 1H), 10.12 (br, 1H), 9.13 (s, 1H), 8.71 (dd, *J*= 3.0, 8.1 Hz, 1H), 7.33 (s, 1H), 7.23 (dd, *J* = 3.6, 6.6 Hz, 1H), 6.96 (dd, *J* = 3.6, 6.9 Hz, 1H), 4.99 (s, 2H), 3.92 (s, 3H) .

Step 7:

3-[(7-methoxy-4-quinolyl)oxymethyl]-6-(4-nitrophenyl)[1,2,4]triazolo[4,3-b][[1,2,4]triazine **(II-6)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- fluorophenyl)- 3- [(7- methoxy- 4- quinolyl) oxyacetylhydrazino]- 1, 2, 4- triazine **(VIII- 25)**. As a result, compound II- 6 was obtained as white solid.

$^1$H- NMR (300Hz, DMSO- $d_6$) δ: 9.51 (s, 1H), 8.73 (d, *J* = 8.7 Hz, 1H), 8.39 (s, 4H), 7.99 (d, *J*= 9.3 Hz, 1H), 7.33 (m, 2H), 7.14 (dd, *J*= 2.7, 9.3 Hz, 1H), 6.00 (s, 2H), 3.87 (s, 3H) .

Example 31:

3-[(7-methoxy-4-quinolyl)oxymethyl]-6-phenyl[1,2,4]triazolo[4,3-b][1,2,4]triazine **(II-7)**

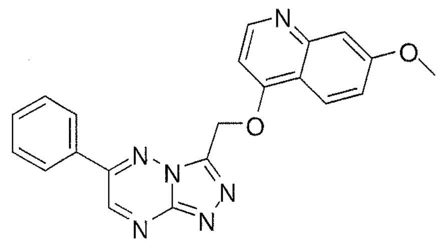

Step 1: 6-phenyl-3-[(7-methoxy-4-quinolyl)oxyacetylhydrazino]-1,2,4-triazine **(VIII-31)**

All used starting material, reagents and preparation method were the same as those used in step 6 of Example 1, except that 6- quinolineacetic acid was replaced with 7- methoxy- 4- quinolyloxy acetic acid. As a result, compound VIII- 31 was obtained as yellow solid.

$^1$H- NMR (300Hz, DMSO- $d_6$) δ: 10.52 (s, 1H), 9.82 (br, 1H), 8.99 (s, 1H), 8.69 (d, *J*= 5.4 Hz, 1H), 8.32 (d, *J*= 9.3 Hz, 1H), 8.05 (d, *J*= 8.4 Hz, 2H), 7.47- 7.58 (m, 3H), 7.33 (d, *J*= 5.7 Hz, 1H), 7.22 (dd, *J*= 2.4, 9.3 Hz, 1H), 6.94 (d, *J*= 5.1Hz, 1H) .

Step 2: 3-[(7-methoxy-4-quinolyl)oxymethyl]-6-phenyl[1,2,4]triazolo[4,3-b][1,2,4]triazine **(II-7)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- phenyl- 3- [(7- methoxy- 4- quinolyl) oxyacetylhydrazino]- 1, 2, 4- triazine **(VIII- 31)**. As a result, compound II- 7 was obtained as white solid.

$^1$H- NMR (300Hz, DMSO- $d_6$) δ: 9.44 (s, 1H), 8.72 (br, 1H), 8.14 (d, *J* = 4.8 Hz, 2H), 7.99 (d, *J*= 9.9 Hz, 1H), 7.62 (m, 3H), 7.34 (m, 2H), 7.13 (d, *J*= 4.8 Hz, 1H), 5.97 (s, 1H), 3.72 (s, 3H) .

Example 32:

3-[(7-methoxy-4-quinolyl)oxymethyl]-6-(4-benzyloxyphenyl)[1,2,4]triazolo[4,3-b][1,2,4]triazi ne **(II-8)**

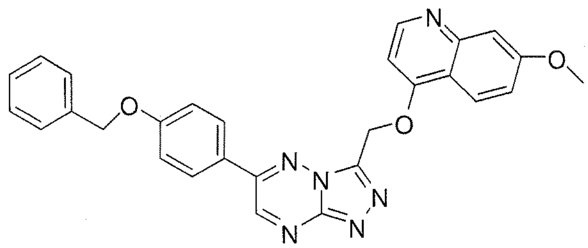

Step 1:

6-(4-benzyloxyphenyl)-3-[(7-methoxy-4-quinolyl)oxyacetylhydrazino]-1,2,4-triazine **(VIII-32)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- benzyloxyphenyl)- 1, 2, 4- triazine **(VII- 2)** , and 6- quinolineacetic acid was replaced with 7- methoxy- 4- quinolyloxyacetic acid. As a result, compound VIII- 32 was obtained as yellow solid.

Step 2:

3-[(7-methoxy-4-quinolyl)oxymethyl]-6-(4-benzylphenyl)[1,2,4]triazolo[4,3-b][1,2,4]triazine **(II-8)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- benzyloxyphenyl)- 3- [(7- methoxy- 4- quinolyl) oxyacetylhydrazino]- 1, 2, 4- triazine **(VIII- 32)** . As a result, compound II- 8 was obtained as white solid.

$^1$H- NMR (300Hz, DMSO- $d_6$) $\delta$: 9.48 (s, 1H), 9.21 (d, *J*=6.6 Hz, 1H), 8.22 (d, *J*=9 Hz, 1H), 8.14 (d, *J*=8.7 Hz, 2H), 7.86 (d, *J*=6.6 Hz, 1H), 7.66 (d, *J*=2.4 Hz, 1H), 7.32- 7.49 (m, 6H), 7.23 (d, *J*=8.7 Hz, 2H), 6.31 (s, 2H), 5.24 (s, 2H), 3.98 (s, 3H) .

Example 33:

3-[(7-methoxy-4-quinolyl)oxymethyl]-6-(4-methoxyphenyl)[1,2,4]triazolo[4,3-b][1,2,4]triazine **(II-9)**

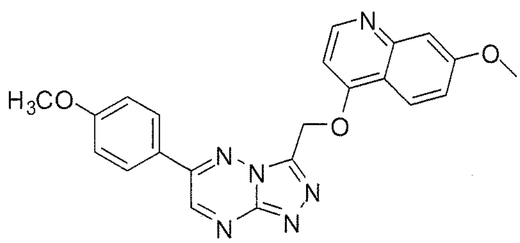

Step 1: 2-oxo-2-(4-methoxyphenyl)acetaldehyde **(III-33)**

All used starting materials, reagents and preparation method were the same as those used in step 1 of Example 1, except that acetophenone was replaced with methoxyacetophenone. As a result, compound III-33 was obtained.

Step 2: 2,2-diethoxy-1-(4-methoxyphenyl)ethanone **(IV-33)**

All used starting materials, reagents and preparation method were the same as those used in step 2 of Example 1, except that 2- oxo- 2- phenylacetaldehyde **(III- 1)** was replaced with 2- oxo- 2- (4- methoxyphenyl) acetaldehyde **(III-33) .** As a result, compound IV- 33 was obtained as yellowish oil.

Step 3: 3-methylthio-6-(4-methoxyphenyl)-1,2,4-triazine **(V-33)**

All used starting materials, reagents and preparation method were the same as those used in step 3 of Example 1, except that 2, 2- diethoxyacetophenone **(IV- 1)** was replaced with 2, 2- diethoxy- 1- (4- methoxyphenyl) ethanone **(IV- 33) .** As a result, compound V- 33 was obtained as yellowish solid.

$^1$H- NMR (300Hz, $CDCl_3$) δ: 8.74 (s, 1H), 8.01 (d, *J*=9 Hz, 2H), 7.06 (d, *J*=9 Hz, 2H), 3.89 (s, 3H), 2.72 (s, 3H) .

Step 4: 3-methylsulfinyl-6-(4-methoxyphenyl)-1,2,4-triazine **(VI-33)**

All used starting materials, reagents and preparation method were the same as those used in step 4 of Example 1, except that 3- methylthio- 6- phenyl- 1, 2, 4- triazine **(V- 1)** was replaced with 3- methylthio- 6- (4- methoxyphenyl)- 1, 2, 4- triazine **(V- 33) .** As a result, compound VI- 33 was obtained as yellowish solid.

Step 5: 3-hydrazino-6-(4-methoxyphenyl)-1,2,4-triazine **(VII-33)**

All used starting materials, reagents and preparation method were the same as those used in step 5 of Example 1, except that 3- methylsulfinyl- 6- phenyl- 1, 2, 4- triazine **(VI- 1)** was replaced with 3- methylsulfinyl- 6- (4- methoxyphenyl)- 1, 2, 4- triazine **(VI- 33) .** As a result, compound VII- 33 was obtained as yellowish solid.

$^1$H- NMR (300Hz, DMSO- $d_6$) δ: 8.83 (s, 1H), 8.74 (br, 1H), 7.96 (d, *J*=8.7 Hz, 2H), 7.07 (d, *J*=8.7 Hz, 2H), 4.41 (s, 2H), 3.82 (s, 3H) .

Step 6: 6-(4-methoxyphenyl)-3-[(7-methoxy-4-quinolyl)oxyacetylhydrazino]-1,2,4-triazine **(VIII-33)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- methoxyphenyl)- 1, 2, 4- triazine **(VII- 33),** and 6- quinoline acetic acid was replaced with 7- methoxy- 4- quinolyloxy acetic acid. As a result, compound VIII- 33 was obtained as yellow solid.

$^1$H- NMR (300Hz, DMSO- $d_6$) δ: 10.52 (s, 1H), 9.73 (br, 1H), 8.98 (s, 1H), 8.70 (d, *J*=5.1 Hz, 1H), 8.32 (d, *J*=9.6 Hz, 1H), 8.01 (d, *J*=8.7 Hz, 2H), 7.34 (d, *J*=2.1 Hz, 1H), 7.22 (dd, *J*=2.1, 9.6 Hz, 1H), 7.10 (d, *J*=8.7 Hz, 2H), 6.95 (d, *J*=5.1 Hz, 1H), 4.98 (s, 2H), 3.92 (s, 3H), 3.83 (s, 3H) .

Step 7:

3-[(7-methoxy-4-quinolyl)oxymethyl]-6-(4-methoxyphenyl)[1,2,4]triazolo[4,3-b][1,2,4]triazine **(II-9)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- methoxyphenyl)- 3- (6- quinolylacetylhydrazino)- 1, 2, 4- triazine **(VIII- 33) .** As a result, compound II- 9 was obtained as white solid.

$^1$H- NMR (300Hz, DMSO- $d_6$) δ: 9.45 (s, 1H), 8.74 (d, *J*=5.4 Hz, 1H), 8.13 (d, *J*=8.4 Hz, 2H), 8.00 (d, *J*=9.6 Hz, 1H), 7.33 (t, J=5.4, 4.2 Hz, 2H), 7.14 (d, *J*=9 Hz, 3H), 5.97 (s, 2H), 3.89 (s, 3H), 3.85 (s, 3H) .

Example 34:

3-[(7-methoxy-4-quinolyl)oxymethyl]-6-(4-hydroxylphenyl)[1,2,4]triazolo[4,3-b][1,2,4]triazine **(II-10)**

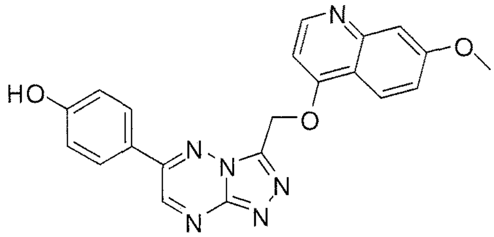

Step 1: 4-(2-oxoacetyl)phenyl benzoate **(III-34)**

All used starting materials, reagents and preparation method were the same as those used in step 1 of Example 1, except that acetophenone was replaced with 4-acetylphenylbenzoate. As a result, compound III-34 was obtained.

$^1$H- NMR (300Hz, $CDCl_3$) δ: 8.21 (d, *J*= 7.2 Hz, 2H), 7.16 (d, *J*= 8.7 Hz, 2H), 7.68 (t, *J*= 7.8, 7.8 Hz, 1H), 7.54 (t, *J* = 7.2, 7.8 Hz, 2H), 7.39 (d, *J* = 8.7 Hz, 2H) .

Step 2: 4-[2,2-diethoxy)acetyl]phenylbenzoate **(IV-34)**

All used starting materials, reagents and preparation method were the same as those used in step 2 of Example 1, except that 2- oxo- 2- phenylacetaldehyde **(III- 1)** was replaced with 4- (2- oxoacetyl) phenyl benzoate **(III- 34) .** As a result, compound IV- 34 was obtained as yellowish oil.

$^1$H- NMR (300Hz, $CDCl_3$) δ: 8.28 (d, *J*= 8.7 Hz, 2H), 8.21 (d, *J*= 8.4 Hz, 2H), 7.67 (t, *J*= 7.8, 7.8 Hz, 1H), 7.52 (t, *J* = 8.4, 7.8 Hz, 2H), 7.32 (d, *J* = 8.7 Hz, 2H), 5.24 (s, 1H), 3.61- 3.84 (m, 4H), 1.26 (t, 6H) .

Step 3: 4-[6-(3-methylthio)-1,2,4-triazinyl]phenylbenzoate **(V-34)**

All used starting materials, reagents and preparation method were the same as those used in step 3 of Example 1, except that 2, 2- diethoxyacetophenone **(IV- 1)** was replaced with 4- [2, 2- diethoxy) acetyl] phenylbenzoate **(IV- 34) .** As a result, compound V- 34 was obtained as yellowish solid.

$^1$H- NMR (300Hz, $CDCl_3$) δ: 8.80 (s, 1H), 8.23 (d, *J* = 7.35 Hz, 2H), 8.14 (d, *J* = 8.7 Hz, 2H), 7.68 (t, *J* = 7.8, 7.8 Hz, 1H), 7.54 (t, *J* = 7.35, 7.8 Hz, 2H), 7.43 (d, *J* = 8.7 Hz, 2H), 2.74 (s, 3H) .

Step 4: 4-[6-(3-methylsulfinyl)-1,2,4-triazinyl]phenylbenzoate **(VI-34)**

All used starting materials, reagents and preparation method were the same as those used in step 4 of Example 1, except that 3- methylthio- 6- phenyl- 1, 2, 4- triazine **(V- 1)** was replaced with 4- [6- (3- methylthio)- 1, 2, 4- triazinyl] phenylbenzoate **(V- 34) .** As a result, compound VI- 34 was obtained as yellowish solid.

Step 5: 3-hydrazino-6-(4-hydroxylphenyl)-1,2,4-triazine **(VII-34)**

All used starting materials, reagents and preparation method were the same as those used in step 5 of Example 1, except that 3- methylsulfnyl- 6- phenyl- 1, 2, 4- triazine **(VI- 1)** was replaced with 4- [6- (3- methylsulfinyl)- 1, 2, 4- triazinyl] phenylbenzoate (VI- 34) . As a result, compound VII- 34 was obtained as yellowish solid.

$^1$H- NMR (300Hz, DMSO- $d_6$) δ: 9.75 (s, 1H), 8.77 (s, 1H), 8.66 (br, 1H), 7.84 (d, *J*=8.4 Hz, 2H), 6.88 (d, *J*=8.4 Hz, 2H), 4.38 (br, 2H) .

Step 6: 6-(4-hydroxylphenyl)-3-[(7-methoxy-4-quinolyl)oxyacetylhydrazino]-1,2,4-triazine **(VIII-34)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- hydroxylphenyl)- 1, 2, 4- triazine **(VII- 34),** and 6- quinoline acetic acid was replaced with 7- methoxy- 4- quinolyloxy acetic acid. As a result, compound VIII- 34 was obtained as yellowish solid.

Step 7:

3-[(7-methoxy-4-quinolyl)oxymethyl]-6-(4-hydroxylphenyl)[1,2,4]triazolo[4,3-b][1,2,4]triazine **(II-10)**

All used starting materials, reagents and preparation method were the same as those used in step 7 of Example

1, except that 6- phenyl- 3- (6- quinolylacetylhydrazino)- 1, 2, 4- triazine **(VIII- 1)** was replaced with 6- (4- hydroxylphenyl)- 3- (6- quinolylacetylhydrazino)- 1, 2, 4- triazine **(VIII- 34)** . As a result, compound II- 10 was obtained as white solid.

$^1$H- NMR (300Hz, DMSO- $d_6$) δ: 10.33 (s, 1H), 9.40 (s, 1H), 8.82 (d, *J*=5.4 Hz, 1H), 8.11 (d, *J*=7.5 Hz, 1H), 8.02 (d, *J*=8.7 Hz, 2H), 7.98 (d, *J*=8.1 Hz, 1H), 7.74 (t, *J*=6.9, 8.4 Hz, 1H), 7.52 (t, *J*=6.9, 8.1 Hz, 1H), 7.45 (d, *J*=5.4 Hz, 1H), 6.93 (d, *J*=8.7 Hz, 2H), 5.99 (s, 2H) .

Example 35:

3-[(7-methoxy-4-quinolyl)oxymethyl]-6-(3-hydroxylphenyl)[1,2,4]triazolo[4,3-b] [1,2,4]triazine **(II-11)**

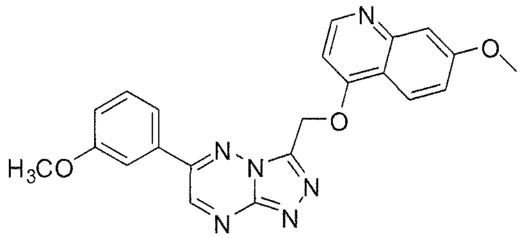

Step 1: 2-oxo-2-(3-methoxyphenyl)acetaldehyde **(III-35)**

All used starting materials, reagents and preparation method were the same as those used in step 1 of Example 1, except that acetophenone was replaced with 3-methoxyacetophenone. As a result, compound III-35 was obtained.

Step 2: 2,2-diethoxy-1-(3-methoxyphenyl)ethanone **(IV-35)**

All used starting materials, reagents and preparation method were the same as those used in step 2 of Example 1, except that 2- oxo- 2- phenylacetaldehyde **(III- 1)** was replaced with 2- oxo- 2- (3- methoxyphenyl) acetaldehyde **(III-35)** . As a result, compound IV- 35 was obtained as yellowish oil.

Step 3: 3-methylthio-6-(3-methoxyphenyl)-1,2,4-triazine **(V-35)**

All used starting materials, reagents and preparation method were the same as those used in step 3 of Example 1, except that 2, 2- diethoxyacetophenone **(IV- 1)** was replaced with 2, 2- diethoxy- 1- (3- methoxyphenyl) ethanone **(IV- 35)** . As a result, compound V- 35 was obtained as yellowish solid.

$^1$H- NMR (300Hz, $CDCl_3$) δ: 8.77 (s, 1H), 7.68 (d, *J*= 3.3 Hz, 1H), 7.54 (d, *J*= 7.5 Hz, 1H), 7.44 (t, *J*= 7.8, 7.5 Hz, 1H), 7.07 (dd, *J*= 3.3, 7.8 Hz, 1H), 3.90 (s, 3H), 2.73 (s, 3H) .

Step 4: 3-methylsulfinyl-6-(3-methoxyphenyl)-1,2,4-triazine **(VI-35)**

All used starting materials, reagents and preparation method were the same as those used in step 4 of Example 1, except that 3- methylthio- 6- phenyl- 1, 2, 4- triazine **(V- 1)** was replaced with 3- methylthio- 6- (3- methoxyphenyl)- 1, 2, 4- triazine **(V- 35)** . As a result, compound VI- 35 was obtained as yellowish solid.

Step 5: 3-hydrazino-6-(3-methoxyphenyl)-1,2,4-triazine **(VII-35)**

All used starting materials, reagents and preparation method were the same as those used in step 5 of Example 1, except that 3- methylsulfinyl- 6- phenyl- 1, 2, 4- triazine **(VI- 1)** was replaced with 3- methylsulfinyl- 6- (3- methoxyphenyl)- 1, 2, 4- triazine **(VI- 35)** . As a result, compound VII- 35 was obtained as yellowish solid.

$^1$H- NMR (300Hz, DMSO- $d_6$) δ: 8.86 (s, 2H), 7.57 (d, *J* = 7.65 Hz, 1H), 7.56 (s, 1H), 7.40 (t, *J*= 7.65, 7.8 Hz, 1H), 7.00 (dd, *J*= 2.4, 7.8 Hz, 1H), 4.43 (br, 2H), 3.81 (s, 3H) .

Step 6: 6-(3-methoxyphenyl)-3-[(7-methoxy-4-quinolyl)oxyacetylhydrazino]-1,2,4-triazine **(VIII-35)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- methoxyphenyl)-1, 2, 4- triazine **(VII- 35),** and 6- quinoline acetic acid was replaced with 7- methoxy- 4- quinolyloxy acetic acid. As a result, compound VIII- 35 was obtained as yellow solid.

$^1$H- NMR (300Hz, DMSO- $d_6$) δ: 10.54 (s, 1H), 9.84 (br, 1H), 9.02 (s, 1H), 8.71 (d, *J*= 5.4 Hz, 1H), 8.32 (d, *J*= 9.3 Hz, 1H), 7.63 (d, *J* = 6.9 Hz, 2H), 7.45 (t, *J* = 8.4, 7.5 Hz, 1H), 7.35 (d, *J* = 2.4 Hz, 1H), 7.22 (dd, *J* = 9.0, 2.4 Hz, 1H), 7.06 (dd, *J* = 7.5, 2.4 Hz, 1H), 6.95 (d, *J* = 5.4 Hz, 1H), 4.99 (s, 2H), 3.92 (s, 3H), 3.85 (s, 3H) .

Step 7:

3-[(7-methoxy-4-quinolyl)oxymethyl]-6-(3-methoxyphenyl)[1,2,4]triazolo[4,3-b][1,2,4]triazine **(II-11)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (3- methoxyphenyl)- 3- (6- quinolylacetylhydrazino)- 1, 2, 4- triazine **(VIII- 35) .** As a result, compound II- 11 was obtained as white solid.

$^1$H- NMR (300Hz, DMSO- $d_6$) δ: 9.47 (s, 1H), 8.73 (d, *J*= 4.5 Hz, 1H), 8.01 (d, *J*= 9.6 Hz, 1H), 7.72 (d, *J* = 7.8 Hz, 1H), 7.65 (s, 1H), 7.51 (t, *J*= 8.1, 7.8 Hz, 1H), 7.34 (s, 1H), 7.31 (d, *J* = 5.7 Hz, 1H), 7.20 (d, *J*= 6.3 Hz, 1H), 7.13 (d, *J*= 9 Hz, 1H), 6.00 (s, 2H), 3.89 (s, 3H), 3.76 (s, 3H) .

Example 36:

3-[(7-methoxy-4-quinolyl)oxymethyl]-6-(3-hydroxylphenyl)[1,2,4]triazolo[4,3-b][1,2,4]triazine **(II-12)**

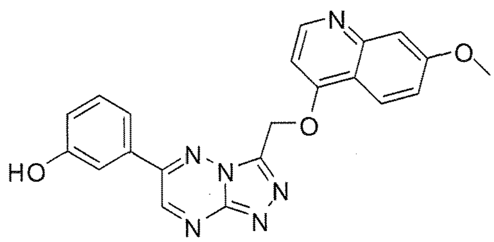

Step 1: 6-(3-hydroxylphenyl)-3-[(7-methoxy-4-quinolyl)oxyacetylhydrazino]-1,2,4-triazine **(VIII-36)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- hydroxylphenyl)- 1, 2, 4- triazine **(VII- 11),** and 6- quinoline acetic acid was replaced with 7- methoxy- 4- quinolyloxy acetic acid. As a result, compound VIII- 36 was obtained as white solid.

$^1$H- NMR (300Hz, DMSO- $d_6$) δ: 10.54 (s, 1H), 9.82 (br, 1H), 9.71 (s, 1H), 8.95 (s, 1H), 8.71 (d, *J* = 5.1 Hz, 1H), 8.33 (d, *J* = 9.3 Hz, 1H), 7.45 (d, *J* = 8.7 Hz, 2H), 7.32 (t, *J* = 7.2, 5.1 Hz, 2H), 7.22 (dd, *J* = 2.4, 9.3 Hz, 1H), 3.92 (s, 3H), 6.96 (d, *J* = 5.7 Hz, 1H), 6.78 (d, *J* = 6.6 Hz, 1H), 4.99 (s, 2H) .

Step 2:

3-[(7-methoxy-4-quinolyl)oxymethyl]-6-(3-hydroxylphenyl)[1,2,4]triazolo[4,3-b][1,2,4]triazine **(II-12)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (3- hydroxylphenyl)- 3- (6- quinolylacetylhydrazino)- 1, 2, 4- triazine **(VIII- 36) .** As a result, compound II- 12 was obtained as white solid.

$^1$H- NMR (300Hz, DMSO- $d_6$) δ: 10.54 (s, 1H), 9.82 (br, 1H), 9.71 (s, 1H), 8.95 (s, 1H), 8.71 (d, *J*=5.1 Hz, 1H), 8.33 (d, *J*=9.3 Hz, 1H), 7.45 (d, *J*=8.7 Hz, 2H), 7.32 (t, *J*=7.2, 5.1 Hz, 2H), 7.22 (dd, *J*=2.4, 9.3 Hz, 1H), 3.92 (s, 3H), 6.96 (d, *J*=5.7 Hz, 1H), 6.78 (d, *J*=6.6 Hz, 1H), 4.99 (s, 2H) .

Example 37:

3-[(7-methoxy-4-quinolyl)oxymethyl]-6-(3-nitrophenyl)[1,2,4]triazolo[4,3-b][1,2,4]triazine **(II-13)**

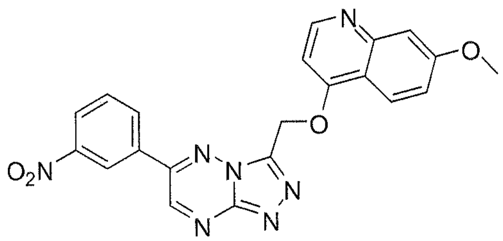

Step 1: 6-(3-nitrophenyl)-3-[(7-methoxy-4-quinolyl)oxyacetylhydrazino]-1,2,4-triazine **(VIII-37)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- nitrophenyl)- 1, 2, 4- triazine **(VII- 15),** and 6- quinoline acetic acid was replaced with 7- methoxy- 4- quinolyloxy acetic acid. As a result, compound VIII- 37 was obtained as white solid.

$^1$H- NMR (300Hz, DMSO- $d_6$) δ: 10.60 (s, 1H), 10.01 (br, 1H), 9.17 (s, 1H), 8.88 (s, 1H), 8.72 (d, *J*= 5.4 Hz, 1H), 8.53 (d, *J*= 9.3 Hz, 1H), 8.35- 8.30 (m, 2H), 7.84 (t, *J*= 7.8 Hz, 1H), 7.34 (d, *J* = 2.4 Hz, 1H), 7.24 (dd, *J* = 2.4, 9.3 Hz, 1H), 6.97 (d, *J* = 5.4, 1H), 5.00 (s, 2H), 3.91 (s, 3H) .

Step 2:

3-[(7-methoxy-4-quinolyl)oxymethyl]-6-(3-nitrophenyl)[1,2,4]triazolo[4,3-b][1,2,4]triazine **(II-13)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (3- nitrophenyl)- 3- (6- quinolylacetylhydrazino)- 1, 2, 4- triazine **(VIII- 37) .** As a result, compound II- 13 was obtained as white solid.

$^1$H- NMR (300Hz, DMSO- $d_6$) δ: 10.54 (s, 1H), 9.82 (br, 1H), 9.71 (s, 1H), 8.95 (s, 1H), 8.71 (d, *J*=5.1 Hz, 1H), 8.33 (d, *J*=9.3 Hz, 1H), 7.45 (d, *J*=8.7 Hz, 2H), 7.32 (t, *J*=7.2, 5.1 Hz, 2H), 7.22 (dd, *J*=2.4, 9.3 Hz, 1H), 3.92 (s, 3H), 6.96 (d, *J*=5.7 Hz, 1H), 6.78 (d, *J*=6.6 Hz, 1H), 4.99 (s, 2H) .

Example 38:

3-[(6,7-dimethoxy-4-quinolyl)oxymethyl]-6-(4-fluorophenyl)[1,2,4]triazolo[4,3-b][1,2,4]triazin e **(II-14)**

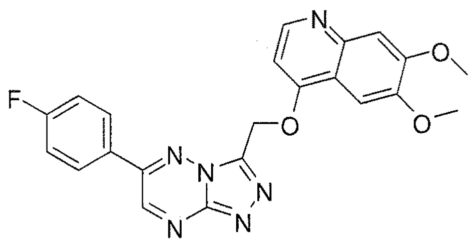

Step 1:

6-(4-fluorophenyl)-3-[(6,7-dimethoxy-4-quinolyl)oxyacetylhydrazino]-1,2,4-triazine **(VIII-38)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- fluorophenyl)- 1, 2, 4- triazine **(VII- 25),** and 6- quinoline acetic acid was replaced with 6, 7- dimethoxy- 4- quinolyloxy acetic acid. As a result, compound VIII- 38 was obtained as white solid.

$^1$H- NMR (300Hz, DMSO- $d_6$) δ: 10.55 (s, 1H), 9.83 (br, 1H), 8.99 (s, 1H), 8.57 (d, *J*= 5.1 Hz, 1H), 8.12 (m, 2H), 7.55 (s, 1H), 7.32- 7.39 (m, 3H), 6.94 (d, *J*= 5.1 Hz, 1H), 4.98 (s, 1H), 3.91 (s, 1H) .

Step 2:

3-[(6,7-dimethoxy-4-quinolyl)oxymethyl]-6-(4-fluorophenyl)[1,2,4]triazoio[4,3-b][1,2,4]triazin e **(II-14)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- fluorophenyl)- 3- [(6, 7- dimethoxy- 4- quinolyl) oxyacetylhydrazino]- 1, 2, 4- triazine **(VIII- 38) .** As a result, compound II- 14 was obtained as white solid.

$^1$H- NMR (300Hz, DMSO- $d_6$) $\delta$: 9.44 (s, 1H), 8.58 (d, *J* = 5.4 Hz, 1H), 8.23 (m, 2H), 7.39- 7.46 (m, 2H), 7.30 (m, 3H), 5.97 (s, 2H), 3.88 (s, 3H), 3.68 (s, 3H) .

Example 39:

3-[(6,7-dimethoxy-4-quinolyl)oxymethyl]-6-(4-chlorophenyl)[1,2,4]triazolo[4,3-b][1,2,4]triazin e **(II-15)**

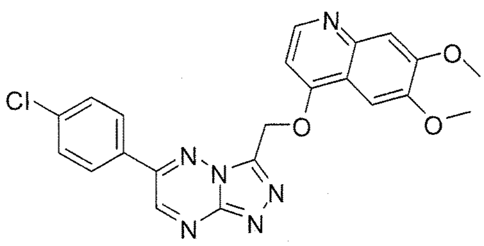

Step 1:

6-(4-chlorophenyl)-3-[(6,7-dimethoxy-4-quinolyl)oxyacetylhydrazino]-1,2,4-triazine **(VIII-39)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- chlorophenyl)- 1, 2, 4- triazine **(VII- 16),** and 6- quinoline acetic acid was replaced with 6, 7- dimethoxy- 4- quinolyloxy acetic acid. As a result, compound VIII- 39 was obtained as white solid.

$^1$H- NMR (300Hz, DMSO- $d_6$) $\delta$: 10.59 (s, 1H), 9.88 (br, 1H), 9.03 (s, 1H), 8.60 (d, *J*= 5.1 Hz, 1H), 8.10 (d, *J*= 8.4 Hz, 2H), 7.63 (d, *J*= 8.4 Hz, 2H), 7.57 (s, 1H), 7.34 (s, 1H), 6.97 (d, *J* = 5.1 Hz, 1H), 5.01 (s, 2H), 3.92 (s, 3H), 3.93 (s, 3H) .

Step 2:

3-[(6,7-dimethoxy-4-quinolyl)oxymethyl]-6-(4-chlorophenyl)[1,2,4]triazolo[4,3-b][1,2,4]triazin e **(II-15)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- chlorophenyl)- 3- [(6, 7- dimethoxy- 4- quinolyl) oxyacetylhydrazino]- 1, 2, 4- triazine **(VIII- 39) .** As a result, compound II- 15 was obtained as white solid.

$^1$H- NMR (300Hz, DMSO- $d_6$) $\delta$: 9.46 (s, 1H), 8.60 (d, J= 5.1 Hz, 1H), 8.19 (d, J= 8.7 Hz, 2H), 7.69 (d, J = 8.7 Hz, 2H), 7.33 (s, 1H), 7.30 (s, 1H), 7.29 (d, *J* =5.1 Hz, 1H), 6.01 (s, 2H), 3.90 (s, 3H), 3.70 (s, 3H) .

Example 40:

3-[(6,7-dimethoxy-4-quinolyl)oxymethyl]-6-(4-bromophenyl)[1,2,4]triazolo[4,3-b][1,2,4]triazin e **(II-16)**

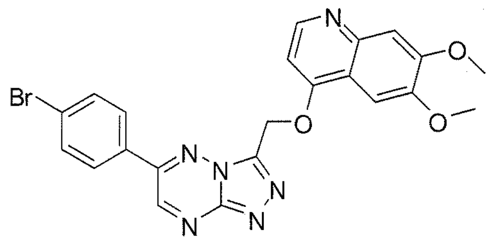

Step 1:

6-(4-bromophenyl)-3-[(6,7-dimethoxy-4-quinolyl)oxyacetylhydrazino]-1,2,4-triazine **(VIII-40)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- bromophenyl)- 1, 2, 4- triazine **(VII- 10),** and 6- quinoline acetic acid was replaced with 6, 7- dimethoxy- 4- quinolyloxy acetic acid. As a result, compound VIII- 40 was obtained as white solid.

$^1$H- NMR (300Hz, DMSO- $d_6$) δ: 10.59 (s, 1H), 9.91 (br, 1H), 9.03 (s, 1H), 7.58 (d, J= 5.1 Hz, 1H), 8.02 (d, J= 8.4 Hz, 2H), 7.72 (d, *J*= 8.4 Hz, 2H), 7.56 (s, 1H), 7.34 (s, 1H), 6.95 (d, J = 5.1 Hz, 1H), 5.00 (s, 2H), 3.93 (s, 3H), 3.92 (s, 3H).

Step 2:

3-[(6,7-dimethoxy-4-quinolyl)oxymethyl]-6-(4-bromophenyl)[1,2,4]triazolo[4,3-b][1,2,4]triazin e **(II-16)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- bromo, phenyl)- 3- [(6, 7- dimethoxy- 4- quinolyl) oxyacetylhydrazino]- 1, 2, 4- triazine **(VIII- 40) .** As a result, compound II- 16 was obtained as white solid.

$^1$H- NMR (300Hz, DMSO- $d_6$) δ: 9.45 (s, 1H), 3.70 (s, 3H), 8.59 (d, *J*= 4.5 Hz, 1H), 8.10 (d, *J* = 8.4 Hz, 2H), 7.81 (d, *J* = 8.4 Hz, 2H), 7.30 (t, *J* = 3.9, 7.5 Hz, 3H), 6.00 (s, 2H), 3.90 (s, 3H) .

Example 41:

3-[(6,7-dimethoxy-4-quinolyl)oxymethyl]-6-phenyl[1,2,4]triazolo[4,3-b][1,2,4]triazine (II-17)

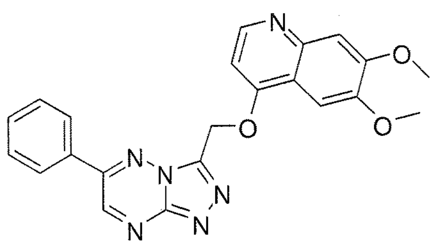

Step 1: 6-phenyl-3-[(6,7-dimethoxy-4-quinolyl)oxyacetylhydrazino]-1,2,4-triazine **(VIII-41)**

All used starting materials, reagents and preparation method were the same as those used in step 6 of Example 1, except that 6- quinoline acetic acid was replaced with 6, 7- dimethoxy- 4- quinolyloxy acetic acid. As a result, compound VIII- 41 was obtained as white solid.

$^1$H- NMR (300Hz, DMSO- $d_6$) δ: 10.55 (s, 1H), 9.83 (br, 1H), 8.99 (s, 1H), 8.57 (d, *J*= 5.7 Hz, 1H), 8.05 (m, 2H), 7.45- 7.54 (m, 4H), 7.32 (s, 1H), 6.94 (d, *J*= 4.5 Hz, 1H), 4.98 (s, 2H), 3.96 (s, 6H) .

Step 2:

3-[(6,7-dimethoxy-4-quinolyl)oxymethyl]-6-phenyl[1,2,4]triazolo[4,3-b][1,2,4]triazine **(II-17)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- phenyl- 3- [(6, 7- dimethoxy- 4- quinolyl) oxyacetylhydrazino]- 1, 2, 4- triazine **(VIII- 41) .** As a result, compound II- 17 was obtained as white solid.

$^1$H- NMR (300Hz, DM: SO- $d_6$) δ: 9.44 (s, 1H), 8.58 (d, *J*= 5.4 Hz, 1H), 8.13 (d, *J*= 7.2 Hz, 2H), 7.59 (m, 3H), 7.31 (m, 3H), 5.99 (s, 2H), 3.88 (s, 3H), 3.67 (s, 3H) .

Example 42:

3-[(6,7-dimethoxy-4-quinolyl)oxymethyl]-6-(4-methoxyformylphenyl)[1,2,4]triazolo[4,3-b][1,2 ,4]triazine **(II-18)**

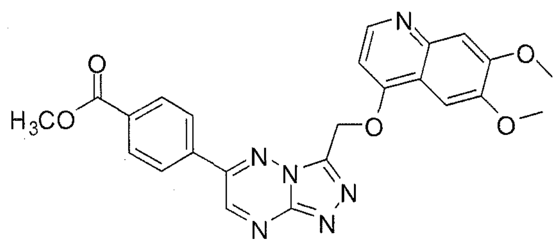

Step 1:

6-(4-methoxyformylphenyl)-3-[(6,7-dimethoxy-4-quinolyl)oxyacetylhydrazino]-1,2,4-triazine **(VIII-42)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- methoxyformylphenyl)- 1, 2, 4- triazine **(VII- 29),** and 6- quinoline acetic acid was replaced with 6, 7- dimethoxy- 4- quinolyl oxyacetic acid. As a result, compound VIII- 42 was obtained as white solid.

$^1$H- NMR (300Hz, DMSO- $d_6$) δ: 10.64 (br, 1H), 9.10 (s, 1H), 8.61 (d, *J*= 5.7 Hz, 1H), 8.49 (d, *J*= 8.4 Hz, 2H), 8.12 (d, *J*= 8.4 Hz, 2H), 7.58 (s, 1H), 7.35 (s, 1H), 6.99 (d, *J*= 5.7 Hz, 1H), 5.03 (s, 2H), 3.94 (s, 3H), 3.93 (s, 3H), 3.90 (s, 3H) .

Step 2:

3-[(6,7-dimethoxy-4-quinolyl)oxymethyl]-6-(4-methoxyformylphenyl)[1,2,4]triazolo[4,3-b][1,2 ,4]triazine **(II-18)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- methoxyformylphenyl)- 3- [(6, 7- dimethoxy- 4- quinolyl) oxyacetylhydrazino]- 1, 2, 4- triazine **(VIII- 42) .** As a result, compound II- 18 was obtained as white solid.

$^1$H- NMR (300Hz, DMSO- $d_6$) δ: 9.50 (s, 1H), 8.62 (d, *J*= 5.1 Hz, 1H), 8.30 (d, *J*= 8.1 Hz, 2H), 8.13 (d, *J*= 8.1 Hz, 2H), 7.33 (s, 1H), 7.32 (s, 1H), 7.31 (s, 1H), 6.02 (s, 2H), 3.90 (s, 3H), 3.89 (s, 3H), 3.68 (s, 3H) .

Example 43:

3-[(6,7-dimethoxy-4-quinolyl)oxymethyl]-6-(4-benzyloxyphenyl)[1,2,4]triazolo[4,3-b][1,2,4]tri azine (II-19)

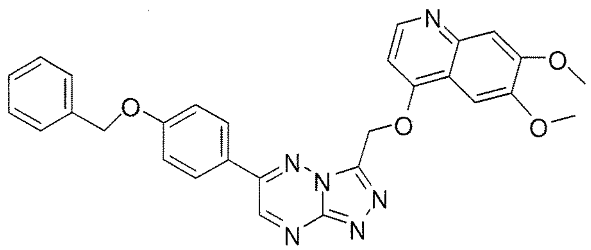

Step 1:

6-(4-benzyloxyphenyl)-3-[(6,7-dimethoxy-4-quinolyl)oxyacetylhydrazino]-1,2,4-triazine (VIII-43)

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- benzyloxyphenyl)- 1, 2, 4- triazine **(VII- 2),** and 6- quinoline acetic acid was replaced with 6, 7- dimethoxy- 4- quinolyloxy acetic acid. As a result, compound VIII- 43 was obtained as white solid.

$^1$H- NMR (300Hz, DMSO- $d_6$) $\delta$: 10.54 (s, 1H), 8.73 (br, 1H), 8.97 (s, 1H), 8.58 (d, *J* = 5.1 Hz, 1H), 8.00 (d, *J* = 8.7 Hz, 2H), 7.56 (s, 1H), 7.34- 7.50 (m, 6H), 7.17 (d, *J* = 8.7 Hz, 2H), 6.95 (d, *J*= 5.1 Hz, 1H), 5.20 (s, 2H), 4.39 (s, 2H), 3.93 (d, *J*= 2.4 Hz, 6H) .

Step 2:

3-[(6,7-dimethoxy-4-quinolyl)oxymethyl]-6-(4-benzyloxyphenyl)[1,2,4]triazolo[4,3-b][1,2,4]tri azine **(II-19)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- benzyloxyphenyl)- 3- [(6, 7- dimethoxy- 4- quinolyl) oxyacetylhydrazino]- 1, 2, 4- triazine **(VIII- 43) .** As a result, compound II- 19 was obtained as white solid.

$^1$H- NMR (300Hz, DMSO- $d_6$) $\delta$: 9.42 (s, 1H), 8.57 (d, *J*= 4.8 Hz, 1H), 8.11 (d, *J*= 8.7 Hz, 2H), 7.27- 7.47 (m, 8H), 7.19 (d, *J*= 8.7 Hz, 2H), 5.96 (s, 2H), 5.21 (s, 2H), 3.88 (s, 3H), 3.68 (s, 3H) .

Example 44:

3-[(7-methoxy-4-quinolyl)oxymethyl]-6-(3,4-dichlorophenyl)[1,2,4]triazolo[4,3-b][1,2,4]triazin e **(II-20)**

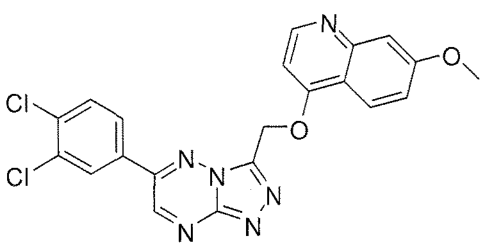

Step 1:

6-(2,4-dichlorophenyl)-3-[(7-methoxy-4-quinolyl)oxyacetylhydrazino]-1,2,4-triazine **(VIII-44)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3, 4- dichlorophenyl)- 1, 2, 4- triazine **(VII- 4),** and 6- quinoline acetic acid was replaced with 7- methoxy- 4- quinolyloxy acetic acid. As a result, compound VIII- 44 was obtained as white solid.

Step 2:

3-[(7-methoxy-4-quinolyl)oxymethyl]-6-(3,4-dichlorophenyl)[1,2,4]triazolo[4,3-b][1,2,4]triazin e **(II-20)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (2, 4- dichlorophenyl)- 3- [(7- methoxy- 4- quinolyl) oxyacetylhydrazino]- 1, 2, 4- triazine **(VIII- 44) .** As a result, compound II- 20 was obtained as white solid.

$^1$H- NMR (300Hz, DMSO- $d_6$) δ: 9.47 (s, 1H), 8.72 (d, *J*= 5.4 Hz, 1H), 8.38 (d, *J*= 2.1 Hz, 1H), 8.13 (dd, *J* = 2.1, 8.7 Hz, 1H), 7.99 (d, *J* = 9.0 Hz, 1H), 7.88 (d, *J* = 8.7 Hz, 1H), 7.32 (d, *J* = 2.1 Hz, 1H), 7.29 (d, *J*= 5.4 Hz, 1H), 7.13 (dd, *J*= 2.1, 9 Hz, 1H), 5.99 (s, 2H), 3.86 (s, 3H) .

Example 45:

3-(4-quinolyloxymethyl)-6-(4-fluorophenyl)[1,2,4]triazolo[4,3-b][1,2,4]triazine **(II-21)**

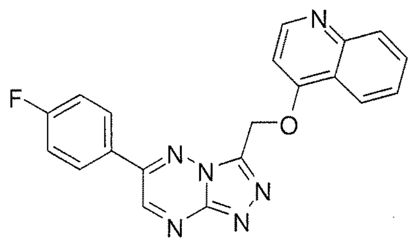

Step 1: 6-(4-fluorophenyl)-3-(4-quinolyloxyacetylhydrazino)-1,2,4-triazine **(VIII-45)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- fluorophenyl)- 1, 2, 4- triazine **(VII- 25),** and 6- quinoline acetic acid was replaced with 4- quinolyloxy acetic acid. As a result, compound VIII- 45 was obtained as white solid.

$^1$H- NMR (300Hz, DMSO- $d_6$) δ: 10.55 (s, 1H), 9.85 (br, 1H), 9.00 (s, 1H), 8.78 (d, *J*= 5.1 Hz, 1H), 8.42 (d, *J* = 8.7 Hz, 1H), 8.12 (m, 2H), 7.97 (d, *J* = 8.7 Hz, 1H), 7.75 (m, 1H), 7.60 (m, 1H), 7.39 (m, 2H), 7.08 (d, *J*= 5.1 Hz, 1H), 5.01 (s, 1H) .

Step 2: 3-(4-quinolyloxymethyl)-6-(4-fluorophenyl)[1,2,4]triazolo[4,3-h][1,2,4]triazine **(II-21)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- fluorophenyl)- 3- (4- quinolyloxy acetylhydrazino)- 1, 2, 4- triazine **(VIII- 45) .** As a result, compound II- 21 was obtained as white solid.

$^1$H- NMR (300Hz, DMSO- $d_6$) δ: 9.44 (s, 1H), 8.81 (d, *J*= 5.1 Hz, 1H), 8.23 (m, 2H), 8.10 (d, *J* = 9.3 Hz, 1H), 7.94 (m, 1H), 7.72 (m, 1H), 7.43- 7.49 (m, 4H), 6.03 (s, 2H) .

Example 46: 3-(4-quinolyloxymethyl)-6-phenyl[1,2,4]triazolo[4,3-b][1,2,4]triazine **(II-22)**

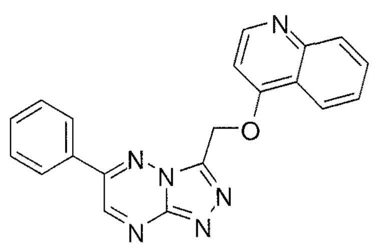

Step 1: 6-phenyl-3-(4-quinolyloxyacetylhydrazino)-1,2,4-triazine **(VIII-46)**

All used starting materials, reagents and preparation method were the same as those used in step 6 of Example 1, except that 6-quinoline acetic acid was replaced with 4-quinolyloxy acetic acid. As a result, compound VIII-46 was obtained as white solid.

$^1$H- NMR (300Hz, DMSO- $d_6$) δ: 10.55 (s, 1H), 9.84 (br, 1H), 9.01 (s, 1H), 8.78 (d, *J* = 5.1 Hz, 1H), 8.42 (d, *J* = 8.1 Hz, 2H), 8.05 (d, *J* = 6.6 Hz, 2H), 7.97 (d, *J* = 9.0 Hz, 1H), 7.77 (m, 1H), 7.47- 7.60 (m, 4H), 7.08 (d, *J*= 5.1 Hz, 1H), 5.01 (s, 2H) .

Step 2: 3-(4-quinolyloxymethyl)-6-phenyl[1,2,4]triazolo[4,3-b][1,2,4]triazine **(II-22)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- phenyl- 3- (4-quinolyloxyacetylhydrazino)- 1, 2, 4- triazine (**VIII- 46) .** As a result, compound II- 21 was obtained as white solid.

$^1$H- NMR (300Hz, DMSO- $d_6$) δ: 9.45 (s, 1H), 8.81 (d, *J*= 5.4 Hz, 1H), 8.09- 8.13 (m, 3H), 7.97 (d, *J*= 9.0 Hz, 1H), 7.72 (m, 1H), 7.42- 7.58 (m, 5H), 6.01 (s, 2H) .

Example 47:

3-(4-quinolyloxymethyl)-6-(4-methoxyphenyl)[1,2,4]triazolo[4,3-b][1,2,4]triazine **(II-23)**

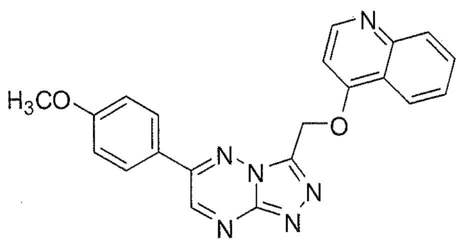

Step 1: 6-(4-methoxyphenyl)-3-(4-quinolyloxyacetylhydrazino)-1,2,4-triazine **(VIII-47)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- methoxyphenyl)- 1, 2, 4- triazine **(VII- 33),** and 6- quinoline acetic acid was replaced with 4- quinolyloxy acetic acid. As a result, compound VIII- 47 was obtained as white solid.

Step 2: 3-(4-quinolyloxymethyl)-6-(4-methoxyphenyl)[1,2,4]triazolo[4,3-b][1,2,4]triazine **(II-23)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- methoxyphenyl)- 3- (4- quinolyloxyacetylhydrazino)- 1, 2, 4- triazine **(VIII- 47) .** As a result, compound II- 23 was obtained as white solid.

$^1$H- NMR (300Hz, DMSO- $d_6$) δ: 9.45 (s, 1H), 8.82 (d, *J* = 5.1 Hz, 1H), 8.12 (t, J = 7.2, 9.0 Hz, 3H), 7.98 (d, *J* = 8.4 Hz, 1H), 7.74 (t, *J* = 7.2, 7.5 Hz, 1H), 7.52 (t, *J* = 7.5, 8.4 Hz, 1H), 7.45 (d, *J*= 5.1 Hz, 1H), 7.13 (d, *J*= 9.0 Hz, 2H), 6.01 (s, 2H), 3.85 (s, 3H) .

Example 48:

3-(4-quinolyloxymethyl)-6-(4-hydroxylphenyl)[1,2,4]triazolo[4,3-b][1,2,4]triazine **(II-24)**

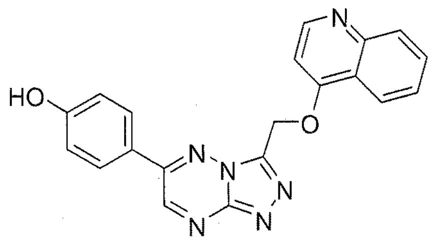

Step 1: 6-(4-hydroxylphenyl)-3-(4-quinolyloxyacetylhydrazino)-1,2,4-triazine **(VIII-48)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- hydroxylphenyl) 1, 2, 4- triazine **(VII- 34),** and 6- quinoline acetic acid was replaced with 4- quinolyloxy acetic acid. As a result, compound VIII- 48 was obtained as white solid.

Step 2: 3-(4-quinolyloxymethyl)-6-(4-hydroxylphenyl)[1,2,4]triazolo[4,3-b][1,2,4]triazine **(II-24)**

All used starting materials, reagents and preparation method were the same as those used in step 7 of Example 1, except that 6- phenyl- 3- (6- quinolylacetylhydrazino)- 1, 2, 4- triazine (**VIII- 1**) was replaced with 6- (4- hydroxylphenyl)- 3- (4- quinolyloxy acetylhydrazino)- 1, 2, 4- triazine **(VIII- 48) .** As a result, compound II- 24 was obtained as white solid.
$^1$H- NMR (300Hz, DMSO- $d_6$) δ: 10.33 (s, 1H), 9.40 (s, 1H), 8.82 (d, *J*= 5.4 Hz, 1H), 8.11 (d, *J* = 7.5 Hz, 1H), 8.02 (d, *J* = 8.7 Hz, 2H), 7.98 (d, *J* = 8.1 Hz, 1H), 7.74 (t, *J* = 6.9, 8.4 Hz, 1H), 7.52 (t, *J* = 6.9, 8.1 Hz, 1H), 7.45 (d, *J* = 5.4 Hz, 1H), 6.93 (d, *J* = 8.7 Hz, 2H), 5.99 (s, 2H) .
Example 49:

3-(4-quinolyloxymethyl)-6-(4-methoxyformylphenyl)[1,2,4]triazolo[4,3-b][1,2,4]triazine **(II-25)**

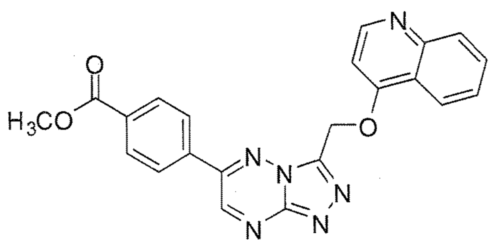

Step 1: 6-(4-methoxyformylphenyl)-3-(4-quinolyloxyacetylhydrazino)-1,2,4-triazine **(VIII-49)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (4- methoxyformylphenyl)- 1, 2, 4- triazine **(VII- 29),** and 6- quinoline acetic acid was replaced with 4- quinolyloxy acetic acid. As a result, compound VIII- 49 was obtained as white solid.
$^1$H- NMR (300Hz, DMSO- $d_6$) δ: 10.62 (s, 1H), 10.01 (br, 1H), 9.11 (s, 1H), 8.81 (d, *J*= 5.1 Hz, 1H), 8.44 (d, *J* = 7.5 Hz, 1H), 8.25 (d, *J* = 8.7 Hz, 2H), 8.12 (d, *J* = 8.7 Hz, 2H), 7.99 (d, *J* = 8.7 Hz, 1H), 7.77 (t, *J*= 7.5 Hz, 1H), 7.60 (t, *J*= 8.7 Hz, 1H), 7.10 (d, *J*= 5.1 Hz, 1H), 5.05 (s, 2H), 3.89 (s, 3H) .

Step 2:

3-(4-quinolyloxymethyl)-6-(4-methoxyformylphenyl)[1,2,4]triazolo[4,3-b][1,2,4]triazine **(II-25)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (4- methoxyformylphenyl)- 3- (4- quinolyloxyacetylhydrazino)- 1, 2, 4- triazine **(VIII- 49) .** As a result, compound II- 25 was obtained as white solid.
$^1$H- NMR (300Hz, DMSO- $d_6$) δ: 9.50 (s, 1H), 8.83 (d, *J*= 5.1 Hz, 1H), 8.30 (d, *J*= 8.4 Hz, 2H), 8.14 (d, *J* = 8.4

Hz, 1H), 7.98 (d, *J* = 8.4 Hz, 1H), 7.74 (t, *J* = 8.4 Hz, 1H), 7.51 (t, *J* = 8.1 Hz, 1H), 7.46 (d, *J* = 5.1, 1H), 6.04 (s, 2H), 3.90 (s, 3H) .

Example 50:

3-(4-quinolyloxymethyl)-6-(3-methoxyphenyl)[1,2,4]triazolo[4,3-b][1,2,4]triazine **(II-26)**

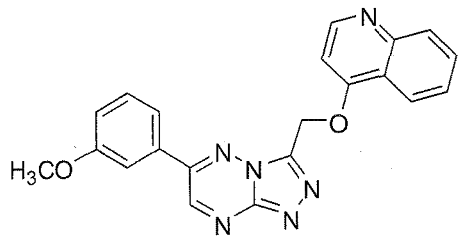

Step 1: 6-(3-methoxyphenyl)-3-(4-quinolyloxyacetylhydrazino)-1,2,4-triazine **(VIII-50)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- methoxyphenyl)- 1, 2, 4- triazine **(VII- 35),** and 6- quinoline acetic acid was replaced with 4- quinolyloxy acetic acid. As a result, compound VIII- 50 was obtained as white solid.

Step 2: 3-(4-quinolyloxymethyl)-6-(4-methoxyphenyl)[1,2,4]triazolo[4,3-b][1,2,4]triazine **(II-26)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (3- methoxyphenyl)- 3- (4- quinolyloxyacetylhydrazino)- 1, 2, 4- triazine **(VIII- 50) .** As a result, compound II- 26 was obtained as white solid.

$^1H$- NMR (300Hz, DMSO- $d_6$) δ: 9.48 (s, 1H), 8.82 (d, *J*= 5.4 Hz, 1H), 8.13 (d, *J*= 8.4 Hz, 1H), 7.97 (d, *J* = 8.7 Hz, 1H), 7.74 (t, *J* = 6.9, 8.4 Hz, 2H), 7.66 (t, *J* = 1.8, 2.1 Hz, 1H), 7.44- 7.54 (m, 3H), 7.19 (dd, *J*= 2.7, 8.1 Hz, 1H), 6.04 (s, 2H), 3.75 (s, 3H) .

Example 51:

3-(4-quinolyloxymethyl)-6-(3-hydroxylphenyl)[1,2,4]triazolo[4,3-b][1,2,4]triazine **(II-27)**

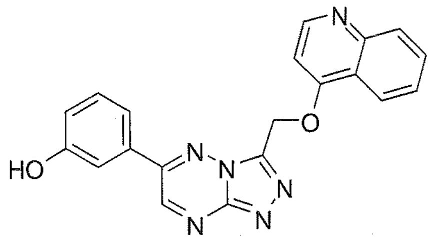

Step 1: 6-(3-hydroxylphenyl)-3-(4-quinolyloxyacetylhydrazino)-1,2,4-triazine **(VIII-51)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- hydroxylphenyl)- 1, 2, 4- triazine **(VII- 11),** and 6- quinoline acetic acid was replaced with 4- quinolyloxy acetic acid. As a result, compound VIII- 51 was obtained as white solid.

Step 2: 3-(4-quinolyloxymethyl)-6-(3-hydroxylphenyl)[1,2,4]triazolo[4,3-b][1,2,4]triazine **(II-27)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (3- hydroxylphenyl)- 3- (4- quinolyloxyacetylhydrazino)- 1, 2, 4- triazine **(VIII- 51) .** As a result, compound II- 27 was obtained as white solid.
$^1$H- NMR (300Hz, DMSO- $d_6$) δ: 9.93 (s, 1H), 9.40 (s, 1H), 8.83 (d, *J*=5.1 Hz, 1H), 8.12 (d, *J*=8.1 Hz, 1H), 7.97 (d, *J*=8.4 Hz, 1H), 7.74 (t, *J*=6.9, 8.4 Hz, 1H), 7.36- 7.62 (m, 5H), 7.02 (q, *J*=2.4, 8.4 Hz, 1H), 6.01 (s, 2H) .

Example 52:

3-(4-quinolyloxymethyl)-6-(3-nitrophenyl)[1,2,4]triazolo[4,3-b][1,2,4]triazine **(II-28)**

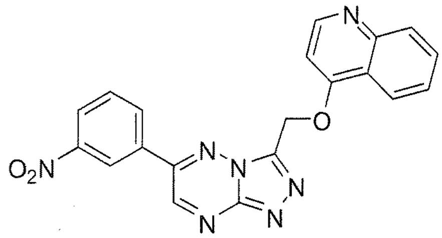

Step 1: 6-(3-nitrophenyl)-3-(4-quinolyloxyacetylhydrazino)-1,2,4-triazine **(VIII-52)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- (3- nitrophenyl)- 1, 2, 4- triazine **(VII- 15),** and 6- quinoline acetic acid was replaced with 4- quinolyloxy acetic acid. As a result, compound VIII- 52 was obtained as white solid.
$^1$H- NMR (300Hz, DMSO- $d_6$) δ: 10.63 (s, 1H), 10.07 (br, 1H), 9.18 (s, 1H), 8.89 (s, 1H), 8.81 (d, *J* = 5.1 Hz, 1H), 8.53 (d, *J* = 5.1 Hz, 1H), 8.44 (d, *J* = 8.1 Hz, 1H), 8.34 (dd, *J* = 1.8, 7.8 Hz, 1H), 7.99 (d, *J* = 7.8 Hz, 1H), 7.87 (t, *J* = 8.1 Hz, 1H), 7.77 (ddd, *J* = 1.8, 7.8, 7.8 Hz, 1H), 7.60 (m, 1H), 7.11 (d, *J* = 5.1 Hz, 1H), 5.05 (s, 2H) .

Step 2: 3-(4-quinolyloxymethyl)-6-(3-nitrophenyl)[1,2,4]triazolo[4,3-b][1,2,4]triazine **(II-28)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- (3- nitrophenyl)- 3- (4- quinolyloxyacetylhydrazino)- 1, 2, 4- triazine **(VIII- 52) .** As a result, compound II- 28 was obtained as white solid.
$^1$H- NMR (300Hz, DMSO- $d_6$) δ: 9.56 (s, 1H), 8.91 (m, 1H), 8.83 (d, *J*= 5.1 Hz, 1H), 8.58 (d, *J* = 7.8 Hz, 1H), 8.45 (m, 1H), 8.15 (d, *J* = 8.1 Hz, 1H), 7.98 (d, *J* = 8.1 Hz, 1H), 7.90 (t, *J* = 8.1 Hz, 1H), 7.73 (m, 1H), 7.51 (m, 1H), 7.47 (d, *J*= 5.1 Hz, 1H), 6.08 (s, 2H) .

Example 53:

3-[(7-methoxy-4-quinolyl)oxymethyl]-6-[(1-methyl)-4-pyrazolyl][1,2,4]triazolo[4,3-b][1,2,4]tri azine **(II-29)**

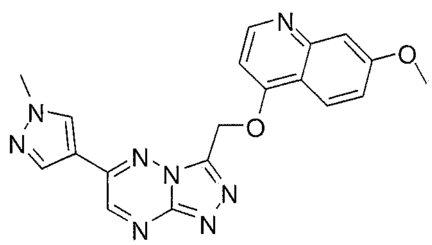

Step 1:

6-[(1-methyl)-4-pyrazolyl]-3-[(7-methoxy-4-quinolyl)oxyacetylhydrazino]-1,2,4-triazine **(VIII-53)**

All used starting materials, reagents and preparation method were the same as those used in step 6 of Example 1, except that 3- hydrazino- 6- phenyl- 1, 2, 4- triazine **(VII- 1)** was replaced with 3- hydrazino- 6- [(1- methyl)- 4- pyrazolyl]- 1, 2, 4- triazine **(VII- 5),** and 6- quinoline acetic acid was replaced with 7- methoxy- 4- quinolyloxy acetic acid. As a result, compound VIII- 53 was obtained as white solid.

$^1$H- NMR (300Hz, DMSO- $d_6$) $\delta$: 10.45 (s, 1H), 9.59 (s, 1H), 8.78 (s, 1H), 8.70 (d, *J* = 5.4 Hz, 1H), 8.29- 8.35 (m, 2H), 8.04 (s, 1H), 7.34 (d, *J* = 2.4 Hz, 1H), 7.23 (dd, *J* = 2.4, 9.6 Hz, 1H), 6.95 (d, *J*= 5.4 Hz, 1H), 4.96 (s, 2H), 3.91 (s, 6H) .

Step 2:

3-[(7-methoxy-4-quinolyl)oxymethyl]-6-[(1-methyl)-4-pyrazolyl][1,2,4]triazolo[4,3-b][1,2,4]tri azine **(II-29)**

All used starting materials, reagents and preparation method were the same as those used in step 7 of Example 1, except that 6- phenyl- 3- (6- quinolylacetylhydrazino)- 1, 2, 4- triazine **(VIII- 1)** was replaced with 6- [(1- methyl)- 4- pyrazolyl]- 3- [(7- methoxy- 4- quinolyl) oxyacetylhydrazino]- 1, 2, 4- triazine **(VIII- 53) .** As a result, compound II- 29 was obtained as white solid.

$^1$H- NMR (300Hz, DMSO- $d_6$) $\delta$: 9.20 (s, 1H), 8.74 (d, *J*= 3.6 Hz, 1H), 8.59 (s, 1H), 8.19 (s, 1H), 7.99 (d, *J*= 9.0 Hz, 1H), 7.27- 7.33 (m, 2H), 7.15 (dd, *J*= 1.8, 9.0 Hz, 1H), 5.89 (s, 2H), 3.92 (s, 3H), 3.88 (s, 3H) .

Example 54: the effects of the compounds on c-Met kinase activity on molecular level

1. Procedure:

Poly (Glu, Tyr)$_{4:1}$ as an enzyme substrate was diluted by potassium ion- free PBS (10 mM, sodium phosphate buffer, 150 mM NaCl, pH7.2- 7.4) into a solution of 20$\mu$g/ml. An ELISA plate was coated by the solution (125 $\mu$l/ well) and kept at 37 °C to be incubated for 12- 16 h. After the liquid in wells was removed, the plate was washed three time by T- PBS (PBS solution containing 0.1% Tween- 20) at 200 $\mu$l/ well, 5 min for each time. The ELISA plate was then dried in an oven at 37°C for 1- 2 h.

Each well was added with 50$\mu$L of ATP solution diluted by reaction buffer (50 mM HEPES pH 7.4, 50 mM $MgCl_2$, 0.5 mM $MnCl_2$, 0.2 mM $Na_3VO_4$, 1 mM DTT) at a final concentration of 5$\mu$M. Compound diluted by 1% DMSO into a suitable concentration was added at 10 $\mu$l/well and then c-Met tyrosine kinase protein diluted by 40 $\mu$l of reaction buffer was added. The plate was placed on a shaking table (100rpm) at 37°C and incubated for 1 h, and then washed three times by T-PBS. For each assay, triplicate wells without enzyme were used as blank control and wells having the corresponding DMSO concentration without compound were used as negative control. Primary antibody PY99 (p-Tyr (PY99), Cell Sinaling Technology, 1:1000 diluted in 5 mg/mL BSA T-PBS) was added at 100 $\mu$l/well. The plate was then placed on a shaking table at 37 °C and incubated for 0.5 h, and washed by T-PBS. Secondary antibody horseradish peroxidase-conjugated goat anti-mouse IgG (1:2000 diluted in 5 mg/mL BSA T-PBS) was added at 100 $\mu$l/well. The plate was then placed on a shaking table at 37 °C for 0.5 h and washed three times by T-PBS. 2 mg/mL of OPD developer (diluted by a 0.1 M citric acid-sodium citrate buffer (pH=5.4) containing 0.03% of $H_2O_2$) was added at 100 $\mu$l/well, and the plate was kept in dark at 25 °C for 1 - 10 min. OPD was ultrasonically dissolved and the developer solution was prepared just before use. 2 M of $H_2SO_4$ was added at 50 $\mu$l/well to stop the reaction. The plate was read using a wavelength regulated multi-well spectrophotometer (SPECTRA MAX 190) at 490 nm.

The inhibition rate was calculated according to the following equation:

$$\text{Inhibition rate}(\%)=(1-(OD_{compound}-OD_{blank})/(OD_{negative}-OD_{blank}))x100\%$$

$IC_{50}$ value was calculated thtough a four- parameter fit according to the inhibition curve.

2. Result:

As seen in enzyme's acitivity assay on molelular level, [1, 2, 4] triazolo [4, 3- b] [1, 2, 4] triazine compounds of the present application exhibited a strongly inhibitory effect on c- Met tyrosine kinase at nanomole level. Among them, the compounds, in which substituted or unsubstituted quinolyl was connected at 3 position of [1, 2, 4] triazolo [4, 3- b]

[1, 2, 4] triazine by methylene, difluoromethylene or methyleneoxy, showed strongest acitivty. Some of them had an $IC_{50}$ value of less than 1 nM, which was superior to positive control compound SU11274. Therefore, compounds of the present application were highly potent c- Met inhibitors.

Table 2: $IC_{50}$ of compounds in examples of the present application on c-Met tyrosine kinase activity

| Compound in Example | $IC_{50}$(nM) |
|---|---|
| SU11274 [a] | 7.9 |
| JNJ-38877605 [b] | 0.95 |
| I-1 | 0.49 |
| I-2 | |
| I-3 | 2.3 |
| I-4 | 1.2 |
| I-5 | 0.46 |
| I-6 | 4.1 |
| I-7 | 3.2 |
| I-8 | 2.3 |
| I-9 | 222.8 |
| I-10 | 129.2 |
| I-11 | 126.6 |
| I-12 | 1.2 |
| I-13 | 5.3 |

| | |
|---|---|
| I-14 | 10.5 |
| I-15 | 2.0 |
| I-16 | 2.5 |
| I-17 | |
| I-18 | |
| I-19 | 0.50 |
| I-20 | 7.9 |
| I-21 | 81.6 |
| I-22 | 2.8 |
| I-23 | 1.4 |
| I-24 | 2.9 |
| II-1 | 0.053 |
| II-2 | 0.37 |
| II-3 | 0.23 |
| II-4 | 20.1 |
| II-5 | 0.78 |
| II-6 | 0.56 |
| II-7 | 0.071 |
| II-8 | 32.4 |
| II-9 | 0.63 |
| II-10 | 0.33 |
| II-11 | 0.39 |
| II-12 | 0.56 |
| II-13 | 0.25 |
| II-14 | 3 |

| | |
|---|---|
| II-15 | 181.6 |
| II-16 | 0.2 |
| II-17 | 2.9 |
| II-18 | 3.7 |
| II-19 | 0.30 |
| II-20 | 0.7 |
| II-21 | 0.81 |
| II-22 | 0.70 |
| II-23 | 0.88 |
| II-24 | 0.78 |
| II-25 | 1 |
| II-26 | 1 |
| II-27 | 0.88 |
| II-28 | 0.85 |
| II-29 | 2.4 |

[a]SU11274 was used as a positive control. (Sattler, M.; Pride. Y. B.; Ma, P.; Gramlich, J. L.; Chu, S. C.; Quinnan, L. A.; Shirazian, S.; Liang, C.; Podar, K.; Christensen, J. G.; Salgia, R. **A novel small molecule met inhibitor induces apoptosis in cells transformed by the oncogenic TPR-MET tyrosine kinase**. *Cancer research* 2003, 63(17), 5462-9.)

[b]JNJ-38877605 was also used as a positive control. (Lu, T.; Alexander, R.; Connors, R. W.; Cummings, M. D.; Galemmo, R. A.; Hufnagel, H. R.; Johnson, D. L.; Khalil, E.; Leonard, K. A.; Markotan, T. P.; Maroney, A. C.; Sechler, J. L.; Travins, J. M.; Tuman, R. W.**Preparation of triazolopyridazines as tyrosine kinase modulators.** PCT Patent WO 2007075567-A1, 2007.)

Example 55: the effect of compounds on the TPR-Met phosphorylation in NIH3T3/TPR-Met cells

1. Procedure:

NIH3T3/TPR- Met cells (in such cells, therein no interference of Met's extracellular fragment, and the intracellular TPR- Met fused protein was expressed in cytoplasm and could be activated continuously independent of HGF) were inoculated on a 12- well plate. After fusion degree reached 80%, the cells were treated with indicated concentration of corresponding compounds and SU11274 for 6 h at 37 °C, collected and washed once by cold PBS (containing 1 mM sodium vanadate), and lysed with 1xSDS gel loading buffer (50 mM Tris- HCl (pH 6.8), 100 mM DTT, 2% SDS, 10% glycerin, 1 mM sodium vanadate, and 0.1 % bromophenol blue) . The lysate was heated in a boiling- water bath for 10 min and then centrifuged (12000 rpm) at 4°C for 10 min.

The supernatant was subjected to SDS- PAGE electrophoresis and the protein was transferred to nitrocellulose membranes (Amersham Life Sciences, Arlington Heights, IL, USA) by a Semi- Dry Electroblotting System. The nitrocellulose memebranes were incubated in a blocking solution (5% dry milk in TBS/T containing 1mM sodium vanadate) at room temperature for 1 h, and then in an antibody of anti- p- c- Met (Y1234/1235, Cell Sinaling Technology) (1: 1000), anti- p- c- Met (Y1349, Cell Sinaling Technology) (1: 1000), anti- p- AKT (S473, Cell Sinaling Technology) (1: 1000), anti- p- ERK (T202/Y204, Cell Sinaling Technology) (1: 1000), anti- c- Met (C12, Santa Cruz) (1: 1000), anti- AKT (Cell Sinaling Technology) (1: 1000), anti- ERK (Cell Sinaling Technology) (1: 1000) or anti- GAPDH (Kangcheng Bio) (1: 6000) at room temperature for 2 h. The membrane was washed three times by TBS/T containing 1mM of sodium vanadate, 15 min for each time, and then placed in a solution of secondary antibody (1: 2000) at room temperature for 1- 2 hours. After the membranes were washed 3 times as above, they were dyed with an ECL reagent, exposed and developed.

The nitrocellulose membranes were placed in Reblot™ solution of Chemicon co. (Temecula, CA, USA) to separate and remove the combined antibodies thereon, blocked again in a blocking solution (5% dry milk diluted in TBS/T) and re- assayed using anti- c- Met (C12, Santa Cruz Biotechnology) antibody (1: 500) and secondary antibody (1: 2000) following the above the procedures.

2. Result:

Results were shown in Fig. 1. After the present compound [1, 2, 4] triazolo [4, 3- b] [1, 2, 4] triazine at a concentration of 10μM treated the NIH3T3/TPR- Met cells for 6 h, it strongly inhibited the phosphorylation of TPR- Met in NIH3T3/TPR- Met cells. At a dose of 0.01μM, compounds 1- 5 and I- 19 almost completely blocked c- Met phosphorylation in NIH3T3/TPR- Met cells, whereas compound JNJ- 38877605 had less potency.

Example 56: test for the effect of compound on cell proliferation ability mediated by c-Met

1. Procedure:

NIH3T3/TPR- Met cells in the logarithmic growth phase and NIH3T3 background cells were seeded in a 96-well microcuture plate at 90μL per well and cultivated overnight, followed by addition of 10μL of the compound at different concentrations. Three concentrations were set, and for each concentration, the test was carried out in triplicate wells. 72 h later, the culture medium was removed. The residue was fixed by precooled 10% TCA at 4 °C for 1 h, washed by distilled water for 5 times and dried at room temperature. After that, 4mg/mL of sulforodamine B (SRB) in 1% glacial acetic acid was added to each well (100μL per well) for stainning at room temperature for 15 min. The excess dye was removed by washed with 1% glacial acetic acid for 5 times and dried in air. At last, 150μL of Tris- HCL solution (10mM Tris, pH 10.0) was added to each well. OD values were measured on a multi- well spectrophotometer at 515 nM. The inhibitory action of compound on Met mediated proliferation was demonstrated by comparing the inhibition rate of compound on NIH3T3/TPR- Met cells' proliferation and that on NIH3T3 background cells as control.

2. Result:

As shown in table 3, the inhibitory activity of [1, 2, 4] triazolo [4, 3- b] [1, 2, 4] triazine compound of the present application on NIH3T3/TPR- Met cells' proliferation was signifcantly superior to that on NIH3T3 cells' proliferation. Among others, compounds having 3- quinolyl exhibited significantly stronger potency than compounds having 3- indolyl. Among them, some compounds with a concentration of 1 μM exhibited inhibition rate of 50% or more on NIH3T3/TPR- Met cells' proliferation, which was superior to that of positive compound SU11274, and compound with the same concentration exhibited significantly weaker action on NIH3T3 cells' proliferation than that on NIH3T3/TPR- Met cells. Such selectivity existing in inhibitory activities on cells' proliferation of the two kinds of cells was consistent with that of SU11274, which

is a c- Met- specific inhibitor. The result indicated that these compounds were able to selectively inhibit the cellular proliferation mediated by c- Met.

Table 3 inhibition rate (%) of compounds in examples of the present applicaition on proliferation of NIH3T3/TPR-Met cells and NIH3T3 background cells as control

| Compound in Example | NIH3T3/TPR-Met | | | NIH3T3 | | |
|---|---|---|---|---|---|---|
| | Concentration (μM) | | | | | |
| | 50 | 10 | 1 | 50 | 10 | 1 |
| SU11274 | 88.7 | 80.1 | 8.5 | 70.6 | 44.2 | 19.9 |
| JNJ-38877605 | 77.3 | 72.2 | 70.8 | 25.7 | 15.1 | 16.0 |
| I-1 | 83.7 | 83.3 | 66 | 55.9 | 55.3 | 38.8 |
| I-2 | 90.9 | 91.6 | 62.9 | 33.1 | 28.4 | 16.25 |
| I-3 | 88.6 | 88.8 | 86.65 | 37.2 | 36.7 | 37.2 |
| I-4 | 87.9 | 85.6 | 83.8 | 30.7 | 33.8 | 29.9 |
| I-5 | 79 | 78.5 | 76.6 | 42.9 | 38.8 | 30.4 |
| I-6 | 89.3 | 88.4 | 87.7 | 39.1 | 36.2 | 32.7 |
| I-7 | 92.1 | 90.0 | 75.0 | 33.7 | 27.8 | 25.8 |

| | | | | | | |
|---|---|---|---|---|---|---|
| I-8 | 85.6 | 83.7 | 76.0 | 30.5 | 25.4 | 19.7 |
| I-9 | 67.5 | 9.4 | 5.0 | 37.3 | 22.7 | 17.8 |
| I-10 | 56.7 | 13.7 | 8.7 | 34.8 | 21.2 | 11.0 |
| I-11 | 67.5 | 8.4 | 4.1 | 42 | 28.9 | 21.4 |
| I-12 | 89.4 | 87.9 | 87.9 | 42.7 | 39.8 | 35.0 |
| I-13 | 90.8 | 88.6 | 78.7 | 33.6 | 26.8 | 18.9 |
| I-14 | 85.0 | 80.6 | 19.9 | 32.1 | 30.3 | 19.1 |
| I-15 | 88.7 | 89.2 | 82.6 | 35.2 | 31.0 | 30.3 |
| I-16 | 90.1 | 84.6 | 37.3 | 31.9 | 28.7 | 23.6 |
| I-17 | 87.0 | 85.8 | 81.1 | 34.9 | 35.9 | 24.9 |
| I-18 | 75.6 | 44.7 | 18.9 | 28.7 | 20.8 | 17.5 |
| I-19 | 85.6 | 79.3 | 77.7 | 49.2 | 43.3 | 41.5 |
| I-20 | 86.5 | 87.8 | 81.2 | 38.6 | 25.8 | 18.8 |
| I-21 | 85.1 | 76.8 | 6.0 | 28.4 | 25.5 | 12.0 |
| I-22 | 85.2 | 83.6 | 80.4 | 24.6 | 21.7 | 22.2 |
| I-23 | 86.6 | 85.7 | 83.9 | 34.0 | 30.6 | 32.8 |
| I-24 | 87.2 | 87.7 | 83.5 | 32.8 | 23.6 | 16.9 |
| II-1 | | 77.3 | | | 0.0 | |
| II-2 | | 79.0 | | | 21.0 | |
| II-3 | | 77.3 | | | 33.8 | |
| II-4 | | 80.6 | | | 0.0 | |
| II-5 | | 79.2 | | | 6.2 | |
| II-6 | 82.3 | 82.4 | 78.6 | 50.6 | 53.3 | 46.2 |
| II-7 | | 80.3 | | | 0 | |
| II-8 | | 81.2 | | | 7.5 | |

| | | | | | | |
|---|---|---|---|---|---|---|
| II-9 | 83.6 | 81.7 | 69.1 | 50.7 | 44.1 | 32.8 |
| II-10 | 73.6 | 78.8 | 75.8 | 45.9 | 45.4 | 35.9 |
| II-11 | 83.5 | 87.4 | 61.6 | 51.8 | 57.6 | 34.5 |
| II-12 | 76.7 | 76.8 | 32.8 | 29.5 | 33.6 | 20.4 |
| II-13 | 81.8 | 80.2 | 74.9 | 54.5 | 50.6 | 44.8 |
| II-14 | | 75.6 | | | 0.0 | |
| II-15 | | 22.6 | | | 0.0 | |
| II-16 | | 87.9 | | | 56.2 | |
| II-17 | | 65.2 | | | 1.2 | |
| II-18 | 82.6 | 22.9 | 5.6 | 63.5 | 37.3 | 29.6 |
| II-19 | 97.0 | | 6.55 | 83.4 | 31.1 | 24.9 |
| II-20 | 86.7 | 89.4 | 78.7 | 21.4 | 22.5 | 10.4 |
| II-21 | 84.3 | 81.4 | 24.6 | 57.5 | 53.9 | 28.9 |
| II-22 | 78.8 | 81.7 | 63.9 | 48.1 | 53.8 | 38.6 |
| II-23 | 88.5 | 83.2 | 29.0 | 31.3 | 49.8 | 22.8 |
| II-24 | 70.6 | 53.1 | 5.3 | 28.4 | 25.1 | 17.1 |
| II-25 | 83.6 | 43.6 | 9.7 | 63.3 | 40.4 | 26.3 |
| II-26 | 83.9 | 82.7 | 20.9 | 62.2 | 58.7 | 36.8 |
| II-27 | 80.7 | 72.6 | 9.2 | 46 | 37.7 | 18.3 |
| II-28 | 80.7 | 77.3 | 38.2 | 48.3 | 45.9 | 28.3 |
| II-29 | 91.9 | 88.2 | 87.4 | 50.9 | 32.9 | 31.8 |

Example 57: the effect of compound on tumor growth using human glioblastoma cells U-87MG xenograft model of nude mice

1. Procedure:

U- 87MG Cells were inoculated subcutaneously in right axillary fossa of nude mice with $5\times10^6$ cells per mouse. After the xenografted tumor was formed, it was passed three generations in nude mice and then used. Tumor tissue in

productive phase was cut into nubs of about 1.5 $mm^3$ and inoculated subcutaneously in right axillary fossa of the nude mice in a sterile condition. The diameters of the xenografted tumors were measured by a vernier caliper. When the tumors grew up to 100- 200 $mm^3$, the animals were randomly assigned into groups according to tumor volume, 12 per group for negative control group, 6 per group for positive control group and 6 per group for treatment group. The treatment group was administered intraperitoneally and daily with compound I- 19 at various concentrations (50mg/kg, 100mg/kg) respectively. The positive control group was administered intraperitoneally and daily with JNJ- 38877605 at various concentration (50mg/kg, 100mg/kg) respectively. The administration was performed once daily and lasted for 17 days. At the same time, the negative group was administered with equivalent physiological saline.

The length (A) and width (B) of the tumor in mice were measured twice a week and tumor volume was calculated thereby as: $V=A\times B^2/2$. Provided that $V_t$ was the tumor volume measured at a given time point and $V_0$ was the tumor volume measured before the mice were grouped and administered, tumor growth inhibition (TGI) was calculated as:

$$TGI(\%)= \frac{(C_{Vt}-C_{V0})-(T_{Vt}-T_{V0})}{(C_{Vt}-C_{V0})} \times 100\%$$

wherein, $C_{Vt}$ was tumor volume in control group measured at a given time point, $C_{V0}$ was tumor volume in control group measured before grouping and treatment, $T_{Vt}$ was tumor volume in treatment group measured at a given time point, and $T_{V0}$ was tumor volume in treatment group measured before grouping and treatment. The significant differences between the treated and control groups were evaluated using t-test. At the same time, body weight of nude mice in each group was measured twice a week to preliminarily evaluate the toxicity and side effect of compound.

2. Result:

At the end of treatment (d17), as for the U-87MG transplated tumor in nude mice in each treatment group using compound I-19, TGI values were 95.3% (***p<0.001, 100mg/kg) and 83.9%(***p<0.001, 50mg/kg) respectively. On the other hand, as for the U-87MG transplated tumor in nude mice in each treatment group using JNJ38877605 TGI values were 106.9% (***p <0.001, 100mg/kg) and 101.4% (***p<0.001, 50mg/kg) respectively. At each administration dose, both compound **I-19** and positive control **JNJ-38877605** exhibited significantly inhibitory action on the growth of transplated tumor, wherein, the inhibitory action of compound **I-19** on tomor growth was weaker than that of positive control **JNJ-38877605** (see Fig. 2). In **I-19** (100mg/kg) treated group, one mouse died on the 9th day, but it didn't show weight loss or obvious abnormal appearance. No mouse in **I-19** or **JNJ-38877605** treated group show obvious weight loss (see Fig. 3).

## Claims

1. A [1, 2, 4] triazolo [4, 3- b] [1, 2, 4] triazine compound having a structure as shown in the following formula (I) or (II), pharmaceutically acceptable salts, prodrugs, hydrates or solvates thereof:

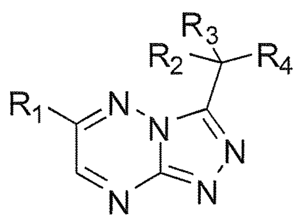
(I)

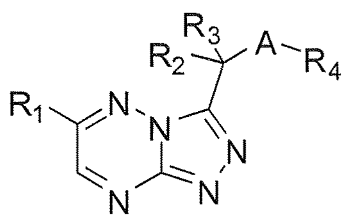
(II)

wherein:

$R_1$ and $R_4$ are each independently a substituted or unsubstituted aryl or heteroaryl,
the aryl is a $C_6$-$C_{20}$ monocyclic or fused ring group having at least one aromatic ring;
the heteroaryl group is a 5- to 10-membered heteroaryl group containing 1 to 5 hetero atoms selected from the group consisting of N, S, P and O;
the substituents for the substitution are 1-4 group(s) selected from the group consisting of halogen, $C_1$-$C_6$ linear

or branched alkyl, nitro group, amino, hydroxyl, hydroxymethyl, trifluoromethyl, trifluoromethoxy, carboxyl, $C_1$-$C_4$ alkoxy, benzyloxy, $C_1$-$C_4$ acyl, benzyl, piperidyl, tert-butoxycarbonyl-substituted piperidyl and methoxyformyl;
$R_2$ and $R_3$ are each independently hydrogen atom or halogen;
A is an oxygen atom, a sulfur atom, an amine group, $C_1$-$C_6$ alkyl substituted amine group or a carbon atom.

**2.** The [1, 2, 4] triazolo [4, 3- b] [1, 2, 4] triazine compound, pharmaceutically acceptable salts, prodrugs, hydrates or solvates thereof according to claim 1, wherein, the aryl is phenyl, substituted phenyl, naphthyl or xenyl; the heteroaryl is pyrazolyl, furyl, pyrrolyl, pyridyl, thienyl, imidazolyl, quinolyl, isoquinolyl or indolyl; the substituents for the substitution are 1- 4 group (s) selected from the group consisting of halogen, $C_1$- $C_6$ linear or branched alkyl, nitro group, amino, hydroxyl, hydroxymethyl, trifluoromethyl, trifluoromethoxy, carboxyl, $C_1$- $C_4$ alkoxy, benzyloxy, $C_1$- $C_4$ acyl, benzyl, piperidyl, tert- butoxycarbonyl- substituted piperidyl and methoxyformyl.

**3.** The [1, 2, 4] triazolo [4, 3- b] [1, 2, 4] triazine compound, pharmaceutically acceptable salts, prodrugs, hydrates or solvates thereof according to claim 1, wherein, the compound has a structure of formula (I), and $R_2$ and $R_3$ are each independently hydrogen atom.

**4.** The [1, 2, 4] triazolo [4, 3- b] [1, 2, 4] triazine compound, pharmaceutically acceptable salts, prodrugs, hydrates or solvates thereof according to claim 1, wherein, the compound has a structure of formula (I), and $R_2$ and $R_3$ are each independently fluorine atom.

**5.** The [1, 2, 4] triazolo [4, 3- b] [1, 2, 4] triazine compound, pharmaceutically acceptable salts, prodrugs, hydrates or solvates thereof according to claim 1, wherein, the compound has a structure of formula (I), and $R_2$ and $R_3$ are each independently hydrogen atom or fluorine atom; $R_4$ is substituted or unsubstituted phenyl, quinolyl or indolyl, the substituents for the substitution are 1- 4 group (s) selected from the group consisting of halogen, $C_1$- $C_6$ linear or branched alkyl, nitro group, amino, hydroxyl, hydroxymethyl, trifluoromethyl, trifluoromethoxy, carboxyl, $C_1$- $C_4$ alkoxy, benzyloxy, $C_1$- $C_4$ acyl, benzyl, piperidyl, tert- butoxycarbonyl- substituted piperidyl and methoxyformyl.

**6.** The [1, 2, 4] triazolo [4, 3- b] [1, 2, 4] triazine compound, pharmaceutically acceptable salts, prodrugs, hydrates or solvates thereof according to claim 1, wherein, the compound has a structure of formula (II), and A is independently an oxygen atom, a sulfur atom, an amine group or a carbon atom, $R_2$ and $R_3$ are each independently a hydrogen atom.

**7.** The [1, 2, 4] triazolo [4, 3- b] [1, 2, 4] triazine compound, pharmaceutically acceptable salts, prodrugs, hydrates or solvates thereof according to claim 1, wherein, the compound has a structure of formula (II), and A is independently an oxygen atom, a sulfur atom, an amine group or a carbon atom, $R_2$ and $R_3$ are each independently a hydrogen atom; $R_4$ is substituted or unsubstituted phenyl, quinolyl or indolyl, the substituents for the substitution are 1- 4 group (s) selected from the group consisting of halogen, $C_1$- $C_6$ linear or branched alkyl, nitro group, amino, hydroxyl, hydroxymethyl, trifluoromethyl, trifluoromethoxy, carboxyl, $C_1$- $C_4$ alkoxy, benzyloxy, $C_1$- $C_4$ acyl, benzyl, piperidyl, tert- butoxycarbonyl- substituted piperidyl and methoxyformyl.

**8.** The [1, 2, 4] triazolo [4, 3- b] [1, 2, 4] triazine compound, pharmaceutically acceptable salts, prodrugs, hydrates or solvates thereof according to claim 1, wherein, the compound is a compound having the following structure:

| | |
|---|---|
| 3-(6-quinolylmethyl)-6-phenyl-[1,2,4]triazolo[4,3-b] [1,2,4] triazine | 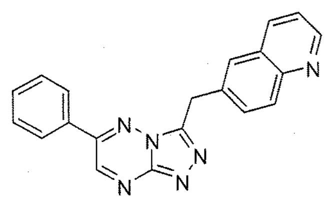 |
| 3-(6-quinolylmethyl)-6-(4-benzyloxyphenyl)-[1,2,4]triazolo[4,3-b][1,2,4]triazine | 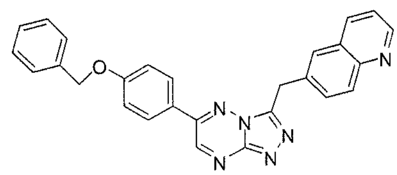 |

(continued)

| | |
|---|---|
| 3-(6-quinolylmethyl)-6-(2-thienyl)-[1,2,4]triazolo[4,3-b][1,2,4] triazine | 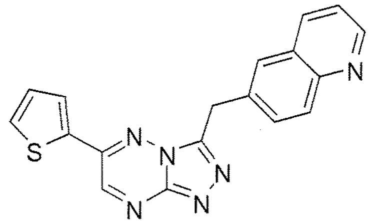 |
| 3-(6-quinolylmethyl)-6-(3,4-dichlorophenyl)-[1,2,4]triazolo[4,3-b][1,2,4]triazine | 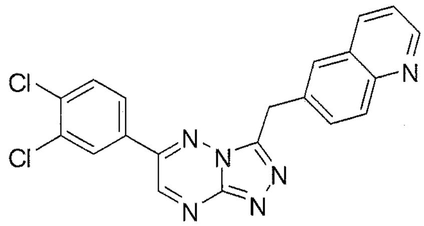 |
| 3-(6-quinolylmethyl)-6-[(1-methyl)-4-pyrazolyl]-[1,2,4]triazolo [4,3-b][1,2,4]triazine | 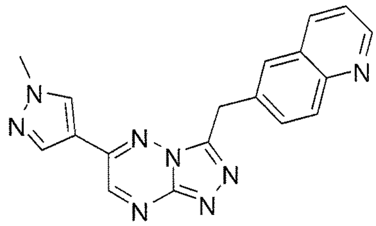 |
| 3-(6-quinolylmethyl)-6-[(1-ethyl)-4-pyrazolyl]-[1,2,4]triazolo [4,3-b][1,2,4]triazine | 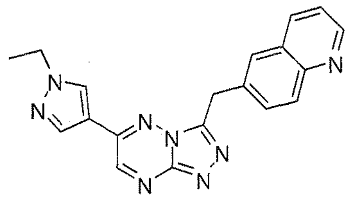 |
| 3-(6-quinolylmethyl)-6-[(1-benzyl)-4-pyrazolyl]-[1,2,4]triazolo [4,3-b][1,2,4]triazine | 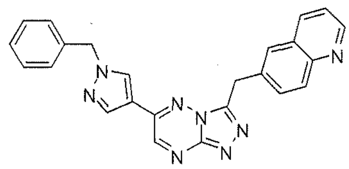 |
| 3-(6-quinolylmethyl)-6-(3-quinolyl)-[1,2,4]triazolo[4,3-b][1,2,4] triazine | 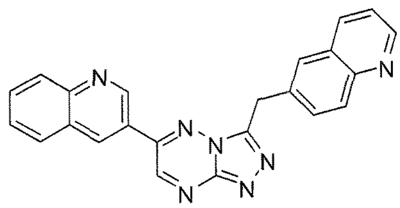 |
| 3-(3-indolylmethyl)-6-[(1-propyl)-4-pyrazolyl]-[1,2,4]triazolo [4,3-b][1,2,4]triazine | 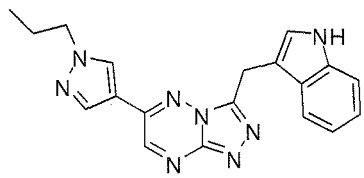 |
| 3-(3-indolylmethyl)-6-(4-bromophenyl)-[1,2,4]triazolo[4,3-b] [1,2,4]triazine | 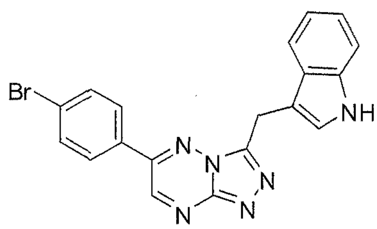 |

(continued)

| | |
|---|---|
| 3-(3-indolylmethyl)-6-(3-hydroxyphenyl)-[1,2,4]triazolo[4,3-b][1,2,4]triazine | 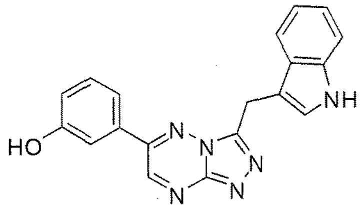 |
| 3-(6-quinolylmethyl)-6-[(1-propyl)-4-pyrazolyl]-[1,2,4]triazolo[4,3-b][1,2,4]triazine | 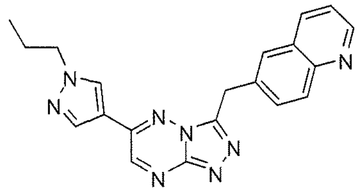 |
| 3-(6-quinolylmethyl)-6-[1-(1-tert-butoxycarbonyl-4-piperidyl)-4-pyrazolyl]-[1,2,4]triazolo[4,3-b][1,2,4]tr iazine | 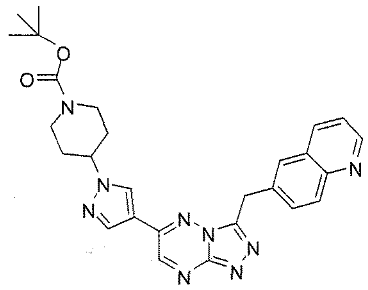 |
| 3-(6-quinolylmethyl)-6-[1-(4-piperidyl)-4-pyrazolyl]-[1,2,4]triazolo[4,3-b][1,2,4]triazine | 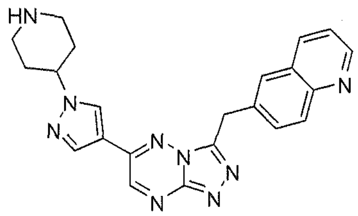 |
| 3-(6-quinolylmethyl)-6-(3-nitrophenyl)-[1,2,4]triazolo[4,3-b][1,2,4]triazine | 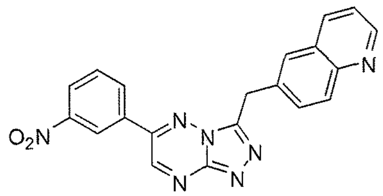 |
| 3-(6-quinolylmethyl)-6-(4-chlorophenyl)-[1,2,4]triazolo[4,3-b][1,2,4]triazine | 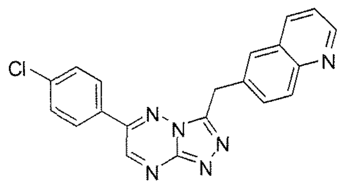 |
| 3-(6-quinolylmethyl)-6-(4-bromophenyl)-[1,2,4]triazolo[4,3-b][1,2,4]triazine | 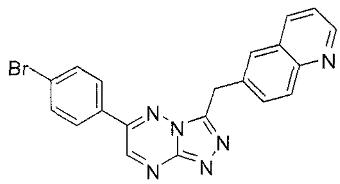 |
| 3-(3-indolylmethyl)-6-(4-benzyloxyphenyl)-[1,2,4]triazolo[4,3-b][1,2,4]triazine | 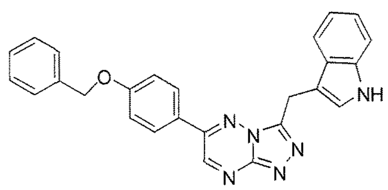 |

(continued)

| | |
|---|---|
| 3-[(6-quinolyl)difluoromethyl]-6-[(1-methyl)-4-pyrazolyl]-[1,2,4]triazolo[4,3-b][1,2,4]triazine | 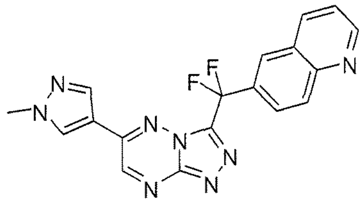 |
| 3-[(6-quinolyl)difluoromethyl]-6-[1-(1-tert-butoxycarbonyl-4-piperidyl)-4-pyrazolyl]-[1,2,4]triazolo[4,3-b][1,2,4]triazine | 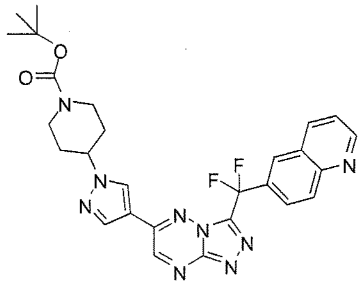 |
| 3-[(6-quinolyl)difluoromethyl]-6-[1-(4-piperidyl)-4-pyrazolyl]-[1,2,4]triazolo[4,3-b][1,2,4]triazine | 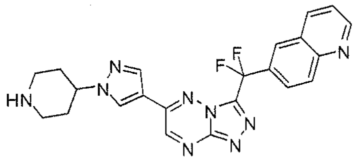 |
| 3-[(6-quinolyl)difluoromethyl]-6-phenyl-[1,2,4]triazolo[4,3-b][1,2,4]triazine | 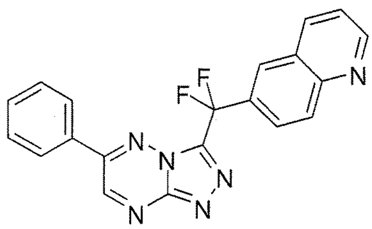 |
| 3-[(6-quinolyl)difluoromethyl]-6-(2-thienyl)-[1,2,4]triazolo[4,3-b][1,2,4]triazine | 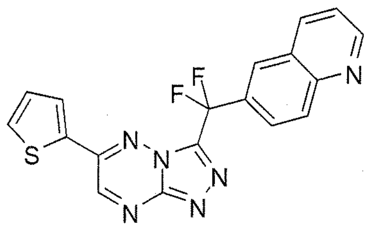 |
| 3-[(6-quinolyl)difluoromethyl]-6-(4-benzyloxypheny1)-[1,2,4]triazolo[4,3-b][1,2,4]triazine | 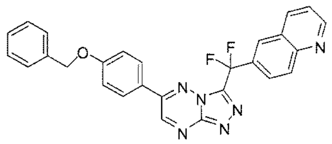 |
| 3-[(7-methoxy-4-quinolyl)oxymethyl]-6-(4-fluorophenyl)-[1,2,4]triazolo[4,3-b][1,2,4]triazine | 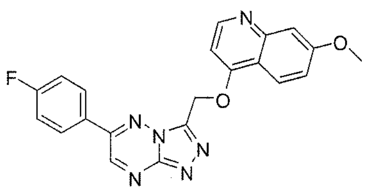 |
| 3-[(7-methoxy-4-quinolyl)oxymethyl]-6-(4-chlorophenyl)-[1,2,4]triazolo[4,3-b][1,2,4]triazine | 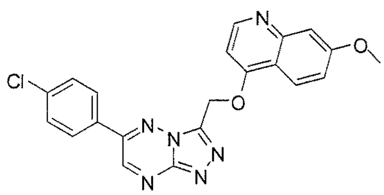 |

(continued)

| | |
|---|---|
| 3-[(7-methoxy-4-quinolyl)oxymethyl]-6-(4-bromophenyl)-[1,2,4] triazolo[4,3-b][1,2,4]triazine | 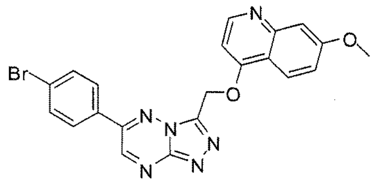 |
| 3-[(7-methoxy-4-quinolyl)oxymethyl]-6-(2-thienyl)-[1,2,4] triazolo[4,3-b][1,2,4]triazine | 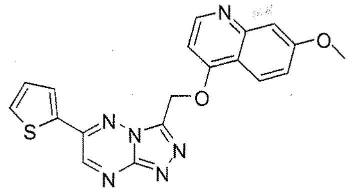 |
| 3-[(7-methoxy-4-quinolyl)oxymethyl]-6-(4-methoxyformylphenyl)-[1,2,4]triazolo[4,3-b][1,2,4]triazine | 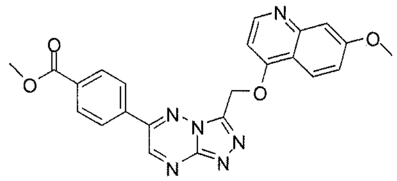 |
| 3-[(7-methoxy-4-quinolyl)oxymethyl]-6-(4-nitrophenyl)-[1,2,4] triazolo[4,3-b][1,2,4]triazine | 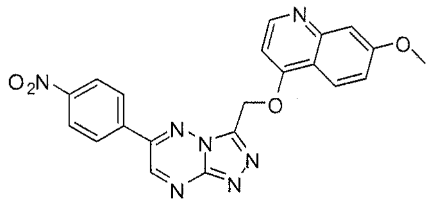 |
| 3-[(7-methoxy-4-quinolyl)oxymethyl]-6-phenyl-[,2,4]triazolo [4,3-b][1,2,4]triazine | 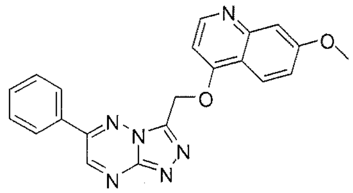 |
| 3-[(7-methoxy-4-quinolyl)oxymethyl]-6-(4-benzyloxyphenyl)-[1,2,4]triazolo[4,3-b][1,2,4]triazine | 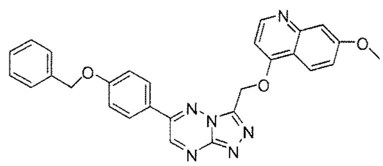 |
| 3-[(7-methoxy-4-quinolyl)oxymethyl]-6-(4-methoxyphenyl)-[1,2,4]triazolo[4,3-b][1,2,4]triazine | 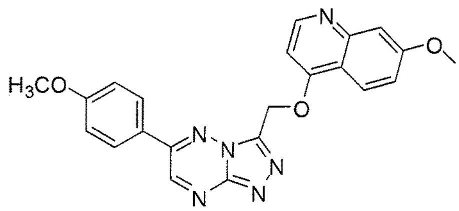 |
| 3-[(7-methoxy-4-quinolyl)oxymethyl]-6-(4-hydroxyphenyl)-[1,2,4]triazolo[4,3-b][1,2,4]triazine | 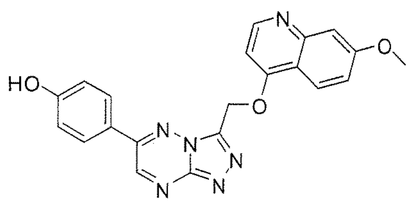 |

(continued)

| | |
|---|---|
| 3-[(7-methoxy-4-quinolyl)oxymethyl]-6-(3-methoxyphenyl)-[1,2,4]triazolo[4,3-b][1,2,4]triazine | 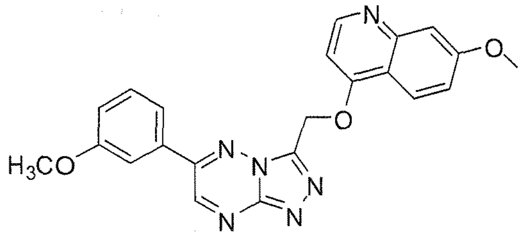 |
| 3-[(7-methoxy-4-quinolyl)oxymethyl]-6-(3-hydroxyphenyl)-[1,2,4]triazolo[4,3-b][1,2,4]triazine | 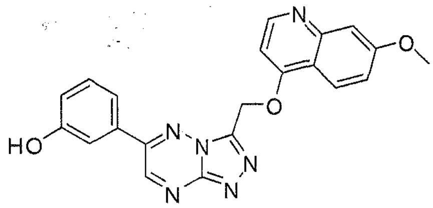 |
| 3-[(7-methoxy-4-quinolyl)oxymethyl]-6-(3-nitrophenyl)-[1,2,4]triazolo[4,3-b][1,2,4]triazine | 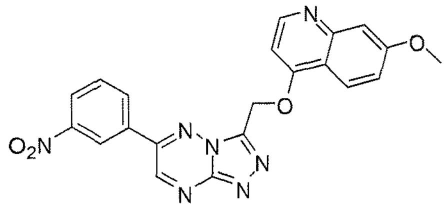 |
| 3-[(6,7-dimethoxy-4-quinolyl)oxymethyl]-6-(4-fluorophenyl)-[1,2,4]triazolo[4,3-b][1,2,4]triazine | 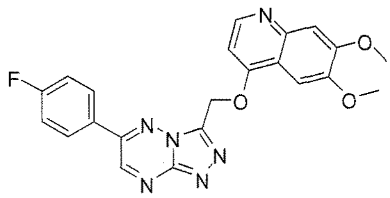 |
| 3-[(6,7-dimethoxy-4-quinolyl)oxymethyl]-6-(4-chlorophenyl)-[1,2,4]triazolo[4,3-b][1,2,4]triazine | 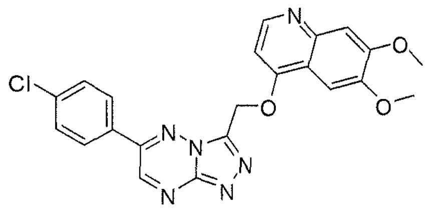 |
| 3-[(6,7-dimethoxy-4-quinolyl)oxymethyl]-6-(4-bromophenyl)-[1,2,4]triazolo[4,3-b][1,2,4]triazine | 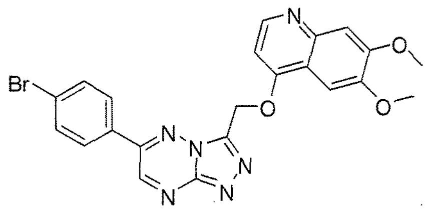 |
| 3-[(6,7-dimethoxy-4-quinolyl)oxymethyl]-6-phenyl-[1,2,4]triazolo[4,3-b][1,2,4]triazine | 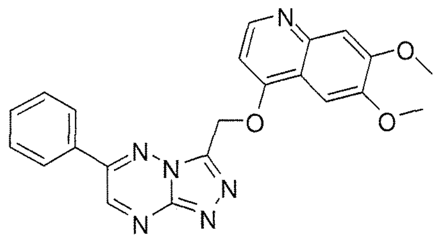 |

(continued)

| | |
|---|---|
| 3-[(6,7-dimethoxy-4-quinolyl)oxymethyl]-6-(4-methoxyformylphenyl)-[1,2,4]triazolo[4,3-b][1,2,4]triazine | 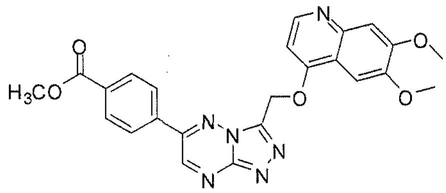 |
| 3-[(6,7-dimethoxy-4-quinolyl)oxymethyl]-6-(4-benzyloxyphenyl)-[1,2,4]triazolo[4,3-b][1,2,4]triazine | 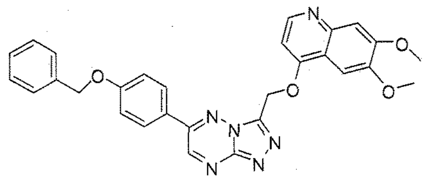 |
| 3-[(7-methoxy-4-quinolyl)oxymethyl]-6-(3,4-dichlorophenyl)-[1,2,4]triazolo[4,3-b][1,2,4]triazine | 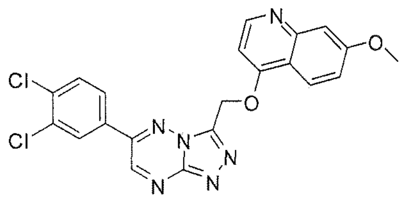 |
| 3-(4-quinolyloxymethyl)-6-(4-fluorophenyl)-[1,2,4]triazolo[4,3-b][1,2,4]triazine | 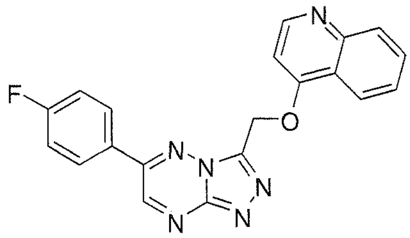 |
| 3-(4-quinolyloxymethyl)-6-phenyl-[1,2,4]triazolo[4,3-b][1,2,4]triazine | 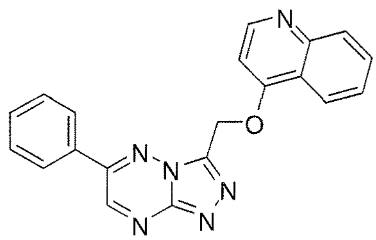 |
| 3-(4-quinolyloxymethyl)-6-(4-methoxyphenyl)-[1,2,4]triazolo[4,3-b][1,2,4]triazine | 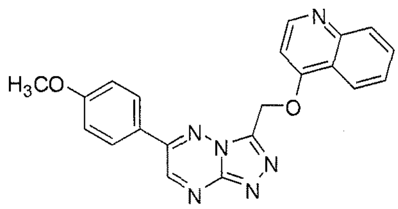 |
| 3-(4-quinolyloxymethyl)-6-(4-hydroxyphenyl)-[1,2,4]triazolo[4,3-b][1,2,4]triazine | 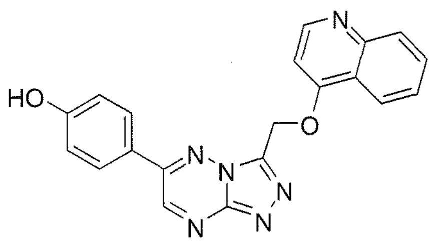 |

(continued)

| | |
|---|---|
| 3-(4-quinolyloxymethyl)-6-(4-methoxyformylphenyl)-[1,2,4] triazolo[4,3-b][1,2,4]triazine | 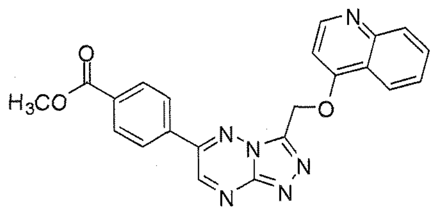 |
| 3-(4-quinolyloxymethyl)-6-(3-methoxyphenyl)-[1,2,4]triazolo [4,3-b][1,2,4]triazine | 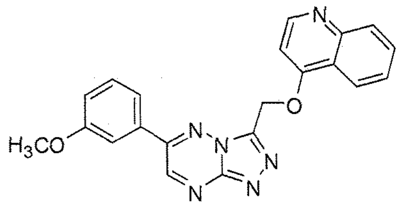 |
| 3-(4-quinolyloxymethyl)-6-(3-hydroxyphenyl)-[1,2,4]triazolo [4,3-b][1,2,4]triazine | 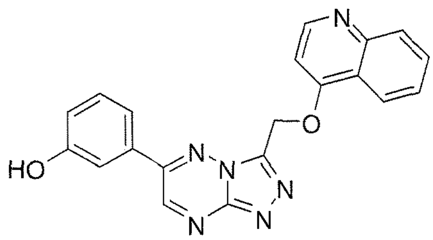 |
| 3-(4-quinolyloxymethyl)-6-(3-nitrophenyl)-[1,2,4]triazolo[4,3-b] [1,2,4]triazine | 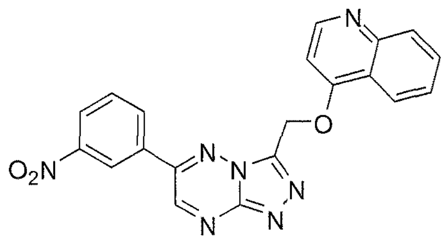 |
| 3-[(7-methoxy-4-quinolyl)oxymethyl]-6-[(1-methyl)-4-pyrazolyl]-[1,2,4]triazolo[4,3-b][1,2,4]triazine | 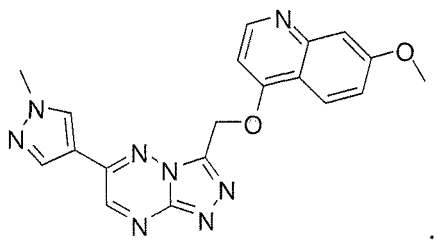 |

**9.** A method for preparating the [1, 2, 4] triazolo [4, 3- b] [1, 2, 4] triazine compound according to any one of claims 1 to 8,

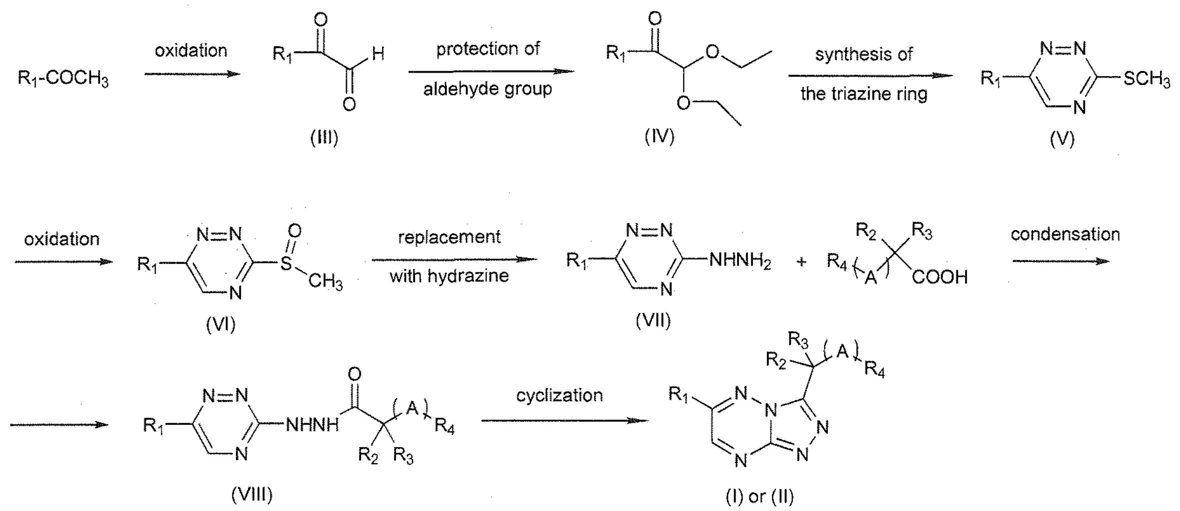


wherein, $R_1$ to $R_4$ and A are as defined in claim 1;
the method comprises:

oxidizing an acetyl compound by selenium dioxide or hydrobromic acid and dimethyl sulfoxide, to give a compound (III) ;
reacting the compound (III) and triethyl ortho-formate in the presence of p-toluenesulfonic acid as a catalyst, to synthesize acetal (IV);
reacting the compound (IV) and thiosemicarbazide via acid catalyzed condensation and methylation to give a compound (V);
oxidizing the methylthio of the compound (V) by m-chloroperoxybenzoic acid, hydrogen peroxide, potassium permanganate, manganese dioxide, potassium periodate or potassium dichromate to give a compound (VI);
substituting the compound (VI) with hydrazine as a nucleophilic reagent to give compound (VII);
condensing the compound (VII) and a carboxylic acid compound by dicyclohexyl- carbodiimide or 1- (3- dimethylaminopropyl)- 3- ethylcarbodiimide hydrochloride to give a hydrazide compound (VIII), or reacting the compound (VII) with an acyl chloride prepared from the carboxylic acid to synthesize the hydrazide compound (VIII) ;
cyclizing the hydrazide compound (VIII) to give the target compound (I) or (II);
wherein, the cyclizing reagent is phosphorous oxychloride, formic acid, acetic acid, methanesulfonic acid, trifluoromethanesulfonic acid, p-toluenesulfonic acid or ethanesulfonic acid.

**10.** A pharmaceutical composition for the prevention and/or treatment of a disease associated with c- Met abnormality, comprising the [1, 2, 4] triazolo [4, 3- b] [1, 2, 4] triazine compound according to any one of claims 1 to 8, or a pharmaceutically acceptable salt thereof, and at least one pharmaceutically acceptable carrier.

**11.** The use of the [1, 2, 4] triazolo [4, 3- b] [1, 2, 4] triazine compound according to any one of claims 1 to 8 in the preparation of a medicament for the prevention and/or treatment of a disease associated with c- Met abnormality.

**12.** The use according to claim 11, wherein, the disease associated with c-Met abnormality is tumor.

**13.** The use according to claim 12, wherein, the tumor is lung cancer, breast cancer, colon cancer, prostate cancer, pancreatic cancer, gastric cancer, liver cancer, ovarian cancer, renal cancer, neurospongioma, melanoma, pancreatic cancer, head and neck cancer, bladder cancer, cervical cancer, bile duct cancer, nasopharyngeal cancer, thyroid cancer, osteosarcoma, synovial sarcoma, rhabdomyosarcoma, fibrosarcoma, leiomyosarcoma, multiple myeloma, lymphoma or leukemia.

Fig. 1

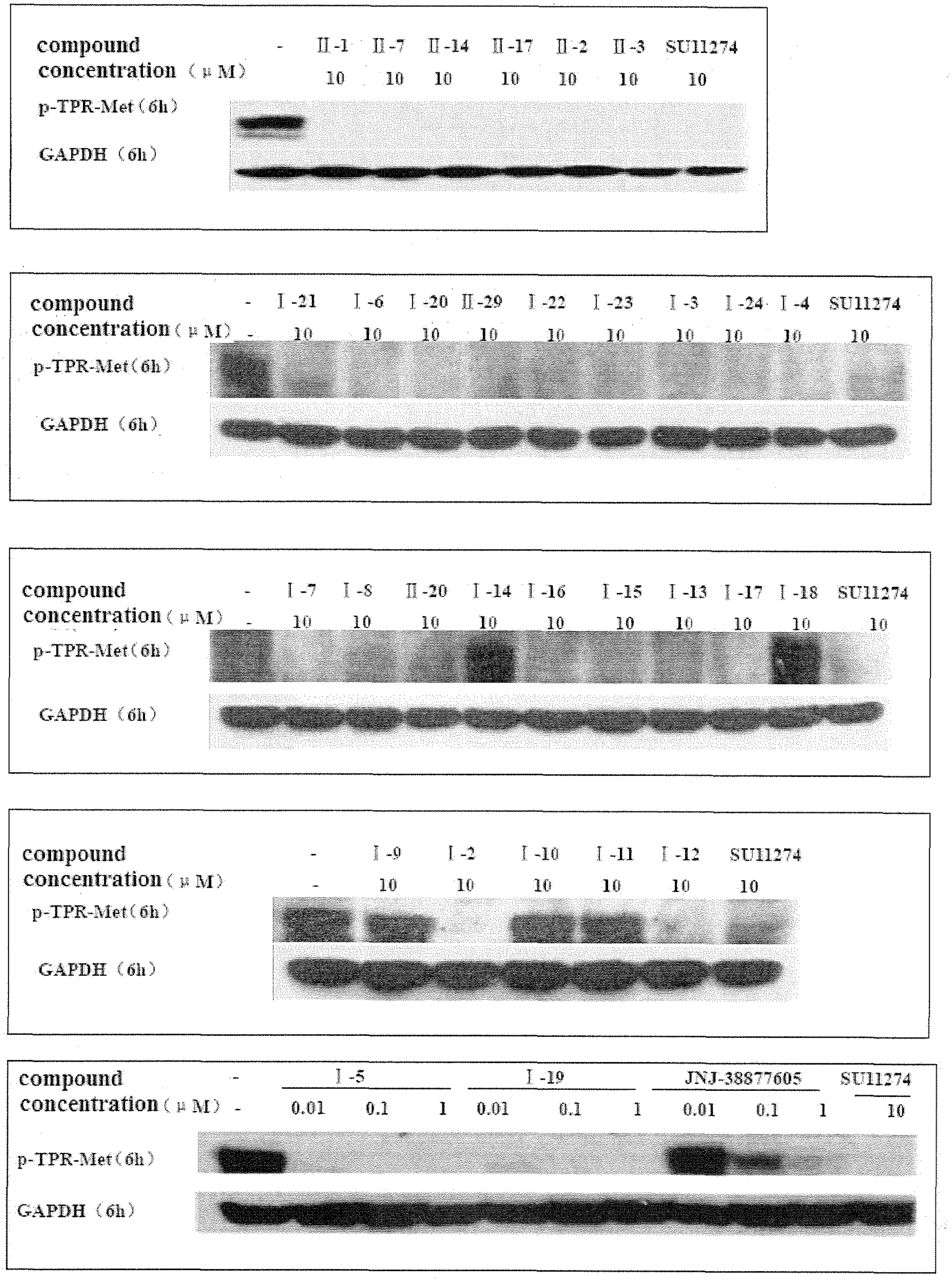
compound - II-1 II-7 II-14 II-17 II-2 II-3 SU11274
concentration (μM) 10 10 10 10 10 10 10
p-TPR-Met (6h)
GAPDH (6h)
compound - I-21 I-6 I-20 II-29 I-22 I-23 I-3 I-24 I-4 SU11274
concentration (μM) - 10 10 10 10 10 10 10 10 10 10
p-TPR-Met (6h)
GAPDH (6h)
compound - I-7 I-8 II-20 I-14 I-16 I-15 I-13 I-17 I-18 SU11274
concentration (μM) - 10 10 10 10 10 10 10 10 10 10
p-TPR-Met (6h)
GAPDH (6h)
compound - I-9 I-2 I-10 I-11 I-12 SU11274
concentration (μM) - 10 10 10 10 10 10
p-TPR-Met (6h)
GAPDH (6h)
compound - I-5 I-19 JNJ-38877605 SU11274
concentration (μM) - 0.01 0.1 1 0.01 0.1 1 0.01 0.1 1 10
p-TPR-Met (6h)
GAPDH (6h)

Fig. 2

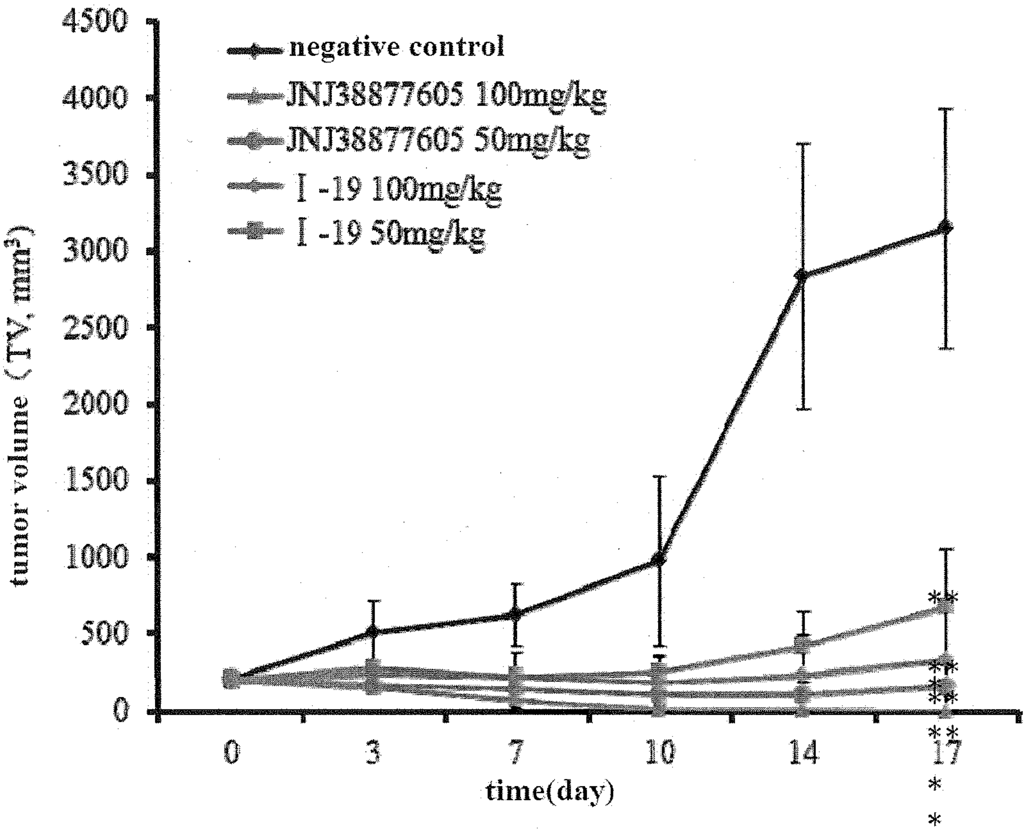
negative control
JNJ38877605 100mg/kg
JNJ38877605 50mg/kg
Ⅰ-19 100mg/kg
Ⅰ-19 50mg/kg
tumor volume（TV, mm³)
time(day)
4500
4000
3500
3000
2500
2000
1500
1000
500
0
0
3
7
10
14
17

Fig. 3

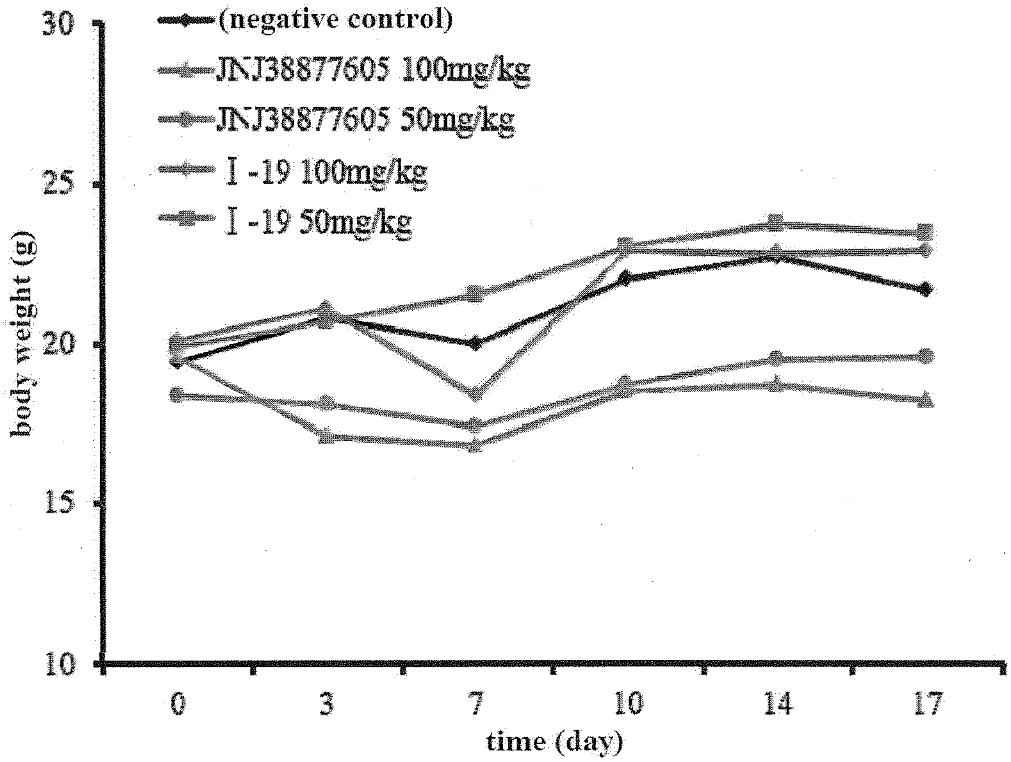
(negative control)
JNJ38877605 100mg/kg
JNJ38877605 50mg/kg
I -19 100mg/kg
I -19 50mg/kg
30
25
20
15
10
body weight (g)
0
3
7
10
14
17
time (day)

| INTERNATIONAL SEARCH REPORT | International application No. PCT/CN2011/002052 |
|---|---|

**A. CLASSIFICATION OF SUBJECT MATTER**

See the extra sheet

According to International Patent Classification (IPC) or to both national classification and IPC

B. FIELDS SEARCHED

Minimum documentation searched (classification system followed by classification symbols)

IPC: C07D487/-, A61K31/53

Documentation searched other than minimum documentation to the extent that such documents are included in the fields searched

Electronic data base consulted during the international search (name of data base and, where practicable, search terms used)

WPI, EPODOC, CNKI, CNPAT, REG, CAPLUS, c-Met，HGFR，triazolo+，+triazin+，tumour，tumor，cancer， substructure search according to the formulas (I) and (II)

C. DOCUMENTS CONSIDERED TO BE RELEVANT

| Category* | Citation of document, with indication, where appropriate, of the relevant passages | Relevant to claim No. |
|---|---|---|
| X | WO2005010005A1 (SUGEN INC), 03 Feb. 2005 (03.02.2005) description,see pages 5-8, embodiments1-6 and table 1 of the and claims | 1-5, 8, 10-13 |
| Y | | 1-2, 6-8, 10-13 |
| A | description, page 27 and scheme I | 9 |
| Y | WO2009091374A2 (AMGEN INC), 23 Jul. 2009 (23.07.2009) description,see pages 4-8 and 24 | 1-2, 6-8, 10-13 |
| A | description, pages 52-53 | 9 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 23 Feb. 2012 (23.02.2012) | 22 Mar. 2012 (22.03.2012) |
| Name and mailing address of the ISA/CN<br>The State Intellectual Property Office, the P.R.China<br>6 Xitucheng Rd., Jimen Bridge, Haidian District, Beijing, China<br>100088<br>Facsimile No.( 86-10)-62019451 | Authorized officer<br>YANG, Yi<br>Telephone No. (86-10)62086352 |

Form PCT/ISA /210 (second sheet) (July 2009)

**INTERNATIONAL SEARCH REPORT**
Information on patent family members

International application No.
**PCT/CN2011/002052**

| Patent Documents referred in the Report | Publication Date | Patent Family | Publication Date |
|---|---|---|---|
| WO2005010005A1 | 03.02.2005 | CA2529238A | 03.02.2005 |
| | | US2005075340A | 07.04.2005 |
| | | EP1644377A | 12.04.2006 |
| | | BRPI0412207A | 22.08.2006 |
| | | JP2007516206A | 21.06.2007 |
| WO2009091374A2 | 23.07.2009 | US2009124609A | 14.05.2009 |
| | | CA2711101A | 23.07.2009 |
| | | AU2008348182A | 23.07.2009 |
| | | US2009318436A | 24.12.2009 |
| | | EP2231663A | 29.09.2010 |
| | | MX2010007683A | 25.10.2010 |
| | | CR11627A | 22.11.2010 |
| | | KR20100129274A | 08.12.2010 |
| | | JP2011509997A | 31.03.2011 |
| | | CN102007125A | 06.04.2011 |
| | | EA201001159A | 29.04.2011 |
| | | CO6290683A | 20.06.2011 |
| | | JP4762367B2 | 31.08.2011 |

Form PCT/ISA /210 (patent family annex) (July 2009)

# INTERNATIONAL SEARCH REPORT

International application No.

**PCT/CN2011/002052**

**A. CLASSIFICATION OF SUBJECT MATTER**

According to International Patent Classification (IPC) or to both national classification and IPC

C07D487/04(2006.01)i
A61K31/53(2006.01)i
A61P35/00(2006.01)i
A61P35/02(2006.01)i

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005004607 A
- WO 2005004808 A
- WO 2005005378 A
- WO 2005010005 A
- WO 2008051808 A
- WO 2007075567 A
- US 2007265272 A


### Non-patent literature cited in the description


- **BLUME JP ; HUNTER T.** Oncogenic kinase signaling [J]. *Nature,* 2001, vol. 411 (6835), 355-365